# EUROPEAN PATENT APPLICATION

(11) **EP 1 550 637 A1**
(43) Date of publication of application: **06.07.2005**
(21) Application number: 03719221.8
(22) Date of filing: 25.04.2003
(51) Int. Cl.: C02F 1/70, C02F 1/46, A61K 7/00, A61K 9/08, A61K 31/375, A61K 33/00, A61K 35/02, A61P 39/06, A23L 1/30, A23L 3/358

(54) **METHOD OF INHIBITING OXIDATION, WATER CAPABLE OF INHIBITING OXIDATION AND USE THEREOF**

(30) Priority: 26.04.2002 JP 2002125986; 28.06.2002 WO PCT/JP02/06560; 27.12.2002 JP 2002381774
(71) Applicant: MIZ Co., Ltd., Fujisawa-shi Kanagawa 251-0871 (JP)
(72) Inventor: Yanagihara, Tomoyuki, c/o MIZ CO., LTD., Fujisawa-shi, Kanagawa 251-0871 (JP); Satoh, Bunpei, c/o MIZ CO., LTD., Fujisawa-shi, Kanagawa 251-0871 (JP); Shudo, Tatsuya, c/o MIZ CO., LTD., Fujisawa-shi, Kanagawa 251-0871 (JP)
(74) Representative: Wiebusch, Manfred
(86) International application number: PCT/JP2003/005386
(87) International publication number: WO 2004/039735

(57) **Abstract**

The objective of the present invention is to provide an antioxidation method and antioxidant-functioning water that can anticipate high benchmarks of safety on the antioxidation subject such as the human body and reduced environmental burden. An antioxidation method and antioxidant- functioning water that can transform or maintain an antioxidation subject, which is in an oxidation state due to a deficiency of electrons or needs to be protected from oxidation, into a reduced state where electrons are filled by promoting the breaking reaction of molecular hydrogen used as a substrate included in the hydrogen- dissolved water into a product of active hydrogen through a process employing a catalyst, which is a precious metal colloid or a hydrogen oxidation/ reduction enzyme (except for those already existing in a living organism), on the hydrogen-dissolved water.

## Description

### TECHNICAL FIELD

The present invention relates to a method of antioxidation, antioxidant- functioning water, and usage of the same that can transform an antioxidation subject, which is in an oxidation state due to a deficiency of electrons or needs to be protected from oxidation, into a reduced state where electrons are filled, by promoting the breaking reaction of a molecular hydrogen substrate included in hydrogen-dissolved water into a product of active hydrogen via a process employing a catalyst, which is a precious metal colloid or a hydrogen oxidation/ reduction enzyme (except for those already existing in a living organism), on the hydrogen-dissolved water.

### BACKGROUND ART

For living organisms, oxygen is a double-edged sword. It has been pointed out that while oxygen is used to procure energy by oxidizing nutrients and/ or performing various oxygen- added reactions essential for living organisms, there is a risk that leads to various types of constitutional disturbances emanating from such oxidizing power.

In particular, a metabolism-produced active oxygen radical and a free radical such as nitric oxide (NO) are highly reactive atoms and molecules, which have unstable unpaired electrons, and try to maintain their stability by capturing or transferring one more electron. Generally, four types:: superoxide anion radical (·O₂⁻), hydroxy radical (·OH), hydrogen peroxide (H₂O₂), and singlet oxygen (¹O₂ ) are called active oxygen radicals. Hydroperoxy radical (HO_{2·}), peroxy radical (LO₂·), and alkoxy radical (LO·) are thought as broad active oxygen radicals. In the following description, these active oxygen radicals may be generically referred to as 'active oxygen species'. In addition, what are referred here as active oxygen species and free radicals such as nitric oxide (NO) may simply be generically called 'radicals'.

Another active oxygen species is produced in the iron ion or the copper ion as a by-product from the active oxygen species. It is becoming clear that the active oxygen species produced in such accelerating manner involves various health problems through damaging cells and DNA, and producing lipid peroxide, a factor that accelerates the aging process. In particular, the hydroxy radical (·OH) is the highest reactive active oxygen species, which have large disturbance effects such as cell damage. Furthermore, it has been confirmed that a hydroxy radical (·OH) is produced and is involved in inflammation of skin due to ultraviolet rays (UV). It is known that viral infections have toxic effects on living organisms since, as a result of excessive immune responses of infected persons, more active oxygen species than the amount necessary for keeping balance in the organisms are produced.

Active oxygen species that have such toxic effects on living organisms are normally scavenged in living organisms with an enzyme such as superoxide dismutase (SOD) or catalase.

However, it has been found that if balance in the organism is upset, for example by factors such as stress, alcohol, smoking, strenuous exercise, or aging, SOD levels decrease and lipid peroxide increases because of the active oxygen species. This brings on various health problems such as heart attacks, arteriosclerosis, diabetes, cancer, strokes, cataracts, stiff shoulders, over sensitivity to cold, high blood pressure, and senile dementia, as well as problems of degradation in physiological functions of the organism, or of degeneration in cosmetic appearance, such as age spots, freckles, and wrinkles.

Meanwhile, it has been pointed out that the involvement of radicals including active oxygen species in oxidation, deterioration of quality, decomposition, contamination, deodorization, and loss of freshness is one of the important factors for an expression and deterioration mechanism. As a result, there is a problem where radicals including active oxygen species lead to serious damage, such as decrease in commercial value, decrease in productivity, and increase in living/ natural environmental burdens.

Free radical scavenging agents and anti-oxidizing agents such as ascorbic acid, alpha-tocopherol, cysteine, glutathione, ubiquinone, butyl hydroxy anisol (BHA), and butyl hydroxy toluene (BHT) are known as substances for overcoming such active oxygen species-derived problems.

Nevertheless, since such anti-oxidizing agents are chemically synthesized compounds, there are problems including remaining doubts as to the safety of such substances on the antioxidation subject such as living organisms when used in large quantities. Another problem is the fact that these and similar anti-oxidizing agents become oxidized themselves through the process of reducing other substances and raises questions as to the safety of such by-product oxides on the antioxidation subject.

Accordingly, development of innovative technology that can anticipate a high benchmark of safety on the antioxidation subject and reduced environmental burden while demonstrating antioxidation capability and radical scavenging capability that is on par with or superior to for instance conventional anti-oxidizing agents has been long awaited.

The present invention has been made in order to solve such problems and aims to provide a method of antioxidation and antioxidant-functioning water that can transform an antioxidation subject, which is in an oxidation state due to a deficiency of electrons or needs to be protected from oxidation, into a reduced state where electrons are filled, by promoting the breaking reaction of molecular hydrogen that is used as a substrate included in hydrogen-dissolved water into a product of active hydrogen through a process employing a catalyst, which is a precious metal colloid or a hydrogen oxidation/ reduction enzyme (except for those already existing in the living organism), on the hydrogen-dissolved water, while anticipating high benchmarks of safety on the human body and reduced environmental burden.

In addition, the present invention aims to provide antioxidant-functioning water, which is water containing a precious metal colloid or a hydrogen oxidation/ reduction enzyme (except for those already existing in the living organism) that catalyzes the breaking reaction of molecular hydrogen used as a substrate that is included in hydrogen-dissolved water, into a product of active hydrogen, and has a function that can transform an antioxidation subject, which is in an oxidation state due to a deficiency of electrons or needs to be protected from oxidation is desired, into a reduced state where electrons are filled, by promoting the breaking reaction of a molecular hydrogen substrate included in hydrogen-dissolved water into a product of active hydrogen via a process employing the catalyst on the hydrogen-dissolved water, and an antioxidant, an anti-aging agent, an anti-deterioration agent, an anti-decomposition agent, an anti-contamination agent, a deodorant, and a freshness-keeping agent, which contain the antioxidant functioning water.

### DISCLOSURE OF THE INVENTION

Before giving a general description of the invention, the history of how the inventors arrived at the present invention is described.

### (1) History of Inventive Idea

In the previously filed and republished patent application No. WO99/10286, the contents of which are incorporated herein by reference, the applicants of the present application disclose an electrolytic cell and an electrolyzed water generation apparatus capable of independently controlling the hydrogen ion exponent (hereafter referred to as 'pH') and the oxidation/ reduction potential (hereafter referred to as 'ORP'). A synopsis of the aforementioned application is given hereinforth. Namely, a reducing potential water generation apparatus has: an electrolytic chamber to which raw water to be dissociated is supplied; at least one membrane which separates inside the electrolytic chamber from outside thereof; at least a pair of electrode plates provided inside and outside the electrolytic chamber, respectively, and sandwiches the membrane; and a power source circuit that applies a voltage between both electrodes, wherein the electrode plate provided inside the electrolytic chamber is given as the cathode and the electrode plate provided outside the electrolytic chamber is given as the anode; wherein the electrode plates provided outside the electrolytic chamber are provided in contact with the membrane or leaving a slight space. On the cathode side in the apparatus, without significantly changing the pH of the raw water, electrolyzed reducing potential water (hereafter, also referred to as 'reducing potential water') is generated having an ORP that is significantly lowered to a negative value. In the following, unless not specifically stated otherwise, 'electrolysis processing' means carrying out continuous- flow electrolysis processing using the above- mentioned reducing potential water generation apparatus under electrolysis conditions of a 5 A constant current and flow rate of 1 L/min.

The inventors herein arrived at the present invention during performance evaluation testing of reducing potential water generated with the reducing potential water generation apparatus described above.

Here, the reducing potential water has a negative ORP value, and also shows an ORP value corresponding to the pH that exceeds a predetermined value. Whether or not the ORP value exceeds the predetermined value may be determined through the following Nernst equation (approximate equation):$\text{ORP = -59pH - 80(mV)}$

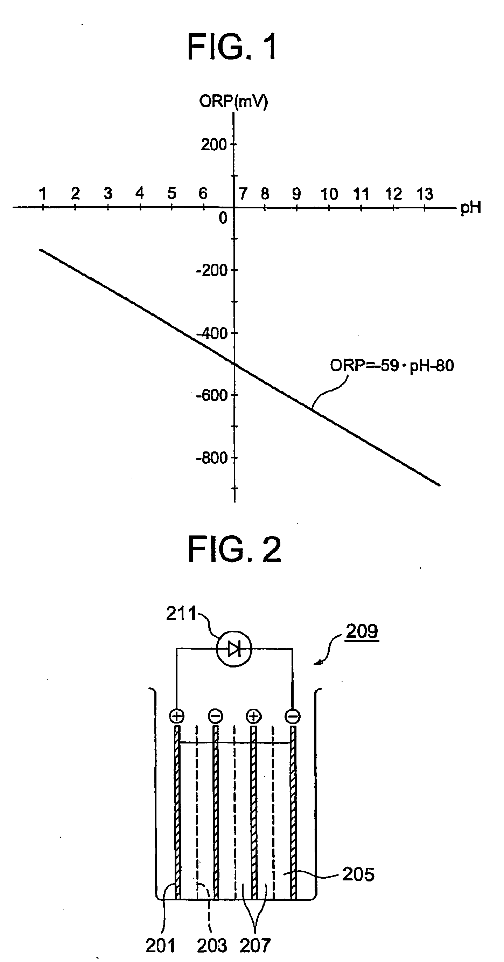

As shown in FIG. 1, this equation shows there is a proportional relationship between the pH and ORP (the ORP value falls towards negative as the pH falls towards the alkaline side). Here, the fact that the ORP value corresponding to pH shows a value that exceeds the predetermined value means that the ORP value is lower than the value according to the Nernst equation described above. It is given here that water meeting such conditions is called reducing potential water. For example, substituting pH 7 into the Nernst equation above gives an ORP of approximately -493 (mV). In other words, at pH 7, water having an ORP of approximately -493 (mV) or lower corresponds to reducing potential water. However, some difference definitely exists in the dissolved hydrogen concentration within the category of reducing potential water defined here, but this is described later together with the quantitative analysis method for this dissolved hydrogen concentration.

Therefore, a considerable amount of high-energy electrons is included in the reducing potential water. This is clearly seen when measured with an ORP meter. The ORP is an indicator showing the proportions with which oxidizing material and reducing material exist in the test water, and generally uses units of millivolts (mV). Generally with an ORP meter, a negative ORP value is observed when the measurement electrode takes a negative charge, and conversely, a positive ORP value is observed when the measurement electrode takes a positive charge. Here, in order for the measurement electrode to take a negative charge, high- energy electrons must be included in the test water. Accordingly, the fact that ORP value shows a negative value having a large absolute value can be said as meaning that the test water includes high- energy electrons.

At this point, illumination testing using a light emitting diode (hereafter abbreviated as 'LED') was carried out for performance evaluation showing to what extent high- energy electrons are included in the reducing potential water. This used the principle behind batteries. More specifically, reducing potential water having an exemplary ORP of approximately -600 (mV) and tap water having an exemplary ORP of approximately +400 (mV) were poured into the cathode chambers 205 and anode chambers 207, respectively, in a testing cell 209 configured with alternating platinum or similar electrodes 201 and membranes 203, and having about three cathode chambers and three anode chambers. Continuous illumination of an LED 211 was observed when the minus end of the LED 211 was connected to the electrode in contact with a cathode chamber 205 and the plus end of the LED 211 was connected to an anode chamber 207. This means that current is flowing from the anode of the cell 209 towards the cathode, and moreover, the fact that current is flowing means that electrons are flowing. At this point, taking into consideration the fact that the electrons flowing through the LED 211 are flowing from the cathode of cell 209 to the anode, the included high- energy electron groups in the reducing potential water are quantitatively evaluated through testing.

As reference examples, alkaline electrolyzed water generated by a commercially available electrolyzed water generation apparatus (exemplary ORP of approximately -50 mV), or natural mineral water, etc, was poured into the cathode chambers and tap water was poured into the anode chambers. However, in this case, continuous illumination of the LED was not observed when the minus end of the LED was connected to the electrode in the cathode chamber and the plus end of the LED is connected to the anode chamber in a manner similar to that described above. This is thought as happening because not enough high- energy electron groups to illuminate the LED are included in the existing alkaline electrolyzed water or natural mineral water.

In addition, even if flow were to be reduced and the ORP value shifted significantly towards the negative with a commercially available electrolyzed water generation apparatus, should the absolute value of the ORP value occurring at the pH level at that time be small in accordance with the above- mentioned Nernst equation, no illumination of the LED would naturally be observed. With for example the commercially available electrolysis generation device, even if the pH is approximately 10 and the ORP value is in the range of -500 to -600 (mV) as a result of reducing the flow, since the ORP value as a percentage of the pH level becomes small, it may be considered as becoming weak in terms of the electron energy, and as long as ORP value fails to be brought down to at least approximately -670 (mV) or lower when the pH level is approximately 10, it is impossible to illuminate the LED.

In addition, there are several varieties of LEDs. In particular, when a diode showing for example a blue or green color that requires a high inter-terminal voltage of approximately 3V or higher was used, continuous illumination of such diode was observed when using a cell 209 having each chamber arranged in a three-layer alternating structure as described above.

Therefore, as eager research progressed on the industrial applicability of having high- energy electrons included in reducing potential water, a hint was received that wondered if it was possible that the reducing potential water had latent reducing power'. In particular, the reducing potential water had quite strong reducing power since the ORP value had fallen to a appreciably negative value that was significant enough cause the LED to illuminate, which led to the feeling that if this reducing power be could tapped there may be applications over a wide range of industrial fields including health care, manufacturing, food, agriculture, automobile, and energy.

What state this 'latent reducing power' is in is now described. For instance, if a reducing agent such as vitamin C (ascorbic acid) is added to ordinary tap water, and thereafter an oxidizing agent is further added, the reducing agent immediately reduces the oxidizing agent. On the other hand, if an oxidizing agent is added to reducing potential water, the oxidizing agent is not immediately reduced at all. Conditions at this point may be considered as including both the significant negative ORP value for the reducing potential water remaining the same, as well as the oxidizing agent also maintaining the same conditions. At this point in time reducing power has not yet been exhibited.

That is, no matter how much the high- energy electrons try to exist in the reducing potential water, or to put it another way, no matter how large and negative the value of the ORP is, it comes up against the fact that the reaction where electrons are immediately released from the reducing potential water to reduce the oxidizing agent does not occur. Therefore, it was thought that the magnitude of the electron energy included in the reducing potential water and how easily the electrons are released or the exhibition of reducing power are probably two separate issues.

So what should be done to make the reducing potential water exhibit reducing power? As the inventors continued with their eager research into this proposition, the idea of using some sort of catalyst hits them with a flash of light. While there are many types of catalysts, with the particular premise of for instance use in living organisms, the idea was conceived that some sort of enzyme or a precious metal colloid (minute particle of a precious metal cluster), which is described later, might be used as the catalyst.

Here, the particular mention of an enzyme is for an enzyme-acting substance that is a chemical reaction catalyst, and the activity of the enzyme is measured by the speed of the catalyzing reaction. In the case of catalyzing the reaction of A→B, A is the substrate and B is the product. Applying this to the case of the present invention, the molecular hydrogen included in the hydrogen- dissolved water corresponds to the substrate, and the active hydrogen corresponds to the product. Also, it is thought that the working- action mechanism of such enzyme can be described in the following manner.

It is assumed here that it is necessary for the high- energy electron group included in the reducing potential water to come into contact with the oxidizing agent and reduce this oxidizing agent. There is an energy wall that this electron group included in the reducing potential water must surpass in order for the electron group to migrate to the oxidizing agent. This energy wall is commonly called a 'potential barrier', 'activation energy', or the like. The higher this energy is, the higher the height of the wall that needs to be surpassed becomes. Also, the energy that can be expressed with the height of this wall is larger than the energy of the electron group; therefore the electron group is normally not able to climb over this wall and as a result does not migrate to the oxidizing agent. In short, it is thought that the oxidizing agent cannot be reduced.

However, the activation energy corresponding to the height of the wall may be lowered if for instance a catalyst such as an enzyme is used. As a result, the electron group included in the reducing potential water is able to migrate to the oxidizing agent rather smoothly compared to when no catalyst is used, and at the endpoint where this migration is complete, the reducing potential water is able to reduce the oxidizing agent.

In this manner, when an enzyme or similar catalyst is used, the high-energy electron group included in the reducing potential water can be more easily released, and results in the reducing power being exhibited. In other words, this is what is meant by the reducing potential water 'having latent reducing power', which may be rephrased as 'the reducing power held by the reducing potential water is kept under seal'. These various thought processes led to the idea that 'the key to lifting the seal on the reducing power held by the reducing water is a catalyst.'

Now that the history of the idea of the invention has been elucidated, a synopsis of the invention will be described.

### (2) Synopsis of Invention

### (2-1) Antioxidation Method

The present invention provides an antioxidation method that includes transforming an antioxidation subject, which is in an oxidation state due to a deficiency of electrons or needs to be protected from oxidation, into a reduced state of electrons being filled, by promoting the breaking (activating) reaction of molecular hydrogen used as a substrate included in hydrogen- dissolved water into a product of active hydrogen via a process employing a catalyst, which is a precious metal colloid or a hydrogen oxidation/ reduction enzyme (except for those already existing in the living organism), on the hydrogen- dissolved water.

The inventors are confident that the substance that provides the negative value for the ORP value of hydrogen- dissolved water such as electrolyzed water or hydrogen bubbling water is the hydrogen that is dissolved in that water. The fact that hydrogen is the ultimate reducing substance, and furthermore, the fact that hydrogen develops on the cathode side during electrolysis processing serves as proof of this conviction.

Nevertheless, as made clear in the history of the idea behind the invention, with the hydrogen- dissolved water as it is, the reducing power is normally kept under seal.

Therefore, in order to cast off the seal on the reducing power held by the hydrogen- dissolved water, as defined with the antioxidation method according to the present invention, it has been found that the step of using a catalyst in the hydrogen- dissolved water is extremely important.

Another important factor is the existence of an antioxidation subject. If there is no antioxidation subject, then there is no stage for the antioxidation action according to the present invention to be exhibited. In other words, the important factors in the present invention are 1) the hydrogen- dissolved water, 2) the catalyst, and 3) the antioxidation subject. When these three factors are organically combined, the seal on the reducing power latently held by the hydrogen is cast off to allow manifest expression of the broad antioxidation function including the reducing function. It should be noted that the expression of the antioxidation function spoken of in the present invention is the reduced state of electrons being filled in the antioxidation subject, which is either in an oxidized state due to a deficiency of electrons or needs to be protected from oxidation. In addition, the reduced state of electrons being filled in the antioxidation subject is a concept including both a case of reducing the antioxidation subject in an oxidized state, and a case of reducing an oxidizing material itself such as active oxygen species, which attempts to oxidize the antioxidation subject that needs to be protected from oxidation.

While magnitude of the reducing power here may be estimated to a certain extent through, for example, the condition of the ORP value (i.e. the stability of the ORP reading or the relationship with the above-mentioned Nernst equation), ultimately it is determined depending on the effective value of the dissolved hydrogen concentration DH found using the dissolved hydrogen concentration quantitative method (described later) that uses an oxidation/ reduction pigment.

Next, the technical scope that is assumed for the present invention regarding these three factors will be laid out.

### (2-1-1) Hydrogen Dissolved Water

Hydrogen dissolved water is assumed to be any water in which there is included hydrogen. In addition, what is called water here (also referred to as raw water) includes all waters including tap water, purified water, distilled water, natural water, activated charcoal processed water, ion exchange water, deionized water, ultra pure water, commercially available (PET) bottled water, biological fluid (described later), and water in which molecular hydrogen is generated through a chemical reaction in the water. Furthermore, all water that includes an auxiliary agent for electrolysis or a reducing agent added to such water also falls within the technical scope of the present invention. Moreover, as long as it meets the condition of being water in which there is included hydrogen, it does not matter if the water is acidic, neutral, or alkaline, nor does it particularly matter if the dissolved concentration is high or low. However, since the antioxidation function expressed through application of the present invention emanates from the electrons released through the process of replacing molecular hydrogen with active hydrogen through a catalyst, more significant expression of the antioxidation function may be expected with a higher dissolved concentration of molecular hydrogen.

Moreover, hydrogen- dissolved water also includes either electrolyzed water generated on the cathode side when raw water is subjected to electrolysis processing between an anode and a cathode via a membrane, or water processed through bubbling (aeration) or pressurized filling of hydrogen into raw water. The definition is made in this way in order to make clear that electrolyzed water such as 'alkaline ion water' that is produced through existing continuous flow-type or batch electrolyzed water generation apparatus as well as hydrogen- dissolved water generated by including hydrogen in raw water through external manipulation also fall within the technical scope of the present invention. Those given as hydrogen- dissolved water here are merely examples and are not intended to mean that they are limited to this. Accordingly, it should be made clear now that even if using for instance natural water and hydrogen is included therein, this does not mean that such water falls outside of the technical scope of the present invention.

In addition, molecular hydrogen thought as being generated by enteric microorganisms, particularly microorganisms that contain hydrogenase, is dissolved inside bodily fluids (also referred to as biological fluids) such as the blood or lymphatic fluid of living organisms. Hydrogen dissolved water mentioned in the present invention, regardless of origin, also includes biological fluid in which molecular hydrogen is dissolved, and as such falls within the technical scope thereof. It should be noted that the location of the molecular hydrogen occurring in the living organism does not remain within the intestinal tract, but is also absorbed from the intestines and distributed through blood. This molecular hydrogen that has entered the blood flow is thought to be transported to each of the internal organs such as the liver and kidneys, and stored in the various parts of the body. In this case, the activation of molecular hydrogen should be facilitated by administering an enzyme such as hydrogenase or a precious metal colloid (described later) to the living organism in order to utilize the molecular hydrogen existing in the living organism as a reducing agent.

However, hydrogen- dissolved water also includes reducing potential water where the ORP is a negative value, and the ORP value corresponding to the pH shows a value that is lower than the value according to the Nernst equation or ORP = -59 pH - 80 (mV). The reducing potential water mentioned here naturally includes electrolyzed reducing potential water generated with the reducing potential water generation apparatus developed by the applicants herein that has: an electrolytic chamber to which deionized raw water is supplied; at least one membrane which separates inside the electrolytic chamber from outside thereof; at least a pair of electrode plates provided inside and outside the electrolytic chamber, respectively, and sandwiches the membrane; and a power source circuit that applies a voltage between both electrodes, wherein the electrode plate provided inside the electrolytic chamber is given as the cathode and the electrode plate provided outside the electrolytic chamber is given as the anode; wherein the electrode plates provided outside the electrolytic chamber is provided in contact with the membrane or leaving a slight space, and it should be made clear now that this also includes water that while generated with an apparatus other than such apparatus meets the conditions for reducing potential water described above. It should be now added that in the case of employing a circulating electrolysis processing technique in the reducing potential water generation apparatus wherein water that has been generated is again introduced into the electrolytic cell (electrolysis chamber) so as to circulate, and then repeating this circulatory process for a predetermined length of time, as shown for instance in the following Table 1, reducing potential water may be obtained having a high dissolved- hydrogen concentration and an even lower ORP value, and superior reducing power (antioxidizing power) may be expressed with such reducing potential water.

Furthermore, it is preferable that the dissolved hydrogen water be water in which the dissolved hydrogen concentration is greater than the saturation concentration (in terms of effective value of dissolved hydrogen concentration value found using a dissolved hydrogen concentration quantitative analysis method that uses oxidation/ reduction pigment) under atmospheric pressure. This is because high benchmarks of the reduction activity and antioxidation activity emanating from the antioxidant-functioning water according to the present invention can be anticipated.

Therefore, the respective physical quantities of reference examples of hydrogen- dissolved water assumed by the inventors and comparative examples of water in which no hydrogen is dissolved are now given. Activated charcoal processing water resulting from processing Fujisawa City tap water through an activated charcoal column, Organo purified water resulting from processing Fujisawa municipal tap water through a ion exchange column made by Organo Corporation, and an example of (PET) bottled water: 'evian' (registered trademark of S.A. des Eaux Minerales d' Evian), which is supplied in Japan through Calpis Itochu Mineral Water Co., Ltd., are given as examples of subject water for purposes of comparison. A first reducing potential water subjected to continuous electrolysis processing using electrolysis conditions of a 5A constant current and flow rate of 1 L/min in the reducing potential water generation apparatus developed by the applicants herein, and a second reducing potential water subjected to continuous circulating electrolysis processing for 30 minutes using the same electrolysis conditions (volume of circulatory water: 2 liters) in the same apparatus are given as examples of each type of post-processing hydrogen-dissolved water for the purpose of dissolving hydrogen in such comparative subject waters. In addition, hydrogen gas bubbling water subjected to hydrogen gas bubbling processing for 30 minutes, and alkaline electrolyzed water subjected to continuous electrolysis processing using electrolysis conditions of electrolysis range '4' with a standard amount of water in a 'Mini Water' electrolyzed water generation apparatus made by MiZ Co., Ltd. are given as examples vis-à-vis each type of comparative subject water.

Furthermore, pH, oxidizing/ reducing potential ORP (mV), electrical conductance EC (mS/ m), dissolved oxygen concentration DO (mg/ L), dissolved hydrogen concentration DH (mg/ L), and water temperature T (°C) are given as the various physical properties in such waters. In addition, the various types of gages used to measure these physical properties include the following: the pH meter (including a temperature gage) is a model D-13 pH meter made by Horiba, Ltd. with a model 9620-10D probe for the same, the ORP meter is a model D-25 ORP meter made by Horiba, Ltd. with a model 9300-10D probe for the same, the EC meter is a model D-24 EC meter made by Horiba, Ltd. with a model 9382-10D probe for the same, the DO meter is a model D-25 DO meter made by Horiba, Ltd. with a model 9520-10D probe for the same, and the DH meter (dissolved hydrogen meter) is a model DHD I-1 made by DKK-TOA Corporation with a model HE-5321 electrode (probe) and model DHM-F2 repeater for the same. The various physical properties of the comparative subject waters were respectively measured using these types of gages.

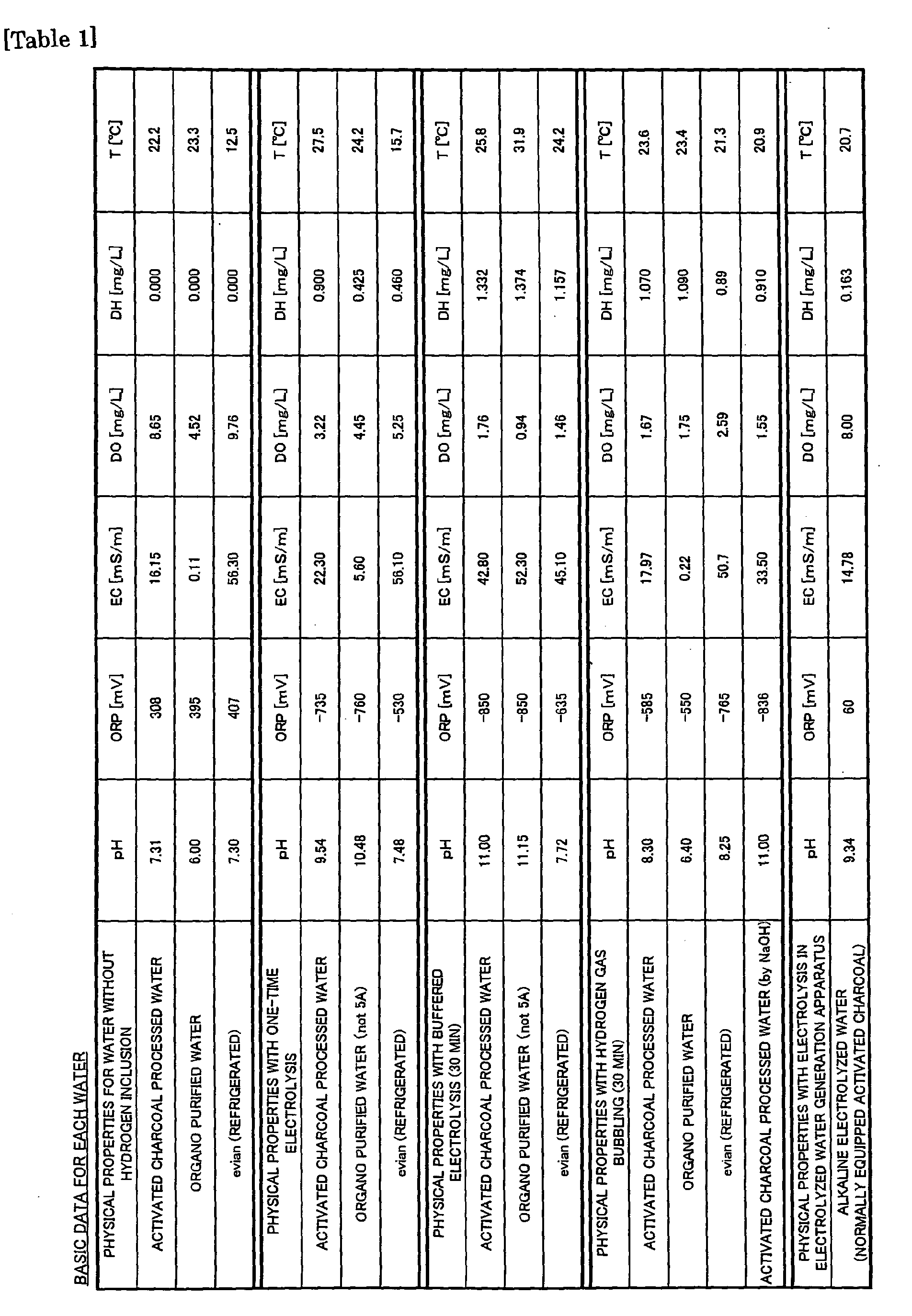

According to this Table 1, focusing on the dissolved hydrogen concentration (DH) measured with the dissolved hydrogen meter, with the first reducing potential water subjected to one-time electrolysis processing using the reducing potential water generation apparatus, despite the fact that the electrolyzed water was instantly removed, it was found that a high concentration of hydrogen ranging between 0.425 and 0.900 (mg/ L) was dissolved therein.

In addition, in the case of the length of processing time being, for example, 30 minutes, comparing the dissolved hydrogen concentrations of the buffered electrolyzed reducing potential water (the second reducing potential water) in this reducing potential water generation apparatus and the hydrogen gas bubbling water, while the latter ranged between 0.89 and 1.090 (mg/ L), the former showed that a high concentration of hydrogen ranging between 1.157 and 1.374 (mg/ L) could also be dissolved therein.

Meanwhile, it is preferable that at least one reducing agent selected from the group consisting of sulfite, thiosulfate, ascorbic acid, and ascorbate be added as required to the hydrogen- dissolved water. This is because it is preferable that the dissolved oxygen concentration in the hydrogen-dissolved water be made as low as possible (as the dissolved oxygen concentration in the hydrogen-dissolved water becomes lower, preference increases, such as in the following order: 0 mg/L, 0.5 mg/ L or lower, 1 mg/ L or lower, 1.5 mg/L or lower, and 2 mg/L or lower) when it is necessary to prevent rapid oxidation due to the dissolved oxygen of the active hydrogen occurring through the action of the catalyst.

To further explain this, in hydrogen- dissolved water where a catalyst has been used, it is possible to reduce the dissolved oxygen concentration DO (mg/ L) to nearly zero (mg/ L) when the amount of reducing agent added is less than the chemical equivalent capable of exactly reducing the dissolved hydrogen.

As a comparative example for this, when the same amount of reducing agent was added to hydrogen- dissolved water where a catalyst had not been used, significant reduction in the dissolved oxygen concentration DO (mg/ L) was not achieved. This is thought to be the result of the intrinsic reducing power held by the hydrogen- dissolved water on which the seal had been lifted bringing out the reducing power held by the reducing agent more strongly.

Accordingly, it should be added that in the case of bottling .antioxidant- functioning water according to the present invention in the condition where both a reducing agent and a dissolved additive such as a vitamin coexist, there is also the dimension that such an additive causes the antioxidizing action intrinsically held by the additive to be brought out even more strongly, and amplification activity can be expected as a result of being in an antioxidizing environment. This is because when antioxidant-functioning water according to the present invention is bottled in the condition where both a reducing agent and the exemplary reducing ascorbic acid coexist, it means that the ascorbic acid causes the antioxidizing action intrinsically held by the reducing ascorbic acid to be brought out even more strongly as a result of continuing to be in reducing form due to being in an antioxidizing environment. In this case, it is preferable that the reducing agent such as the exemplary reducing ascorbic acid be added in an amount greater than that required to reduce/ neutralize the oxidizing material such as dissolved oxygen in the coexistent system. However, it is preferable that an appropriate amount of additive ascorbic acid be added in consideration of the pH expressed by the antioxidant- functioning water and the minimum recommended daily amount that should be ingested.

### (2-1-2) Catalyst

The catalyst is assumed to be all those having the function of catalyzing the breaking reaction of the molecular hydrogen used as a substrate included in the hydrogen- dissolved water into a product of active hydrogen. More specifically, the essential qualities of the catalyzing function according to the present invention lies in smoothly accelerating the activation of molecular hydrogen, and within such function, accepting electrons from the molecular hydrogen (by activating one molecular hydrogen, two electrons are obtained or H2 → 2e- + 2H+) and donating the accepted electrons to the antioxidation subject following temporary pooling (including the idea of absorption or occlusion into the catalyst) or without pooling. It should be noted that donating the electrons to the antioxidation subject is a concept including both a case of reducing the antioxidation subject, which is in an oxidized state, and a case of reducing an oxidizing material itself such as active oxygen species that attempts to oxidize the antioxidation subject, which needs to be protected from oxidation.

The catalyst according to the present invention may be, for example, a hydrogen oxidation/ reduction enzyme. Furthermore, a hydrogenase, a precious metal colloid (described later), or one of the electromagnetic waves selected from the group consisting of visible light, ultraviolet light, and electron beams also falls within the technical scope. It should be noted that the precious metal colloid assumed with the present invention means the inclusion of platinum, palladium, rhodium, iridium, ruthenium, gold, silver, or rhenium, along with the respective salts thereof, alloy chemical compounds, or colloid molecules themselves such as complex chemical compounds, as well as mixtures of these. When making or using these precious metal colloids, reference should be made to the contents of 'Fabrication and Use of Pt Colloids (Pt koroido no tsukurikata to tsukaikata)' (NANBA, Seitaro and OKURA, Ichiro); Hyomen Kagaku (Surface Science) Vol. 21; No. 8 (1983), the contents of which are included herein by reference. In addition, the colloid mentioned in the present invention is assumed as having molecules with diameters ranging between 1 nm and 0.5 µm, which is said as showing innate behavior of a general colloid. However, when employing the exemplary Pt colloid as the precious metal colloid, it is considered proper to use a molecular diameter that increases the catalytic activity of this Pt colloid, preferably ranging between 1 and 10 nm and more preferably between 2 and 6 nm. This is, as written in the above-mentioned 'Fabrication and Use of Pt colloids' by Nanba and Okura, the molecular size is derived from the trade- off relationship between the fact that the innate property is expressed as a precious metal and the fact that the surface area is increased to improve the catalytic activity. However, the colloids mentioned in the present invention are in accordance with the definition proposed by Staudinger of Germany that 'colloids are configured with between 103 and 109 atoms.' Moreover, the precious metal colloid according to the present invention preferably has a round molecular shape in order to increase the surface area. Here, since the fact that the surface area of the precious metal colloid is large means increased opportunities for connection with the molecular hydrogen used as the substrate, it is superior from the viewpoint of catalytic function expressed by the precious metal colloid.

Moreover, a catalyst includes the idea of electron carriers such as a coenzyme that assists the functioning thereof, inorganic compounds, and organic compounds.

It is preferable that such an electron carrier have properties capable of efficiently accepting electrons from hydrogen, a hydrogen oxidation/ reduction enzyme, a hydrogenase, or a precious metal colloid, which are all electron donors, and at the same time, efficiently carrying electrons to the antioxidation subject, which is an electron acceptor. To put it more simply, the electron carrier acts to transport the hydrogen (electron).

### (2-1-2-1) Candidates for the Electron Carrier

In the following, candidates for the electron carrier are now given. It should be noted that it does not matter if the electron carrier is oxidizing or reducing. Since the reducing electron carrier has surplus electrons beforehand, it is beneficial from the viewpoint of easily releasing electrons.
(a) Methylene blue (normally oxidizing)
   methylthionine chloride, tetramethylthionine chloride

   chemical formula = C₁₆H₁₈ClN₃S·3(H₂O)

   Reducing methylene blue is referred to as leucomethylene blue.
(b) Pyocyanin

   chemical formula = C₁₃H₁₀N₂O

   One of the antibiotic substances produced by Pseudomonas aeruginosa. Pyocyanin performs reversible oxidation/ reduction reactions, and there are two types of the oxidizing type: one that is alkaline and a blue color, and one that is acidic and a red color. In addition, the reducing type is colorless, as is the reduced methylene blue (leucomethylene blue).
(c) Phenazine methosulfate
   abbreviation = PMS

   chemical formula = C₁₄H₁₄N₂O₄S

   Phenazine methosulfate tends to easily photo-decompose.
(d) 1 ― Methoxy PMS
   Is stable when exposed to light and was developed as a substitute for the PMS mentioned above that is unstable when exposed to light.
(e) Chemical compounds including the iron (III) ion
   Many exist such as FeCl₃, Fe₂(SO₄)₃, and Fe(OH)₃. The intrinsic purpose is as a reagent for obtaining Iron (III) or Fe (³⁺) as an ion. In living organisms, it is thought as existing as heme iron in the hemoglobin of red blood cells. It should be noted that heme iron has characteristics that are different from the independent iron ion.
   In particular, when acting with ascorbic acid, since it produces a hydroxyl radical (·OH) having strong oxidizing power, the iron ion is not always required when in vitro. However, in vivo, when the iron ion coexists with nitric oxide (NO), it is said that it does not always generate the hydroxyl radical (·OH).
   In particular, although the iron (II) ion Fe (²⁺) is the reduced form of the iron (III) ion Fe (³⁺), there are many occasions where even with the reduced form, the oxidizing action is accentuated. In particular, if there is lipid peroxide, a radical chain reaction may easily occur. When the iron (III) ion Fe (³⁺) is reduced through ascorbic acid or the like, a radical generating chain reaction occurs if it coexists with lipid peroxide. In other words, it may be considered as producing many lipid radicals and having a negative effect on living organisms.
(f) Reduced ascorbic acid (chemical formula = C₆H₈O₆)
   Exists in living organisms, but it is absorbed from outside the body, and is not synthesized by humans.
(g) Glutathione (chemical formula = C₁₀H₁₇N₃O₆S)
   abbreviation = GSH
   Is an SH chemical compound existing in large quantities in living organisms, and it is thought that humans have a gene for synthesizing this. Glutathione is a poly-peptide configured from three amino acids (glutamic acid - cysteine - glycin = Glu-Cys-Gly), a coenzyme of glyoxylase, and is known to function as an intracellular reducing agent, an anti-aging agent, and the like. In addition, glutathione has the function of directly (nonenzymatically) reducing oxygen (O₂).
(h) Cysteine (Cys)
   One of the amino acids and an SH chemical compound, it is ingested as a protein and is the final product of digestive decomposition. Cysteine is a structural component of the above-mentioned glutathione and is an amino acid having an SH group. As with glutathione, two cysteines (Cys) respectively release one hydrogen atom, and become oxidized cysteine through a disulfide bond (-s-s-).
(i) Benzoic acid (C₇H₆O₂)
   Rarely exists in living organisms, strawberries include approximately 0.05%. Benzoic acid is a basic reducing agent and has the function of nonenzymatically and effectively scavenging the hydroxyl radical and making it into water.
(j) p-amino Benzoic acid (C₇H₇O₂)
(k) Gallic acid (C₇H₆O₅) (3,4,5-trihydroxy benzoic acid)

Widely exists in leaves, stems, and roots of plants, and is used as a general hemostatic agent and an antioxidant (preservative) in food (food additive). This alkaline solution has particularly strong reducing power. Gallic acid tends to react easily with oxygen.

It should be noted that those given as catalysts here are merely examples, and it is not intended to mean that they are limited to these. Accordingly, as long as contributing to the catalyzing reaction assumed by the present invention, it should be clearly noted that it does not mean that other parameters such as physical external forces including temperature, pressure, ultrasonic waves, or agitation may be excluded.

In addition, it should be added that the product of active hydrogen comprehensively includes atomic hydrogen (H•) and hydride ions (H-).

Moreover, catalysts such as those described here may be each used independently, or as needed, may be used in an appropriate mixture of a plurality of these. Basically, electrons are transmitted in the order of the hydrogen- dissolved water to catalyst to antioxidation subject, however, besides this the following orders may also be considered: the hydrogen-dissolved water to enzyme (hydrogenase) to antioxidation subject, the hydrogen- dissolved water to electron carrier to antioxidation subject, the hydrogen- dissolved water to enzyme (hydrogenase) to electron carrier to antioxidation subject, the hydrogen- dissolved water to precious metal colloid to antioxidation subject, or the hydrogen- dissolved water to precious metal colloid to electron carrier to antioxidation subject. In addition, it is possible to use such electron carrier system in combination with at least one of the electromagnetic waves selected from the group consisting of visible light, ultraviolet light, and electron beams.

### (2-1-3) Antioxidation Subject

An antioxidation subject is assumed to be any subject in an oxidized state due to a deficiency in electrons or for which protection from oxidation is desired. It should be noted that oxidation mentioned here means the drawing away of electrons from a subject through the direct or indirect action of oxygen, heat, light, pH, ions, etc. In addition, to be more specific, an antioxidation subject includes for instance cells or organs of living organisms, or antioxidation substances such as vitamins, food, unregulated drugs, medical supplies, cosmetics, animal feed, oxidation/ reduction pigments (to be described later), as well as water itself, all fall within the technical scope of the present invention. It should be noted that these given as antioxidation subjects here are merely examples and it should be clearly stated here that is not intended to mean that they are limited to these.

Next, the relationship between a catalyst and an antioxidation subject is described from the standpoint of the catalyst.

With the present invention, the catalyzation of the breaking reaction of the molecular hydrogen used as a substrate included in the hydrogen-dissolved water into a product of active hydrogen is performed with for example a hydrogen oxidation/ reduction enzyme, hydrogenase, or a precious metal colloid.

The reducing potential water to which a hydrogen oxidation/ reduction enzyme such as the exemplary hydrogenase (i.e., hydrogenase catalyst-added antioxidant-functioning water) is added is now considered. In the case of a hydrogenase-added low alkaline reducing potential water being ingested through drinking, and oxidizing agents such as active oxygen species coexisting in digestion- related cells (antioxidation subjects) of the living organism such as those of the intestines, these oxidizing agents are immediately reduced. In addition, when other additives such as fruit juice or a vitamin species (antioxidation subjects) coexist, the reducing potential water acts as an antioxidizing agent on these additives under the condition of hydrogenase coexisting. Such action mechanism is considered to include the molecular hydrogen dissolved in the reducing potential water dissociating and activating the two atomic hydrogens (H•) through the hydrogen- breaking action of the hydrogenase, the formed atomic hydrogen (H•) splitting into protons and electrons in the water, and the formed electrons then being donated to the antioxidation subject. Here, donating the electrons to the antioxidation subject is a concept including both a case of reducing the antioxidation subject, which is in an oxidized state, and a case of reducing an oxidizing material itself such as active oxygen species that attempts to oxidize the antioxidation subject, which needs to be protected from oxidation.

The reducing potential water to which a precious metal colloid such as platinum (Pt) or palladium (Pd) colloidal particles is included (that is, precious metal colloid catalyst-added antioxidant-functioning water) is also considered. In the case of a low alkaline reducing potential water added with Pt colloid or Pd colloid being ingested through drinking, and oxidizing agents such as active oxygen species coexisting in digestion related cells (antioxidation subjects) of the living organism such as those of the intestines, these oxidizing agents are immediately reduced. In addition, when other additives such as fruit juice or a vitamin species (antioxidation subjects) coexist, the reducing potential water acts as the antioxidizing agent of these additives under the condition of Pt colloid or palladium (Pd) coexisting. Such action mechanism is considered to include the molecular hydrogen-dissolved in the reducing potential water dissociating and activating the two atomic hydrogens (H•) along and being adsorbed into the minute particle surface of the Pt colloid or palladium (Pd), the formed atomic hydrogen (H•) splitting into protons and electrons in the water, and the formed electrons then being donated to the antioxidation subject. Here, as with the case of the above-mentioned hydrogenase catalyst-added antioxidation function water, donating the electrons to the antioxidation subject is a concept including both a case of reducing the antioxidation subject, which is in an oxidized state, and a case of reducing an oxidizing material itself such as active oxygen species that attempts to oxidize the antioxidation subject, which needs to be protected from oxidation.

This sort of antioxidation function is expressed only when the three items--hydrogen- dissolved water such as the reducing potential water, the hydrogen oxidation/ reduction enzyme hydrogenase or the precious metal colloid used as a catalyst, and the antioxidation subject such as the digestive system cell of the living organism--come together. In other words, the reducing power is only expressed when necessary and has no operational effect when not required. Moreover, when looking at the chemical component composition, reducing potential water, for instance, is nothing more than very ordinary water obtained by electrolyzing raw water. Accordingly, the fact that even after expressing reducing power, the water only acts as ordinary water and imparts no negative side effects onto, for example, the living organism is especially noteworthy. To restate this in another way, the fact that the positive effects aimed for may be obtained without the any negative effects or side effects is the critical difference from conventional antioxidizing agents and radical scavenging agents.

Here, quoting the thesis of HIGUCHI, Yoshiki, an associate professor at the Faculty of Science at Kyoto University Graduate School, entitled 'X-ray Structural Chemistry of Hydrogen Oxidation/ Reduction Enzymes (Suiso sanka kangen kouso no Xsen kouzou kagaku)', SPring-8 Information; Vol. 4; No. 4; July 1999, research results were announced as follows: "Hydrogen oxidation/ reduction enzymes are referred to as hydrogenase, which are proteins that are widely seen in bacteria. While generally metallic proteins containing iron, nickel or the like, recently a new hydrogenase that contains none of these metals has been discovered. Electrons occurring through the breaking of hydrogen by this molecule are used to facilitate various oxidation/ reduction reactions in the bacteria. In addition, since the proton concentration gradient at the surface layer of the cell membrane is directly governed inside and outside of the membrane, it may be thought as playing an important role in the energy/ metabolic system within the bacteria including one related to the ATP synthesis/ disassembly enzyme." In a separate thesis entitled 'X-ray Crystallography of Hydrogenase Structure Through Multi-wavelength Abnormal Dispersion with Emitted Light (Hoshakou wo mochiita tahachou ijoubunsanhou niyoru hidorogenaaze no Xsen kesshou kouzou kaiseki)', the same researcher announced the following research results: 'The main enzyme of the chain of reactions for an organism to obtain energy is the ATP synthesis/ disassembly enzyme.' It is well known that in order to activate this enzyme, it is necessary for the proton concentration gradient to be built both inside and outside the cell membrane. The hydrogenase is a membrane protein existing in the surface layers of the cell membrane and has the function of catalyzing the oxidation/ reduction of the molecular hydrogen near the membrane. Namely, this hydrogenase directly governs the proton concentration gradient inside/ outside the membrane and controls the function of the ATP synthesis/ disassembly enzyme. Accordingly, it is likely that the hydrogenase plays an extremely important role in facilitating the energy/ metabolic system in the organism. Revealing the three-dimensional structure of the hydrogenase has significant meaning because it will unravel the relationship between the structure and function of the portion related to energy/ metabolism, the most important of the life-sustaining mechanisms.'

The inventors herein, focused especially on 'hydrogenase directly governs the proton concentration gradient inside/ outside the membrane and controls the function of the ATP synthesis/ disassembly enzyme. Accordingly, it is likely that the hydrogenase plays an extremely important role in facilitating the energy/ metabolic cycle in the organism.' This was because the fact that the hydrogenase has such effect on the organism could be considered as proof that it (hydrogenase) may have the effect of facilitating the energy/ metabolic system due to the improved proton concentration gradient as well as expressing antioxidation function at the cell level when the antioxidation method, antioxidant- functioning water, and the living organism- applicable fluid according to the present invention are applied to living cells.

Accordingly, the hydrogen oxidation/ reduction enzyme, hydrogenase, and precious metal colloid according to the present invention can be thought of as opening the way for pharmaceuticals/ medical supplies that prevent, improve, and treat illnesses related to/ caused by monocyte/ macrophage system cellular functions, in particular, medical conditions or malfunctioning of an organ or system and illnesses related to/ caused by the increase or decrease in macrophage system cellular functions.

Specific examples of pharmaceuticals or medical products are as follows. Namely, since water generally has properties that allow it to immediately reach every location in the body including fatty membranes, cellular membranes, and the blood- brain barrier, curative effects in damaged portions may be expected by delivering hydrogen oxidation/ reduction enzyme hydrogenase or a precious metal colloid together with or separate from the hydrogen- dissolved water to the damaged portions of the living cells caused by activated oxygen through maneuvers such as an injection, intravenous drip, or dialysis.

The hydrogen oxidizing/ reducing enzyme hydrogenase here is a protein, and when assuming this is delivered to the damaged portion of the body via a maneuver such as an injection, intravenous drip, or dialysis, there is a danger that the body's immune system will recognize this as being foreign and cause an antigen antibody reaction. In order to resolve this problem, the oral tolerance principle of the body should be clinically applied. Oral tolerance refers to the antigen- specific T/B cell non- responsiveness to a foreign antigen that enters through oral/ enteral means. Simply put, oral tolerance is the phenomena where even if a substance ingested orally is a protein that may become, for example, an antigen, and if it is absorbed from the small intestine, the immune tolerance allows it. Treatment using this principle has already been tested. Accordingly, through clinical application of the principle of oral tolerance, a new door of antioxidation may be opened in clinical strategy.

### (2-2) Antioxidant- Functioning Water and Usage of the Same

The present invention provides antioxidant-functioning water, which is hydrogen-dissolved water containing a precious metal colloid or a hydrogen oxidation/ reduction enzyme (except for those already existing in the living organism) that catalyzes the breaking reaction of molecular hydrogen used as a substrate into a product of active hydrogen, and has an antioxidation function of transforming an antioxidation subject, which is in an oxidation state due to a deficiency of electrons or needs to be protected from oxidation, into a reduced state of electrons being filled, by promoting the breaking reaction via the catalyst.

Of the three important factors in the present invention, since the dissolved hydrogen water and a catalyst are included in the antioxidant-functioning water employing this constitution, when put in contact with the antioxidation subject, the seal on the reducing power latently held by the hydrogen is cast off to allow expression of the antioxidation function specific to the present invention. It should be noted that the antioxidation function, which transforms an antioxidation subject into a reduced state of electrons being filled, is a concept including both a case of reducing the antioxidation subject, which is in an oxidized state, and a case of reducing an oxidizing material itself such as active oxygen species that attempts to oxidize the antioxidation subject, which needs to be protected from oxidation.

However, in the case of antioxidant- functioning water that adopts the aforementioned constitution being ingested through drinking, and for instance, the large intestine being the antioxidation subject, there is a problem where it is impossible to achieve the primary objective since almost all of latent reducing power of the hydrogen is unsealed before reaching the large intestine.

Therefore, it is preferable that processing or manipulation be employed on the hydrogen oxidation/ reduction enzyme, hydrogenase, or precious metal colloid used as a catalyst in order to adjust the activation time and/ or the reaction time of the catalyst.

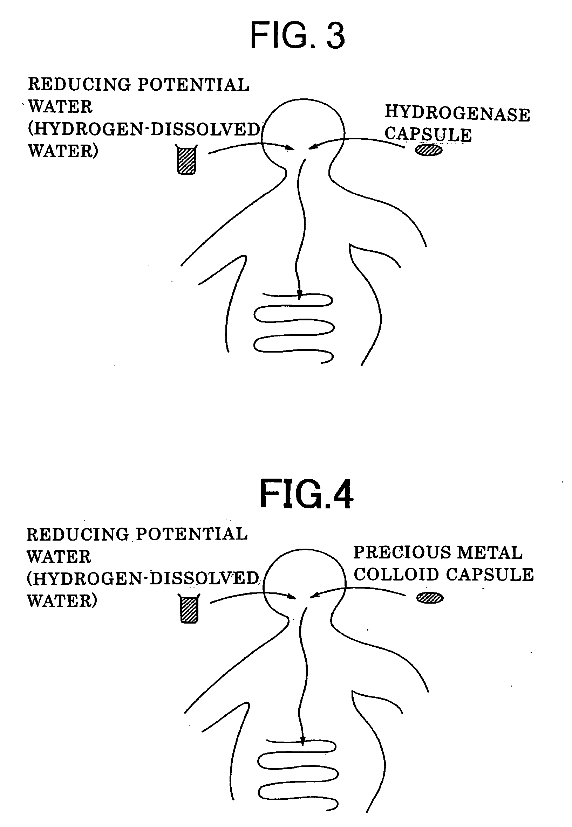

Here, the processing or manipulation for adjusting the activation time and/ or the reaction time of the catalyst, as shown in FIG. 3 or FIG. 4, includes processing to seal the exemplary hydrogenase or a precious metal colloid in an enteric capsule or the like, adjusting the pH or temperature of the hydrogenase- included antioxidant- functioning water within a range where the activation of the enzyme hydrogenase is controlled without deactivating the activity, or the like, with the aim of having the primary catalytic action begin when the hydrogenase or a precious metal colloid reaches the subject portion such as the large intestine or small intestine. It should be noted that the optimal pH for the hydrogenase is considered to be in the neighborhood of 9, and the optimal temperature approximately 49 °C. In addition, anything that employs processing or manipulation for adjusting the reaction time of such catalyst on the hydrogenase, etc., or the environment there surrounding, falls within the technical scope of the present invention.

The present invention provides a living organism-applicable fluid using the antioxidant-functioning water as a main component, which is hydrogen-dissolved water containing a precious metal colloid or a hydrogen oxidation/ reduction enzyme (except for those already existing in the living organism) that catalyzes the breaking reaction of molecular hydrogen used as a substrate into a product of active hydrogen, and has an antioxidation function of transforming an antioxidation subject, which is in an oxidation state due to a deficiency of electrons or needs to be protected from oxidation, into a reduced state of electrons being filled, by promoting the breaking reaction via the catalyst, and is prepared so as to allow usage on living organisms including drinking, injection, intravenous drip, dialysis, and cosmetics.

Namely, using the antioxidant-functioning water according to the present invention on living organisms for intended applications such as injection, intravenous drip, and dialysis, in particular, flowing through the bloodstream as is, adjusting the osmotic pressure so as to be almost isotonic with blood and adjusting the pH so as to lie within a physiologic range of liquid (e.g., pH 4.5 through 8.0, preferably, pH 7.0 through 7.8, more preferably pH 7.1 through 7.5) for the antioxidant-functioning water according to the present invention must be implemented. In this case, the osmotic pressure-adjusting substance is not particularly limited so long as it can be physiologically tolerable. For example, various electrolytes (e.g., dissolved salts of inorganic elements such as sodium, potassium, calcium, magnesium, zinc, iron, copper, manganese, iodine, and phosphorus), saccharides such as glucose and hyaluronic acid, proteins such as albumin, and amino acids may be used. In addition, the pH adjustor is not particularly limited so long as it can be physiologically tolerable. For example, various organic acids, mineral acids, organic bases, and mineral bases may be used. In particular, organic acids are preferably used. For example, citric acid, acetic acid, succinic acid, gluconic acid, lactic acid, malic acid, maleic acid, molonic acid, and the like can be used as the organic acid, and hydrochloric acid, phosphoric acid, and the like can be used as the mineral acid. On the other hand, sodium citrate, sodium gluconate, sodium lactate, sodium malate, sodium acetate, sodium maleate, sodium malonate, and the like can be used as the organic base, and alkali hydroxide metal and the like can be used as the mineral base.

Furthermore, in order to improve symptoms of a living organism, it is preferable that chemical components such as various electrolytes, amino acids, high calorie components, enteral feeding products, and pharmaceutical components such as vitamins, antibiotics, and the like are added to adjust the components contained in the antioxidant-functioning water according to the present invention, and this obtained water is used as transfusion fluid. It should be noted that dissolved salts of the inorganic elements such as sodium, potassium, calcium, magnesium, zinc, iron, copper, manganese, iodine, phosphorus, and the like can be used as the various electrolytes. In addition, essential amino acids, non-essential amino acids, and/ or salts of these amino acids, esters, N-acyl substances, or the like can be used as the amino acid. Furthermore, monosaccharides such as glucose, fructose, and the like, and disaccharides such as maltose and the like can be used as the high calorie components.

Here, it may be thought that with the living organism-applicable fluid, which is antioxidant-functioning water according to the present invention to which vitamins such as vitamin C and amino acid (superior protein) are added, immune system activation for living organisms in conformity with the following working-action mechanism can be expected. Namely, vitamins such as vitamin C have radical scavenging activity as with the antioxidant-functioning water of the present invention, and act as a coenzyme for synthesizing or demonstrating primary functions of living-organism enzymes (such as SOD, catalase, glutaminperoxidase, and interferon synthesized enzyme), which controls the metabolism of living organisms, and interferon (a substance made of saccharum and protein, demonstrating immunity). In addition, amino acids (superior protein) play an important role of a raw material for living-organism enzymes and interferon.

Here, it is assumed that excessive amounts of (•O₂⁻) generate locally in living organisms. As a result, vitamins such as vitamin C and vitamin E and living-organism enzymes such as SOD, catalase, and glutathionperoxidase collaborate and scavenge (•O₂⁻). At this time, since vitamins such as vitamin C and vitamin E are self-oxidized in the process of reducing/ scavenging (•O₂⁻), they cannot perform their primary duties including the role as a coenzyme. As a result, primary functions or synthesis of living-organism enzymes and interferon in living organisms decreases, thereby leading to a decrease in immunity.

Meanwhile, it may be thought that with the living organism-applicable fluid, which is antioxidant-functioning water according to the present invention to which vitamins such as vitamin C and amino acid (superior protein) are added, immune system activation of living organisms can be expected as a result of promoting synthesis or primary functions of the living-organism enzymes and interferon, caused by supplying amino acids and devotion to the primary duties including acting as a vitamin coenzyme, along with scavenging of (•O₂⁻) by the antioxidant-functioning water.

It should be noted that even with antioxidant-functioning water according to the present invention not containing vitamins and amino acids, living organism-applicable fluid according to the present invention containing vitamins, or living organism-applicable fluid according to the present invention containing amino acids, it may be thought that immune system activation of living organisms in conformity with the exemplary working-action mechanism described above can be expected since consumption of vitamins existing in the living organisms can be controlled, and vitamins can devote to their primary functions including acting as a coenzyme.

Meanwhile, it is essential that safety be guaranteed when using a precious metal colloid as a catalyst for application in a living organism. More specifically, it is necessary to consider biocompatibility including the acute toxicity of the precious metal colloid itself. In regards to this, with for example platinum and palladium, considering that when it is ingested by a person nearly all of it passes through the liver and is promptly eliminated in urine, and in addition, considering the fact that it has been allowed as a food additive by the Japanese Ministry of Health, Labour, and Welfare (there are no restrictions on the amount of additives), there should be no problem with biocompatibility. One more problem that must be considered might be the possible need to add some sort of dispersion agent in order for the precious metal colloid to disperse into the antioxidant- functioning water stably and evenly. In regards to this, for instance in the case where it will be ingested through drinking or used as a cosmetic, that which has dispersion agent function should be appropriately selected from those that have been allowed by the Japanese Ministry of Health, Labour, and Welfare as food additives. In this case, the exemplary polyvinyl pyrrolidone (PVP) and sucrose esters of fatty acids, which are hypoallergenic and widely used in cosmetics and medical products, may be favorably used. It should be noted that including sucrose esters of fatty acids or polyvinyl pyrrolidone (PVP) in antioxidant-functioning water as a component for forming a dispersion agent or a protective membrane (has a catalytic activity-adjusting function) for a precious metal colloid is included in the category of processing or manipulation for adjusting the catalytic activity time period and/ or reaction time period in relation to the appended Claims.

Such antioxidant- functioning water may be considered for possible deployment in for example the following industrial fields.

Firstly, application may be made in the fields of medicine and pharmaceuticals. For example, it may be used as base water in the manufacturing process of transfusion fluid and other medical agents. In addition, it may also be used as artificial dialysis fluid, peritoneal dialysis fluid, and pharmaceuticals. Furthermore, it may also be favorably used as preservation fluid for transplanted organs when transplanting living organs (in this case, it is preferable that osmotic regulation is performed separately). Through this, it is possible to expect prevention/ treatment of various diseases caused by active oxygen species, inhibition of aging and reduction of side effects due to pharmaceuticals, and improvements in preservation quality of transplanted organs.

Secondly, application may be made as a prevention/ treatment agent for aging and degeneration caused by oxidation of cutaneous tissue. For example, it may be used in the manufacturing process of cosmetic toners and other cosmetics.

Thirdly, application may be made in antioxidant food and functional food. For example, it may be considered for use in food manufacturing processes.

Fourthly, application may be made in potable water, processed water, and the like. For example, it may be considered for use as drinking water (antioxidant water), and also for use as base water in processed potable water such as canned juices, canned coffees, (PET) bottled water, and soft drinks.

Fifthly, application may be made to reduce contamination/ deterioration of food due to fertilizers, herbicides, pesticides, etc., and also maintain freshness. For example, it may be used as a pre-shipment rinsing fluid for vegetables, fruits, and the like.

Sixthly, application may be made as a substitute for antioxidants, anti-deterioration agents, anti-decomposition agents, anti-contamination agents, deodorants, and freshness-keeping agents, and the like in prepared food manufacturing. More specifically, it may be considered for instance as a substitute for the over 347 types of food additives.

To further explain this, it has been pointed out that the involvement of radicals including active oxygen species in oxidation, aging, deterioration of quality, decomposition, contamination, deodorization, and loss of freshness is one of the important factors of a mechanism which causes expression and deterioration thereof. As a result, radicals including active oxygen species may lead to serious damage, such as health problems, expression of diseases, deterioration of physiological functions, degeneration of cosmetic appearance, decrease in commercial value, decrease in productivity, and increase of living/ natural environmental burdens. This also causes industrial losses such as increase in medical costs due to diseases easily occurring but difficult to remedy, opportunity loss, and high cost expenses in production and distribution. There are limited cases where the involvement of the active oxygen species is desirable, for example, in sterilization, disinfection, bleaching, and the like. In most cases, the active oxygen species only have negative influences. The present invention provides a most effective, low cost and widely applicable means for solving the various industrial problems due to the radicals including the above-mentioned active oxygen species.

Cases of suddenly exposing a living organism to large amounts of the active oxygen species are relatively limited to, for example, emergency blood recirculation during operations, organ transplants, burns on the entire body, and pulmonary emphysema due to sudden surfacing when diving. In most cases, a small amount of active oxygen species causes injury, leading to extensive harmful effects over time.

There are various occasions where oxygen and oxygen molecule-containing chemical compounds in normal or inert condition are activated and even changed to radicals, for example, with the metabolizing process and accumulation of waste products in living organisms; when being exposed to direct sunlight, ultraviolet rays, or radiation; when touching carcinogenic substances, mutagenic compounds, or heavy metals; when suffering from a burn or viral infection; or when suffering from a cut or cytoclasis. In addition, when exposed to food, drink or feed in the nature of the active oxygen species, cigarette smoke, smoke and exhaust fumes, or chlorinated organic solvents, not only being directly effected by the radical action from these elements, but also generation of active oxygen species is caused or induced in the living organisms. Although the amount of generated active oxygen species is extremely small and localized in the beginning, as the condition of generation thereof surpassing the normal level continues, or the condition of the generated active oxygen species not being completely removed and still remaining continues, the amount of active oxygen species gradually increases at a certain accelerating rate, and adverse effects as with the above-mentioned cases begin to express.

In other words, the active oxygen species cause damage in a different way than from enzyme reactions and normal chemical reactions (non-radical reactions) such as the oxidation/ reduction reaction where its generation and influence increase continuously. In short, deterioration and degeneration of physiological functions inherently progress due to the generation of active oxygen species, and it rapidly comes to be expressed as a severe injury as if it suddenly becomes manifest one day.

To exemplify cases of human diseases, it is said that chronic oxidation stress burden due to active oxygen species is the principal cause that is involved in the underlying mechanism of various diseases such as diabetes (particularly insulin-independent diabetes), liver cirrhosis (particularly fatty liver cirrhosis), cardiovasucular diseases (particularly arteriosclerosis) including angina pectoris, dementia (particularly cerebral infarction), and malignant tumors (mainly chemical carcinogenesis). In addition, deterioration of physiological functions due to aging or exhaustion of physical strength, abnormal exasperation of metabolism and breathing due to continuous strenuous exercise, excess and deficiency of nutritional balance and intake, and insufficient sleep and exercise are expressed as a phenomenon such as aging, degeneration, or fatigue. In the above-mentioned phenomena, metabolic waste products become easily accumulated, and it is confirmed that the generation of active oxygen species or amount of residual accumulation thereof increases along with reconstruction failure and function deterioration of cellular tissue or skin. It is an important factor in aggravating symptoms or delaying recovery thereof.

To exemplify cases of animals and plant other than humans, excessive generation or residual accumulation or exposure of active oxygen species causes various health impairments or growth disorders as with the case of humans; as a result thereof, this also leads to reduction in productivity and commercial value such as decrease in milking, deterioration of carhosity and decline in fattening rate, decline in oviposition rate, poor breeding including depilation, decline in breeding success rate, increase suffering from insect plague, decrease in appreciative value, decrease in cultivated harvest and catch, and deterioration of quality thereof in industries, such as breeding of domestic animals, breeding of pets, aquaculture, and plant culture.

To exemplify cases of food, drink and animal feed, generation of active oxygen species due to exposure to ultraviolet rays, mixing of raw materials effected by active oxygen species, and residual hydrogen peroxide, which is a raw material of active oxygen species and is used for disinfection and bleaching during the manufacturing process thereof, induce oxidizing destruction of nutritional elements and effective elements such as vitamins, decomposing fats and emulsion breakdown with fat-containing or -using elements, color fading or discoloration in pigment-containing or -using elements, and deterioration from cuts with perishable products, which are accompanied by fetor and degrading taste and quality of food that develop at an accelerating pace, leading to significant deterioration of quality.

To exemplify an environmental case, existence of suspended matters affected by or easily affected by radicals including active oxygen species provides an environment where fetor, allergy, or inflammation occurs easily in a living space or working place, and promotes change in water quality.

It is safe to say that damages in the above-mentioned cases may develop even if the active oxygen species are not involved, and development thereof is drastically promoted when the active oxygen species are involved.

When radicals including active oxygen species are involved in increasing damages, it is difficult to prevent or suppress the damages with the conventional antioxidants, anti-aging agents, anti-deterioration agents, anti-decomposition agents, anti-contamination agents, deodorants, and freshness-keeping agents. The inventors are highly confident that the only solution thereof is the present invention of the antioxidation method, antioxidant-functioning water, and usage of the same.

Since the present invention has a principal action of antioxidation and never produces side effects associated therewith compared to the usage of an active oxygen species scavenger such as the conventional antioxidant, high benchmarks of both of safety and radical scavenging efficiency can be simultaneously achieved, and many problems such as degradation of flavor or color tone, breakdown of physical properties, higher costs, fear of secondary infection, difficult to use, and limited application that were difficult to solve with conventional materials can be resolved.

In addition, since the present invention is not easily affected by pH, as described in the following embodiments, it can be widely applied in the fluid field from acidity to alkalinity differing from the case of enzymes or antioxidants, and by demonstrating favorable effects at room temperature, it proves extremely industrially useful in broad fields such as food and pharmaceutical fields.

### OPERATION AND EFFECTS OF THE INVENTION

As described above, the important factors in the present invention are 1) the hydrogen- dissolved water, 2) the catalyst, and 3) the antioxidation subject. When these three factors are organically combined, the seal on the reducing power latently held by the hydrogen is cast off to allow manifest expression of the antioxidation function.

According to the antioxidation method and antioxidant- functioning water according to the present invention, an antioxidation subject, which is in an oxidation state due to a deficiency of electrons or needs to be protected from oxidation, may be transformed into a reduced state of electrons being filled by promoting the breaking reaction of a molecular hydrogen substrate included in the hydrogen- dissolved water into a product of active hydrogen through a process employing a catalyst on the hydrogen- dissolved water, while anticipating high benchmarks of safety on the living organisms and reduced environmental burden.

Namely, the present invention of the antioxidation method, the antioxidant-functioning water, and usage of the same demonstrates excellent radical scavenging functions in a wide pH range from acidity to alkalinity even at room temperature.

Therefore, the usage of the present invention is expected in order to prevent or improve various disturbances such as oxidizing stress or aging, which is easily caused by involvement of the active oxygen species during physiological activity in an organism such as humans, animals other than humans, plants, zymotic microorganism, and cultured cells, or in a field where prevention of reduction of activity and deterioration of quality such as transformation, deconstruction, decomposition, contamination, off-odor, loss of freshness, reduced effectiveness, and deterioration of efficiency of products in breeding, aquaculture, cultivation, fermentation, incubation, fabrication, and preservation, or in the production process thereof are in demand.

Furthermore, the availability of the present invention is expected in various industrial fields such as food products, animal feed, pharmaceutical/ medical supplies, unregulated drugs, cosmetics, cleaning agents, deodorants, sanitary goods, garments, materials for maintaining freshness, packaging containers, animal breeding, aquaculture, plant cultivation, fermentation, and incubation.

Some exemplary specific aspects, which can be provided to a user to easily exhibit the availability of the present invention and easy utilization thereof, of the groups of products in the above-mentioned industries are as follows.

For example, in the field of food products, the present invention can be utilized as a food additive having an advanced active oxygen species-removing function for improving the quality of processed food products and keeping quality, or maintaining freshness of perishable food products, or for maintaining health and preventing diseases by providing specified health food and healthy food products containing the antioxidant-functioning water of the present invention as an active principle, respectively. In the field of animal feed, the present invention can be utilized as additive for animal feed or pet food for managing health, and improving animal feed efficiency and productivity.

In the fields of medical supplies, unregulated drugs, cosmetics, and medical tools provided in each sub-section in Section 2 of the Japanese Drugs, Cosmetics and Medical Instruments Act, not only is the present invention useful for improving the quality of preparation as pharmaceutical additives and cosmetic additives, but it can also be used as an active principal for treating and preventing diseases, improving physical conditions and constitution, maintaining and improving beauty, and preserving sanitary conditions and comfortable environments. Of these, in the field of medical supplies, the availability of the present invention is particularly expected in medical treatment water for diseases, which may express, take a delayed recovery time, or aggravate due to involvement of the active oxygen species, or in general, fields such as medicines for revitalizing health, digestive medicines, cold medicines, medicines for stomtic/ rhinitics, and dermatologic medicines. As for unregulated drugs, the present invention is highly available in the fields of medicated dental agents, mouth refrigerant, medicated cosmetics, hair agents, bath agents, underarm deodorants, and sanitary treatment products. Furthermore, in the case of cosmetics, it is highly available in the fields of hair care products, shampoo cosmetics, skin toners, skin creams and milky lotions, facial masks, foundation, lip rouge, facial wash, soaps, and dental agents.

It should be added that the antioxidation method and antioxidant-functioning water according to the present invention can be provided in various specific aspects, and therefore the embodiments and availability of the present invention are not limited to the above-mentioned exemplary cases.

### BRIEF DESCRIPTION OF THE DRAWINGS

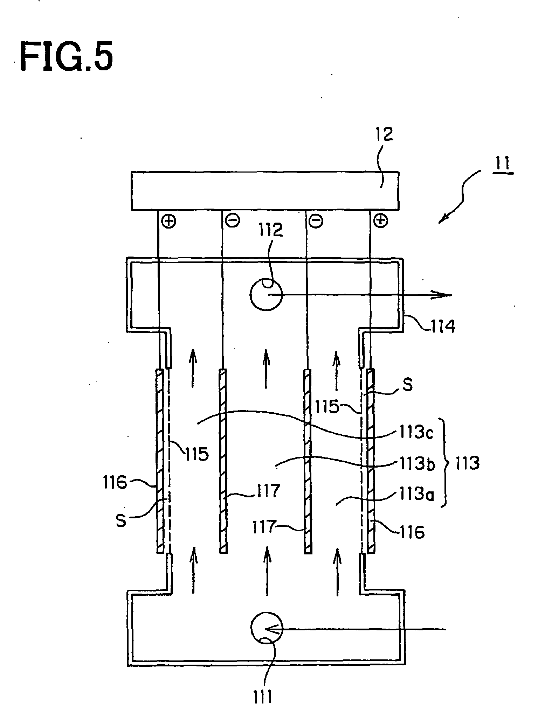

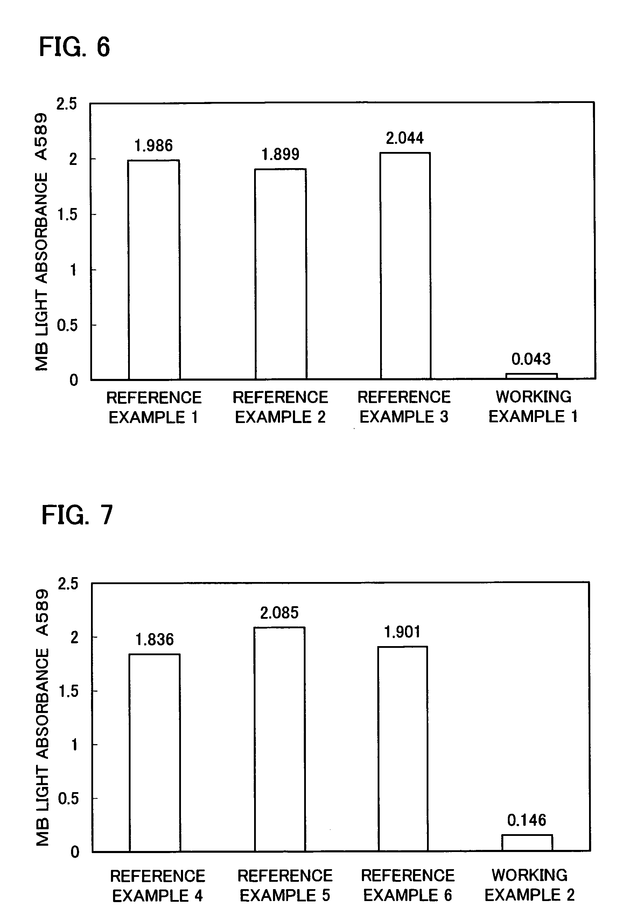

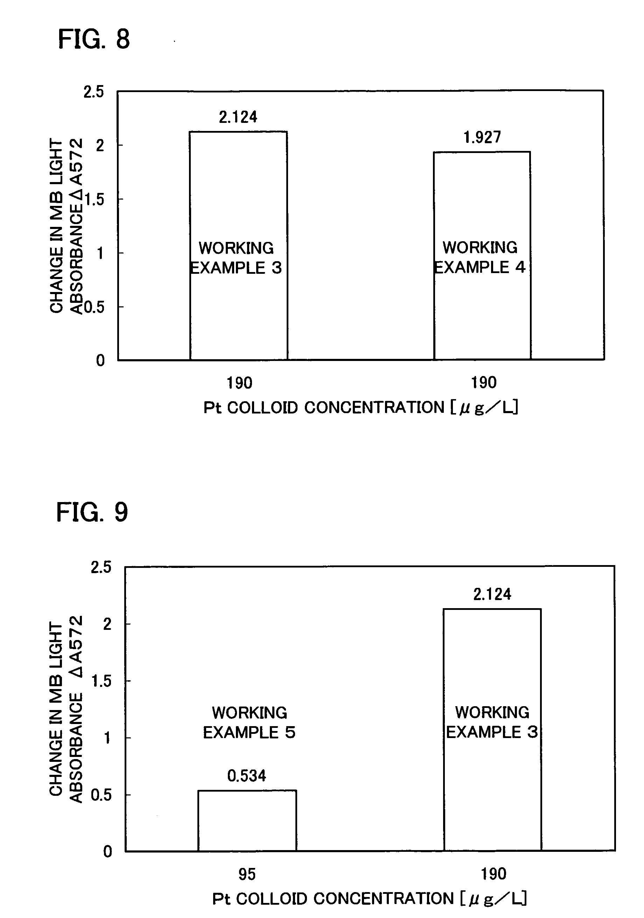

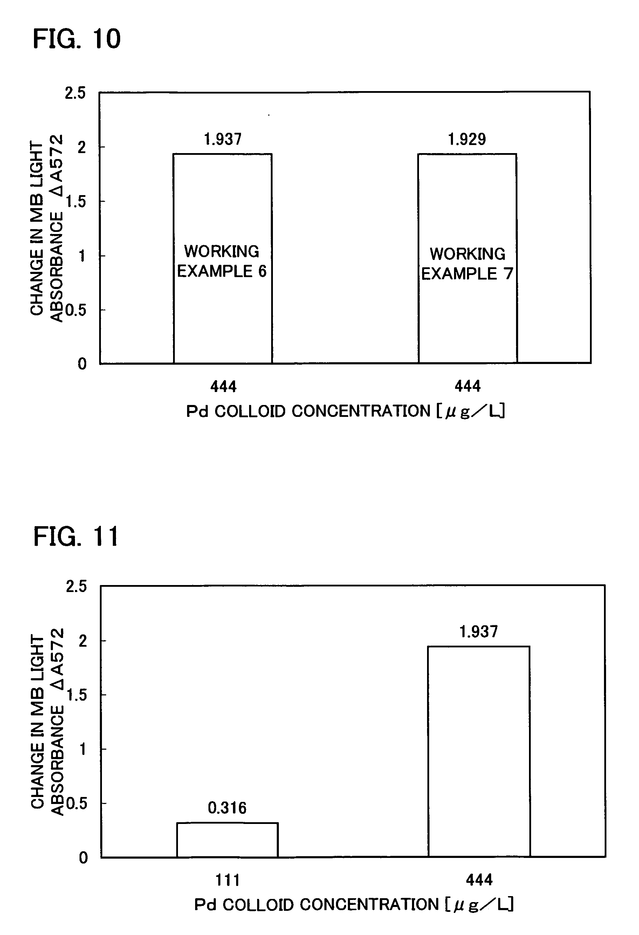

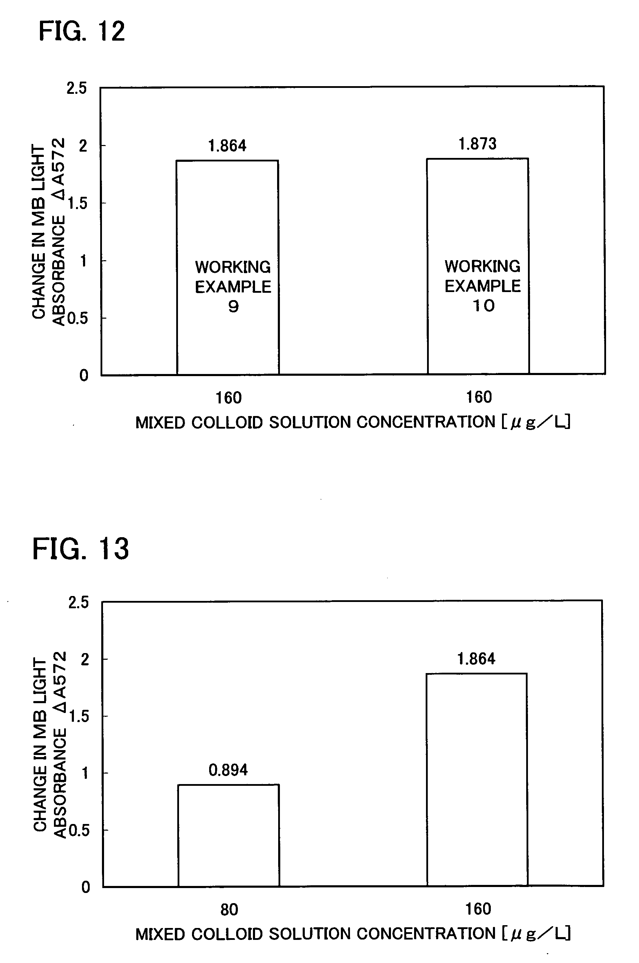

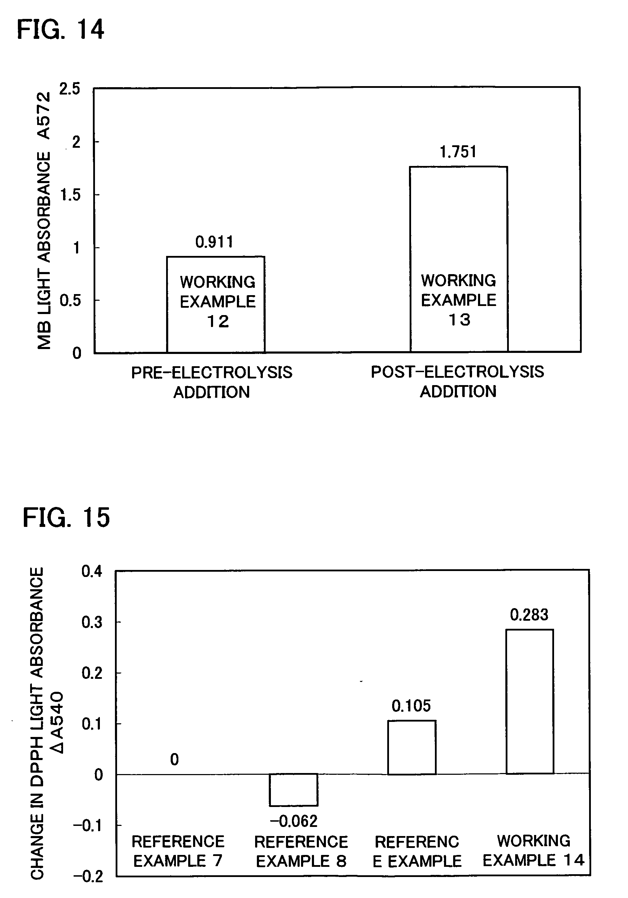

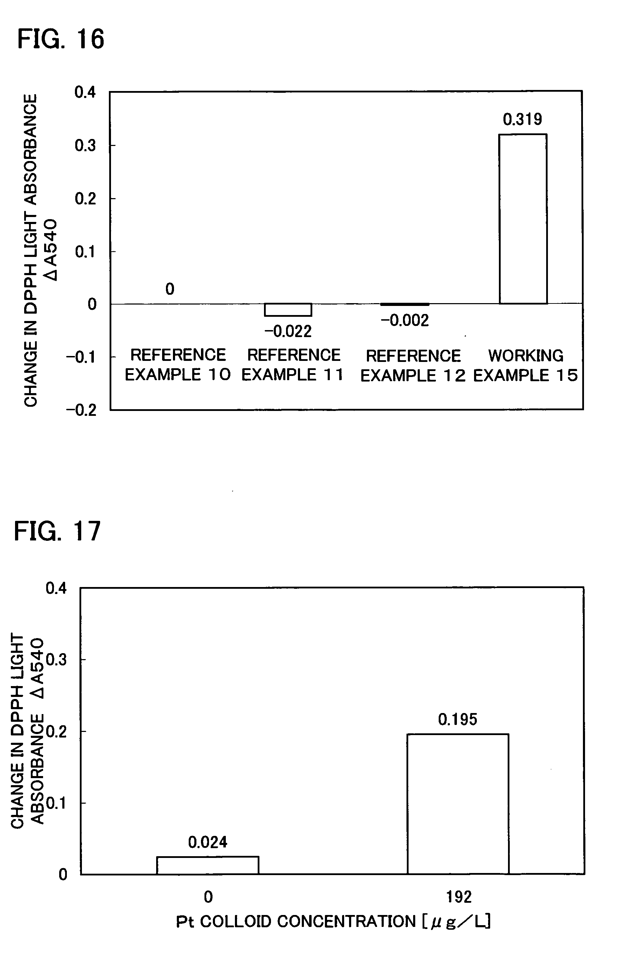

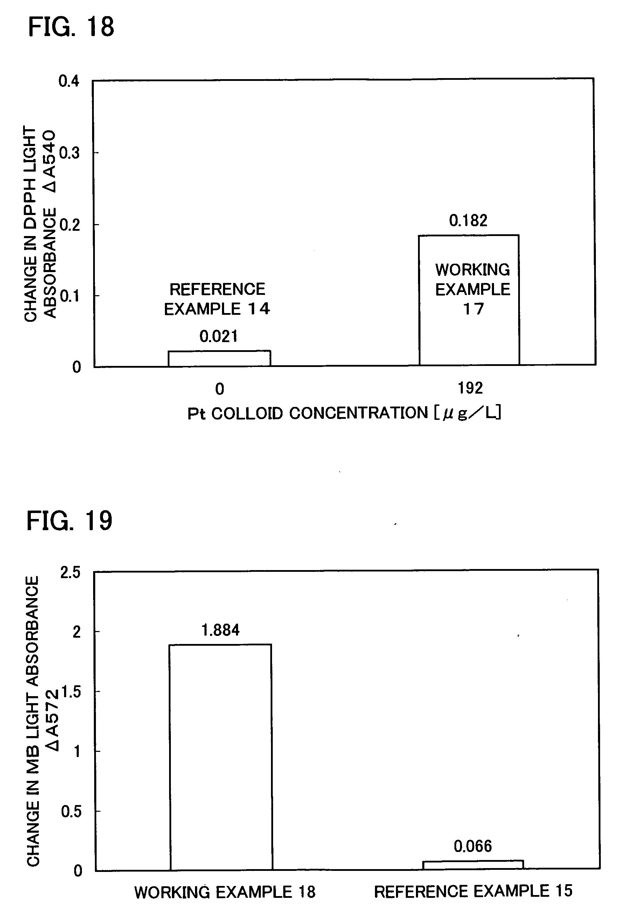

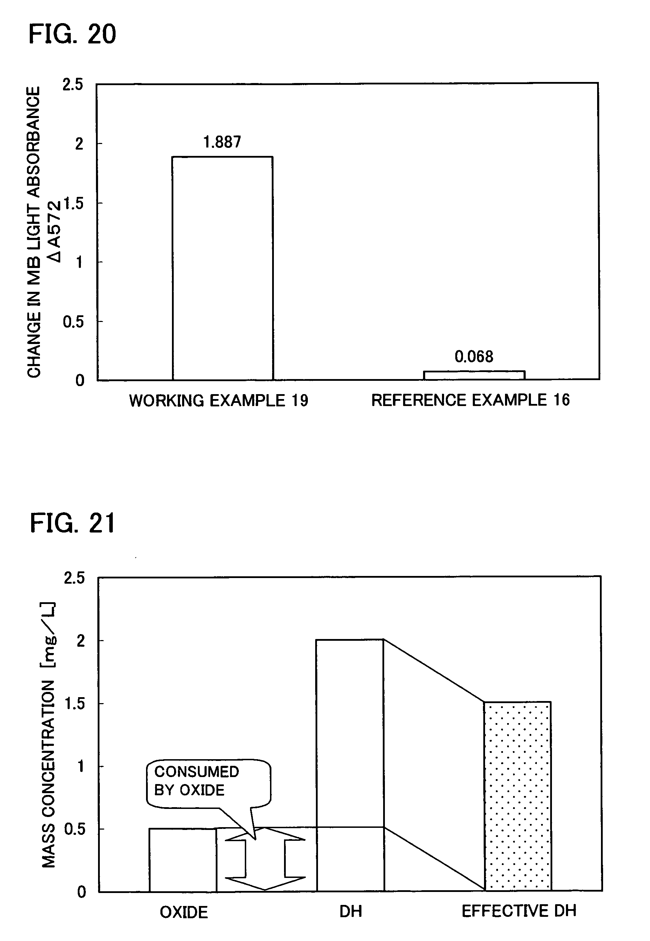

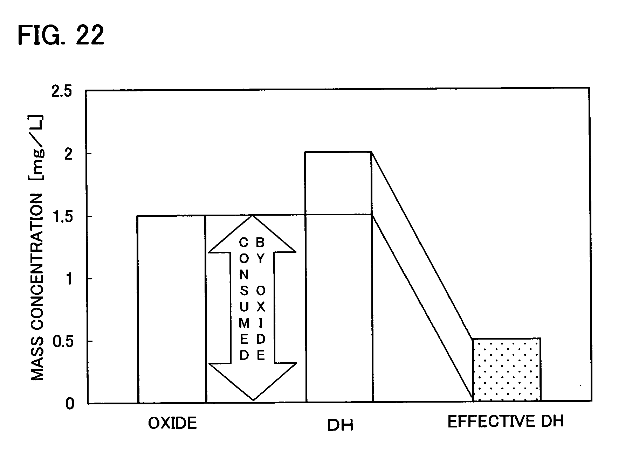

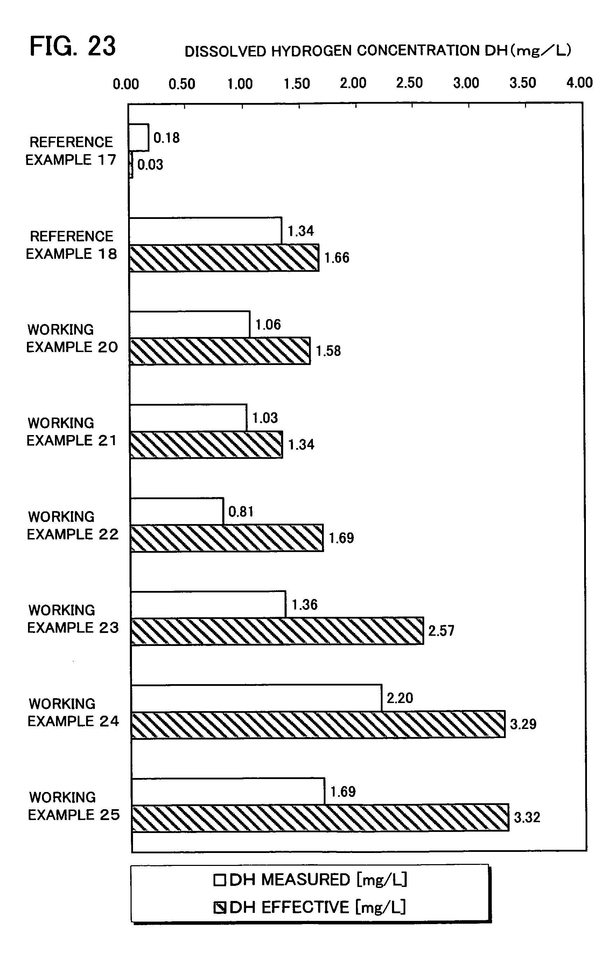

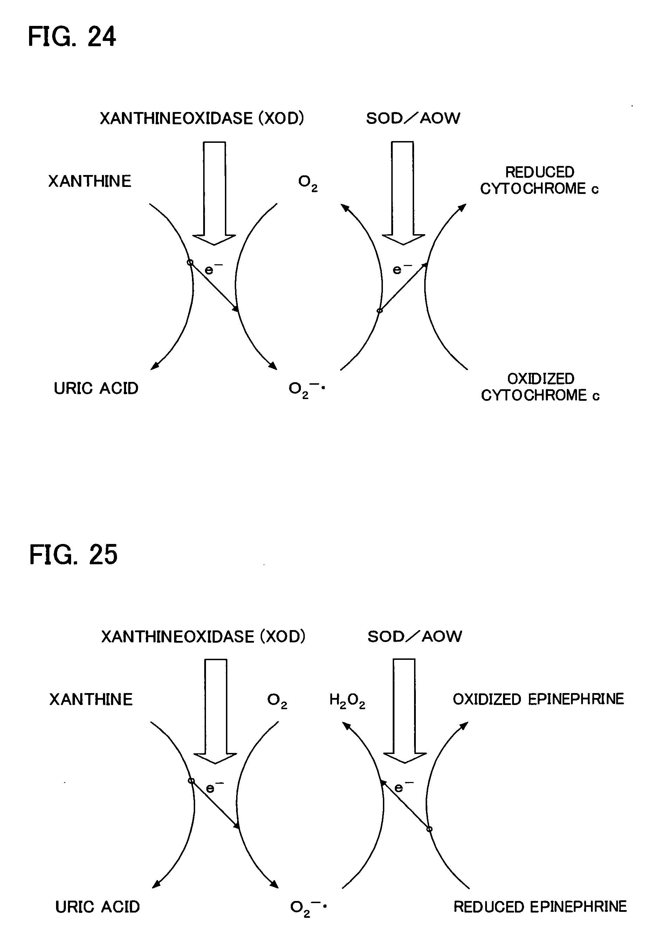

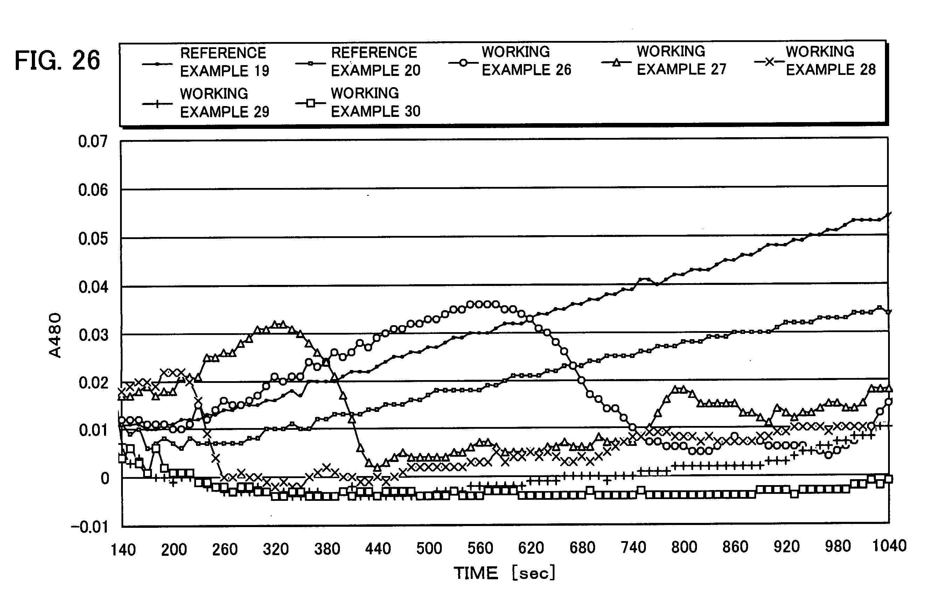

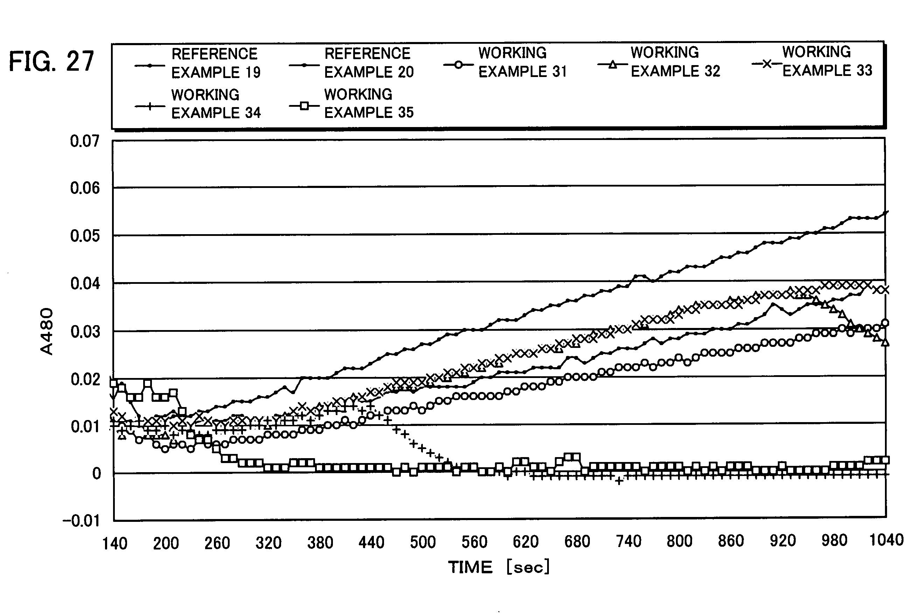

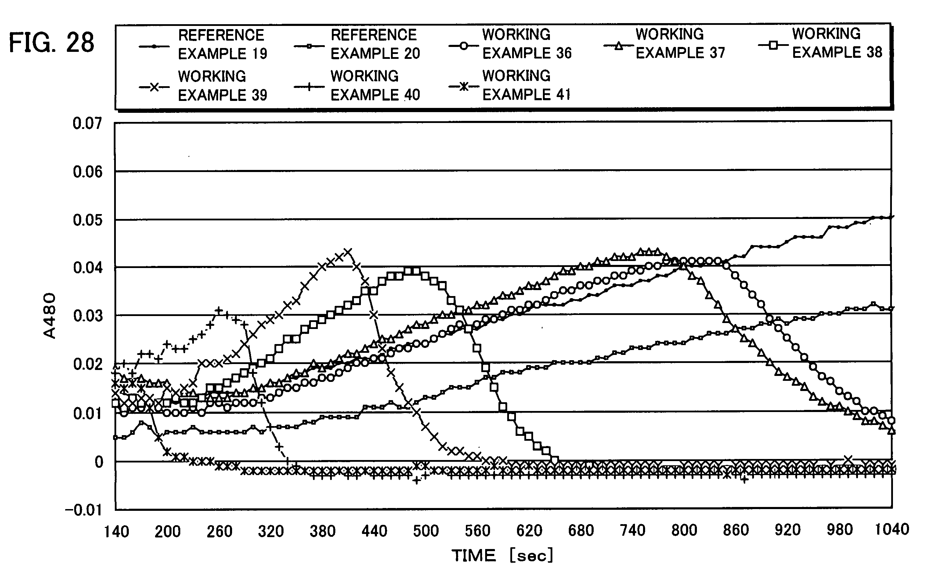

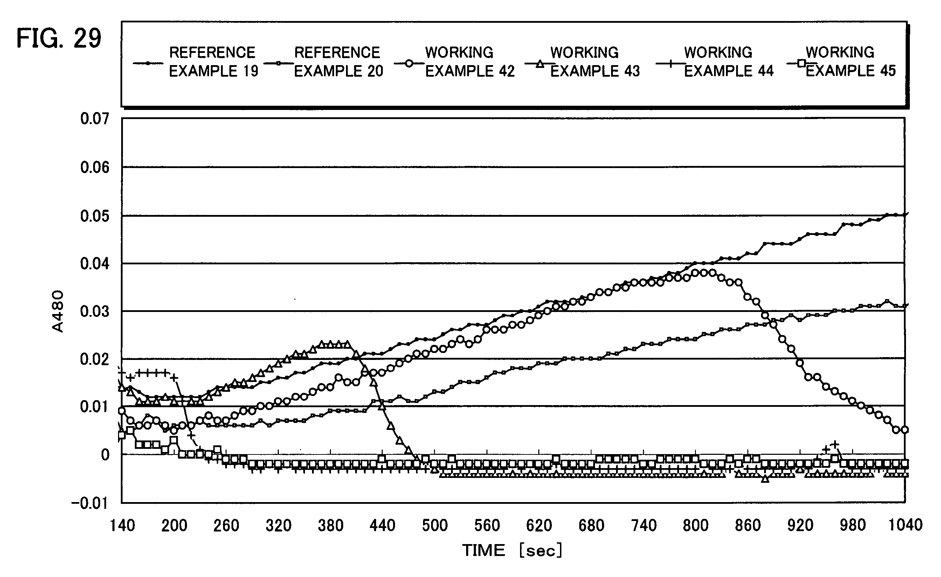

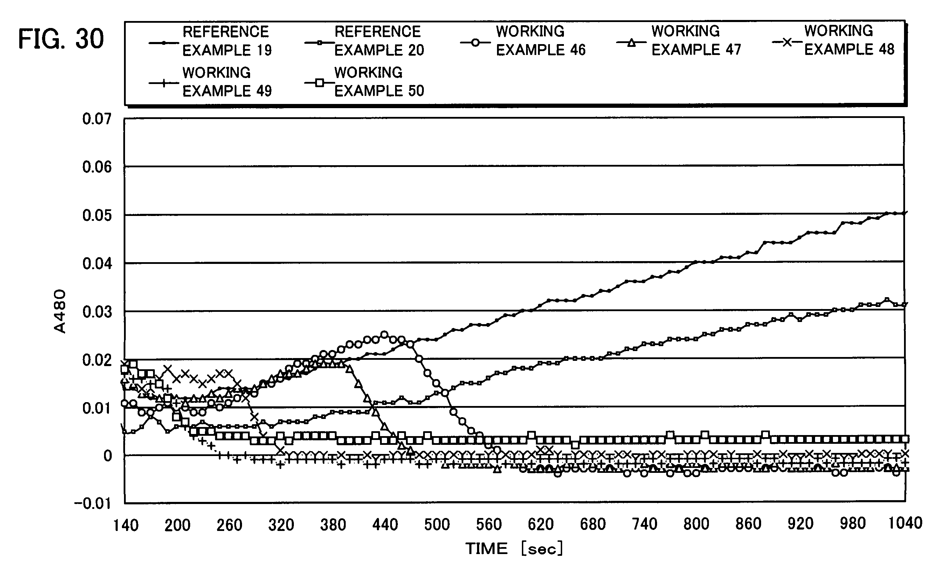

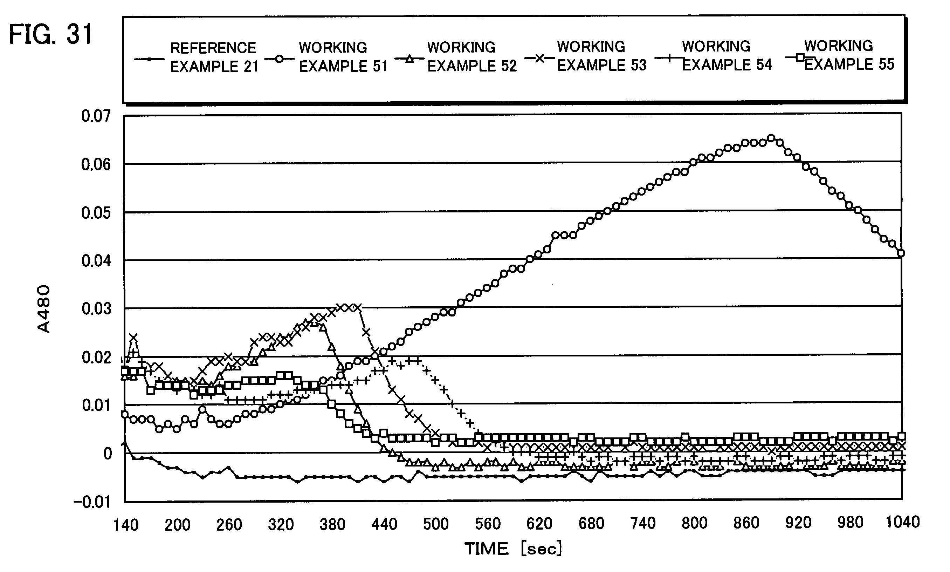

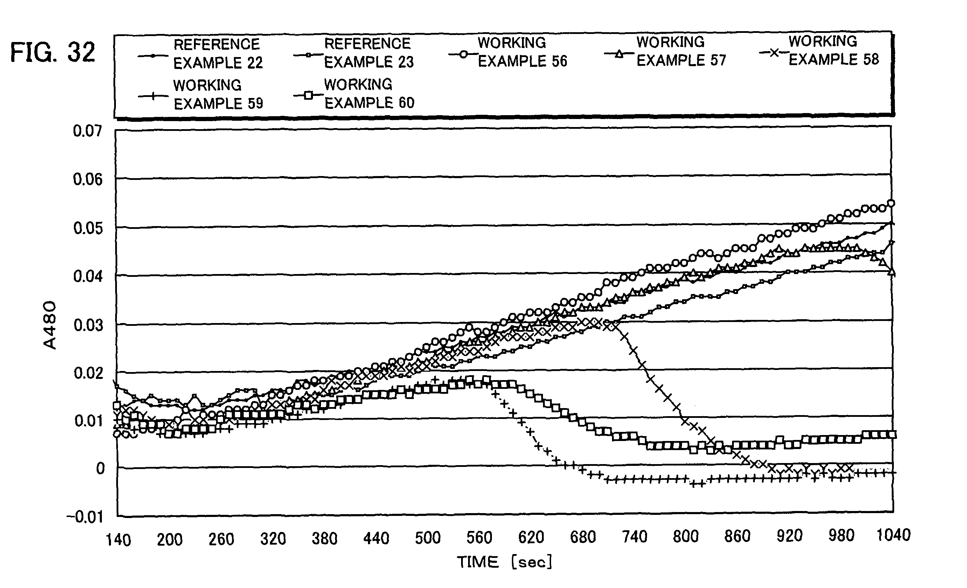

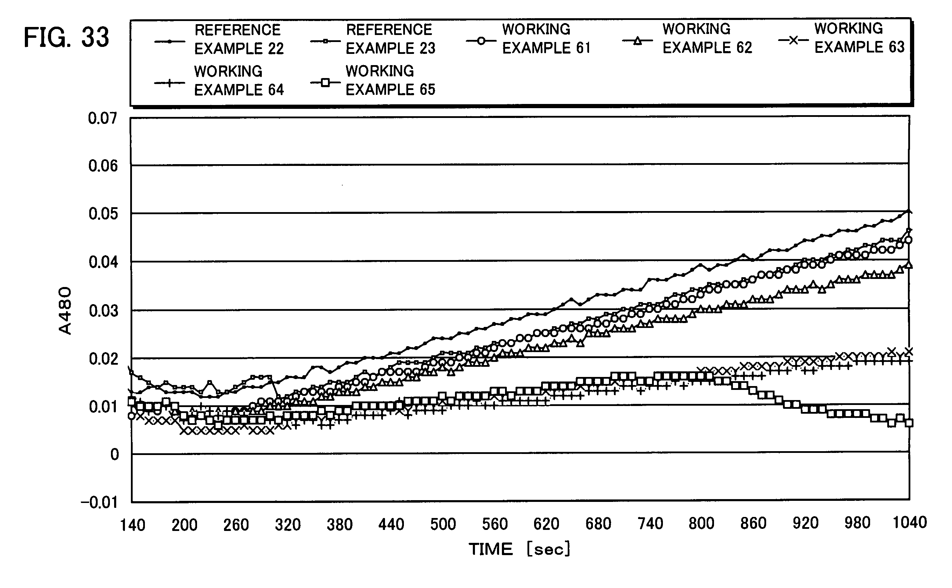

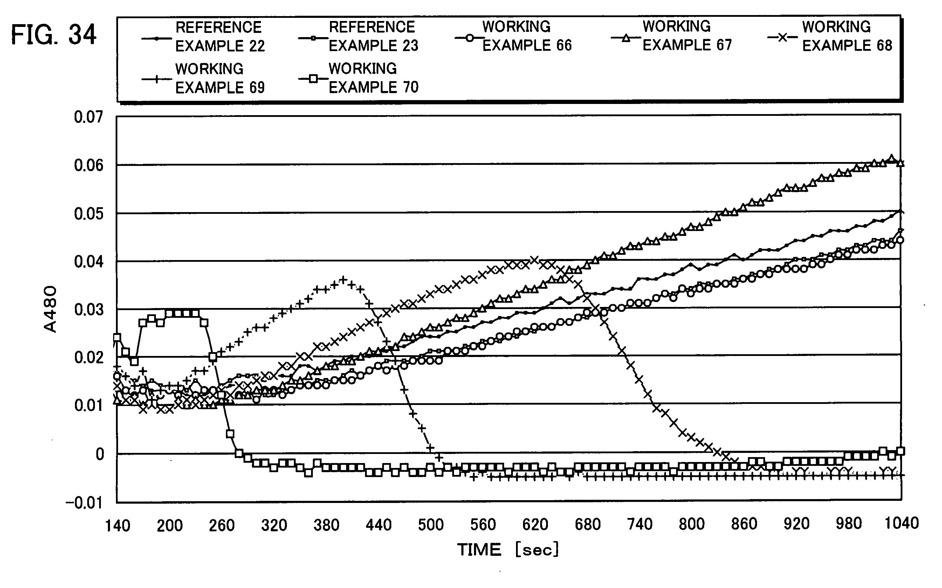

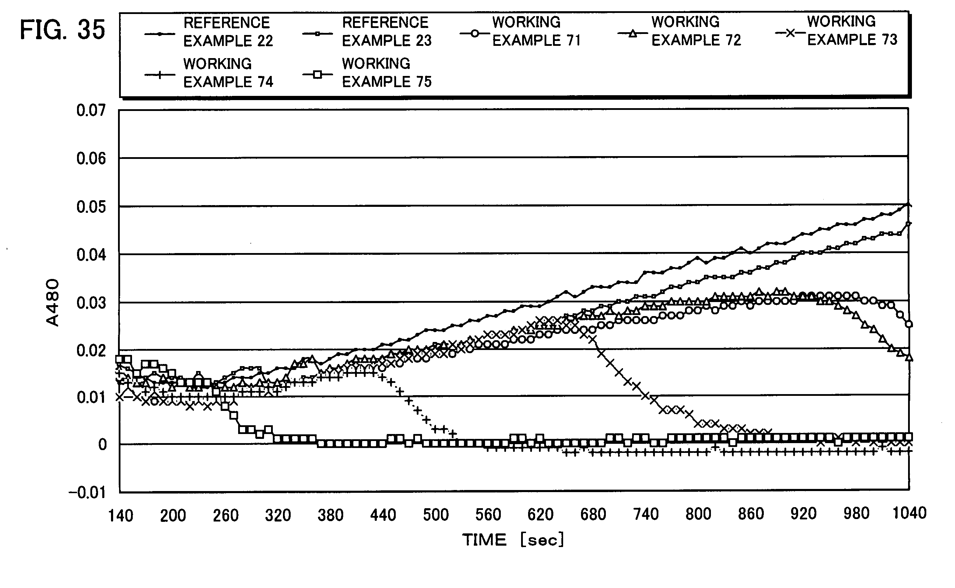

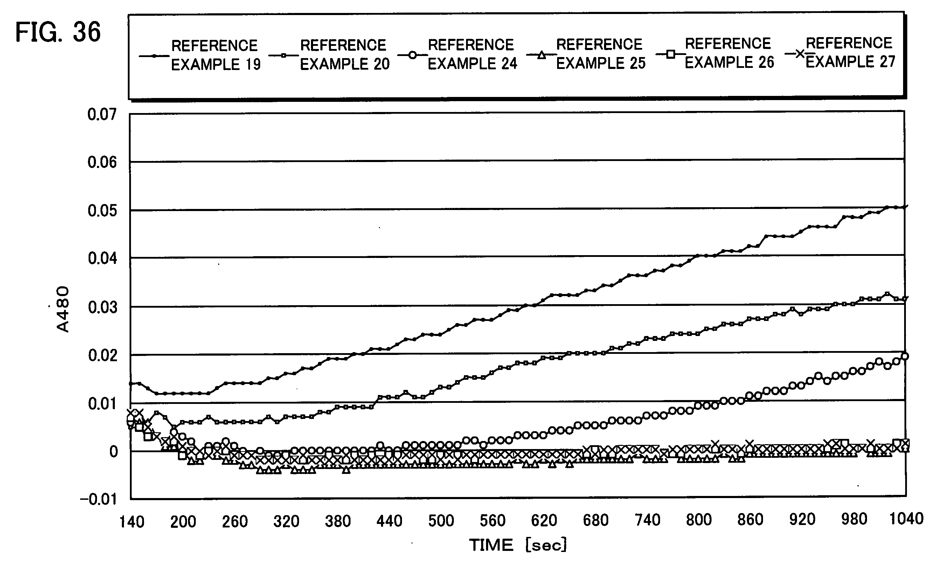

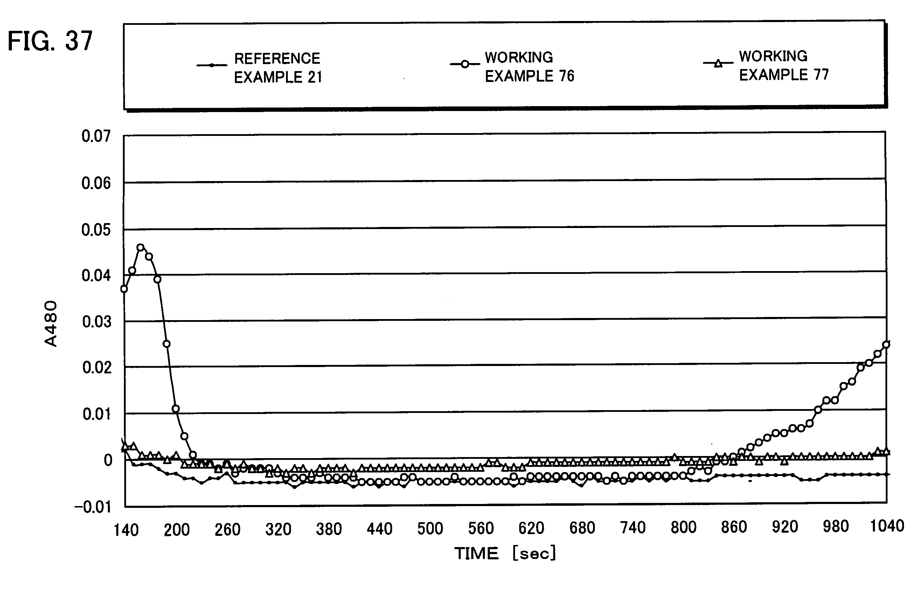

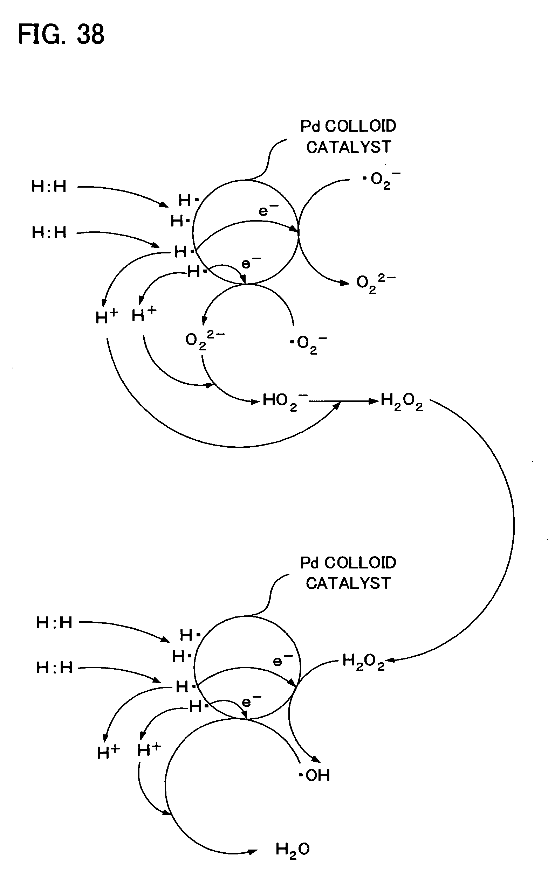

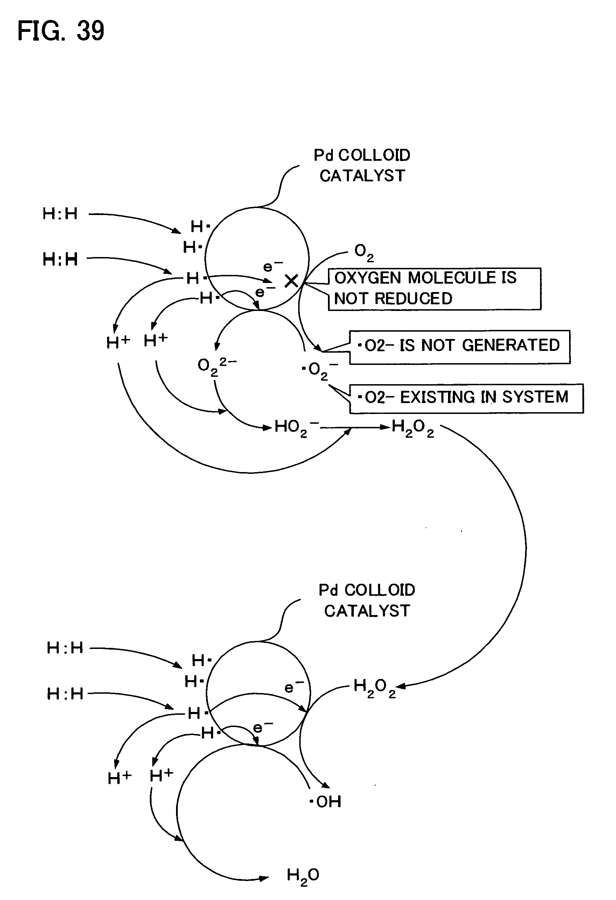

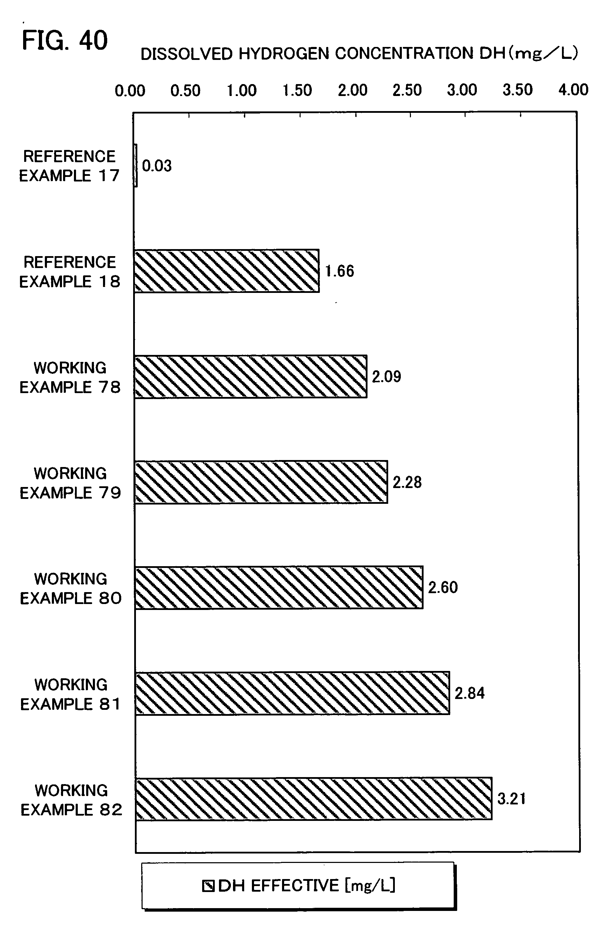

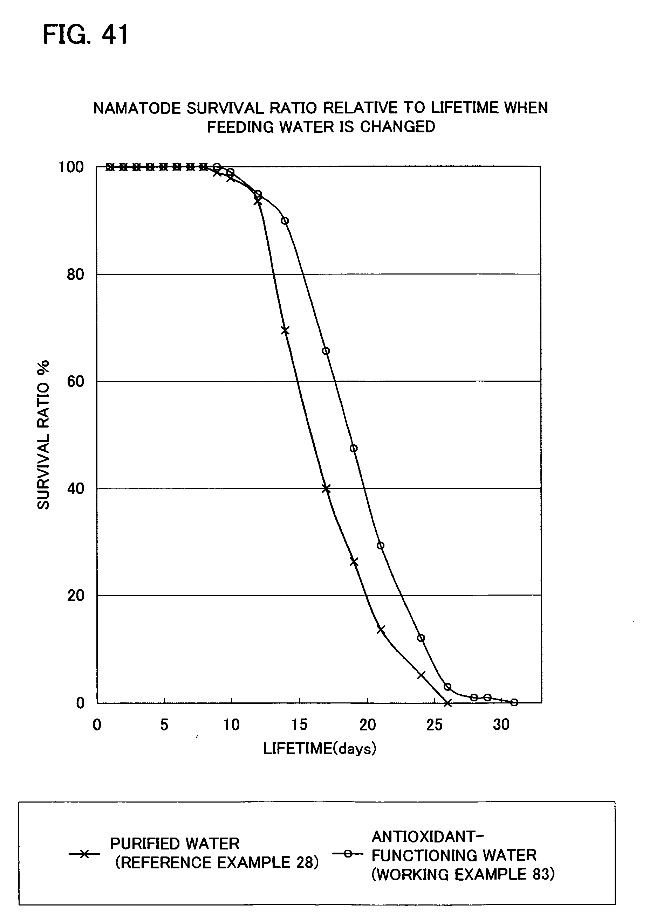

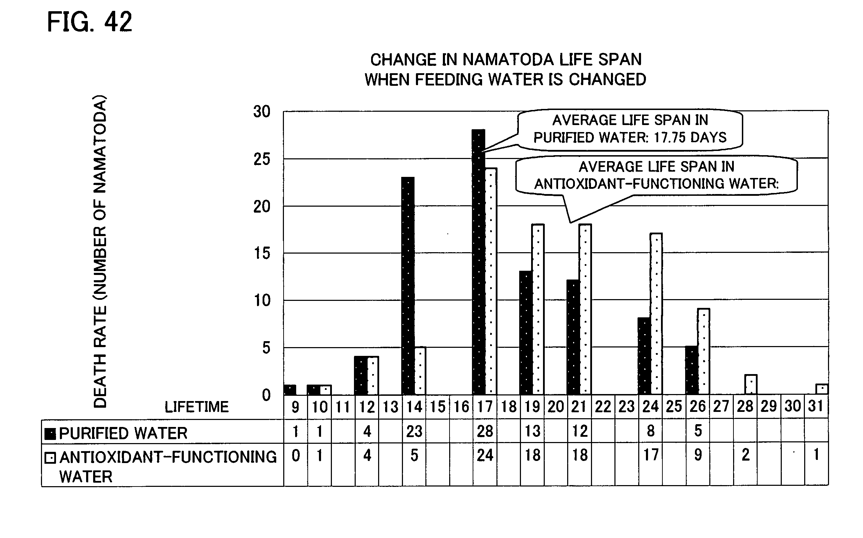

FIG. 1 is a graph showing the Nernst equation; FIG. 2 is a diagram for describing the conditions of an illumination test using an LED; FIG. 3 is a diagram for describing an exemplary application of the present invention; FIG. 4 is a schematic diagram showing a semiconductor wafer rinsing system 100 using the method of antioxidation of the present invention; FIG. 5 is a vertical cross- sectional view showing the basic configuration of a reducing potential water generation apparatus 11 used in the rinsing system 100 of the present invention; FIG. 6 and FIG. 7 are diagrams showing reduction activity evaluation test results for Pt colloid catalyst- added electrolyzed water using methylene blue color change; FIG. 8 and FIG. 9 are diagrams showing reduction activity evaluation test results for Pt colloid catalyst- added hydrogen- dissolved water using methylene blue color change; FIG. 10 and FIG. 11 are diagrams showing reduction activity evaluation test results for Pd colloid catalyst- added hydrogen- dissolved water using methylene blue color change; FIG. 12 and FIG. 13 are diagrams showing reduction activity evaluation test results for mixed precious metal (Pt + Pd) colloid catalyst- added hydrogen- dissolved water using methylene blue color change; FIG. 14 is a diagram showing reduction activity evaluation test results for Pt colloid catalyst- added electrolyzed water (pre-electrolysis processing addition vs. post-electrolysis processing addition) using methylene blue color change; FIG. 15 and FIG. 16 are diagrams showing antioxidation activity evaluation test results for Pt colloid catalyst-added electrolyzed water using DPPH radical color change; FIG. 17 and FIG. 18 are diagrams showing antioxidation activity evaluation test results for catalyst- added hydrogen- dissolved water (degasification treatment + hydrogen gas inclusion treatment) using DPPH radical color change; FIG. 19 and FIG. 20 are diagrams showing reduction activity evaluation test results for enzyme hydrogenase catalyst- added hydrogen- dissolved water (degasification treatment + hydrogen gas inclusion treatment) using methylene blue color change; FIG. 21 and FIG. 22 are diagrams for describing a method for quantitative analysis of dissolved hydrogen concentration through redox titration with oxidation/ reduction pigment; FIG. 23 is a diagram for describing the comparison of the actually measured value and the effective value of the concentration of dissolved hydrogen DH in each type of sample water; FIG. 24 is a diagram for describing a cytochrome (c) reduction method; FIG. 25 is a diagram for describing an epinephrine oxidation method; FIG. 26 is a diagram showing characteristics of the radical scavenging activity expressed in Pt colloid catalyst-containing hydrogen-dissolved water changing over time using the Pt colloid concentration as a main parameter; FIG. 27 is a diagram showing characteristics of the radical scavenging activity expressed in Pd colloid catalyst-containing hydrogen-dissolved water changing over time using the Pd colloid concentration as a main parameter; FIG. 28 is a diagram showing characteristics of the radical scavenging activity expressed in Pt colloid catalyst-containing hydrogen-dissolved water changing over time using the Pt colloid concentration as a main parameter; FIG. 29 is a diagram showing characteristics of the radical scavenging activity expressed in mixed (Pt + Pd) colloid catalyst-containing hydrogen-dissolved water changing over time using the mixed (Pt + Pd) colloid concentration as a main parameter; FIG. 30 is a diagram showing characteristics of the radical scavenging activity expressed in mixed (Pt + Pd) colloid catalyst-containing hydrogen-dissolved water changing over time using the mixed (Pt + Pd) colloid concentration as a main parameter and a mixed mole ratio Pt : Pd as a sub parameter; FIG. 31 is a diagram showing characteristics of the radical scavenging activity expressed in mixed (Pt + Pd) colloid catalyst-containing hydrogen-dissolved water changing over time using the mixed (Pt + Pd) colloid concentration as a main parameter and a mixed mole ratio Pt : Pd as a sub parameter; FIG. 32 is a diagram showing characteristics of the radical scavenging activity expressed in pre-Pt colloid catalyst addition one-time electrolyzed water changing over time using Pt colloid concentration as a main parameter; FIG. 33 is a diagram showing characteristics of the radical scavenging activity expressed in pre- Pd colloid catalyst addition one-time electrolyzed water changing over time using the Pd colloid concentration as a main parameter; FIG. 34 is a diagram showing characteristics of the radical scavenging activity expressed in pre-Pt colloid catalyst addition circulating electrolyzed water changing over time using the Pt colloid concentration as a main parameter; FIG. 35 is a diagram showing characteristics of the radical scavenging activity expressed in pre- Pd colloid catalyst addition circulating electrolyzed water changing over time using the Pd colloid concentration as a main parameter; FIG. 36 is a diagram showing characteristics of the radical scavenging activity expressed in an AsA solution changing over time using the AsA solution concentration as a main parameter; FIG. 37 is a diagram showing characteristics of the radical scavenging activity expressed in catalyst-added hydrogen-dissolved water changing over time using differences in precious metal catalyst (fixed concentration) type as a main parameter; FIG. 38 is a diagram showing a working-action mechanism of the Pt colloid catalyst in a hydrogen-oxygen coexisting solution system; FIG. 39 is a diagram showing a working-action mechanism of the Pd colloid catalyst in the hydrogen-oxygen coexisting solution system; FIG. 40 is a diagram showing effective values of the dissolved-hydrogen concentration DH value according to an exemplary embodiment; and FIGS. 41 and 42 are diagrams showing influences of Pt colloid catalyst-containing two-time electrolyzed water (AOW) on the life span of C. elegans.

### BEST MODE FOR CARRYING OUT THE INVENTION

An exemplary embodiment of the present invention is described in detail forthwith while referencing the drawings.

To begin with, referencing FIG. 5, the basic structure of a reducing potential water generation apparatus 11 according to the present invention used for producing base water (hydrogen-dissolved water) in antioxidant-functioning water is described.

The reducing potential water generation apparatus 11 of this embodiment is formed with an inlet 111 for conducting raw water such as the deionized water, an outlet 112 for extracting the generated reducing potential water, and an electrolysis chamber 113 between the inlet 111 and the outlet 112. Although not limited to the following configuration, the reducing potential water generation apparatus 11 of this embodiment has the inlet 111 formed at the bottom of a casing 114 so as to allow conduction of raw water in a direction that is substantially perpendicular to the surface of the paper on which the drawing is shown. The outlet 112 is formed in the top portion of the casing 114 so as to allow intake of the electrolyzed water in a direction that is substantially perpendicular to the surface of the paper on which the drawing is shown.

In addition, a porous membrane 115 is provided on both the left and right inner walls of the reducing potential water generation apparatus 11, and an electrode plate 116 is provided outside each of these respective membranes 115. The other electrode plates 117 are provided inside the electrolysis chamber 113 with the respective principal surfaces thereof facing a corresponding electrode plate 116.

Thus there are two pairs of electrode plates facing each other, each having a membrane 115 sandwiched therebetween. These two pairs of electrode plates 116 and 117 are connected to a direct- current power source (power source circuit) 12; wherein an anode thereof is coupled to one of the plates in each pair of electrode plates 116 and 117, and a cathode thereof is coupled to the other electrode plate. When generating reducing potential water in the electrolysis chamber 113, for example as shown in FIG. 5, the cathodes of the direct- current power source 12 are connected to the electrode plates 117 arranged inside the electrolysis chamber 113, and the anodes are connected to the electrode plates 116 arranged outside the electrolysis chamber 113.

It should be noted that in the case of generating electrolyzed oxidation water in the electrolysis chamber 113, the anodes of the direct-current power source 12 may be connected to the electrode plates 117 arranged inside the electrolysis chamber 113, and the cathodes may be connected to the electrode plates 116 arranged outside the electrolysis chamber 113.

The electrode plates 116 and 117 used in this example are configured through baking and coating across the entire titanium material surface one or more combinations of precious metals selected from a group consisting of platinum, iridium, palladium, and the like. Furthermore, the electrode plates 116 and 117 have multiple punched holes as described later.

It is preferable that the membrane 115 used in this embodiment have properties that allow easy permeation of water flowing through the electrolysis chamber 113 yet allow little permeated water to leak out. More specifically, with the reducing potential water generation apparatus 11 of this embodiment, during electrolysis the membrane 115 itself and the narrow space S between the membrane 115 and the electrode plate 116 forms a water screen, and electric current flows into both of the electrode plates 116 and 117 via this water screen. Accordingly, the water configuring this water screen is successively replaced, which becomes important since it increases the effectiveness of the electrolysis. In addition, if the water that permeates the membrane 115 leaks out from between the membrane 115 and the electrode plate 116, processing thereof becomes necessary, and therefore it is preferable that the membrane have water- holding properties strong enough to keep the permeated water from dripping down. However, when employing an exemplary solid electrolyte film as the membrane, since this solid electrolyte film itself has electrical conduction properties, the narrow space S formed between the membrane 115 and the electrode plate 116 may be omitted.

An exemplary membrane 115 may include a nonwoven polyester fabric or a polyethylene screen, and the film material may be a chlorinated ethylene or a polyfluorinated vinylidene and a titanium oxide or a polyvinyl chloride, and be a solid electrolyte film or a porous film having a thickness ranging between 0.1 and 0.3 mm, an average pore diameter ranging between 0.05 and 1.0 µm, and a permeable water rate that is no greater than 1.0 cc/cm²·min. If a cation exchange membrane is to be utilized for the membrane 115, then a cation exchange group perfluorosufonic acid film having a base material of polytetrafluoroethylene (e.g. the Nafion(R) Membrane made by DuPont(tm)), a copolymer consisting of a cation exchange group vinyl ether and tetrafluoroethylene (e.g. flemion film made by Asahi Glass Co.), or the like may be used.

Meanwhile, the distance between the respective pairs of mutually facing electrode plates 116 and 117 sandwiching such membranes 115 may range between 0 mm and 5.0 mm, and is more preferably 1.5 mm. Here, a distance of 0 mm between the electrode plates 116 and 117 denotes the exemplary case of using a zero gap electrode wherein electrode films are formed directly on both principal surfaces of the respective membranes 115, and means that there is a distance substantially equal to the thickness of a membrane 115. It is also allowable to use zero gap electrodes where an electrode is formed on only one of the principal surfaces of the respective membranes 115. In addition, in the case where such a zero gap electrode is employed, it is preferable that openings (e.g. punched holes) or space be provided for electrode plates 116 and 117 to allow the gas that develops from the electrode surface to be released to the back surface opposite the membrane 115. It should be noted that the configuration providing such openings or space in the electrode plates 116 and 117 may also be employed for the electrode plates arranged in the electrolysis tank shown in FIG. 5.

In addition, the distance between the electrode plates 117 and 117, while not specifically limited, may range between 0.5 mm and 5 mm, and more preferably is 1 mm.

In order to generate reducing potential water using the reducing potential water generation apparatus 11 with such configuration, to begin with, the negative electrodes (-) of the direct- current power source 12 are connected to the two electrode plates 117 and 117 arranged inside the electrolysis chamber 113, the positive electrodes (+) of the direct- current power source 12 are connected to the electrode plates 116 and 116 arranged outside the electrolysis chamber 113, and voltage is applied to the two pairs of mutually facing electrode plates 116 and 117 sandwiching the respective membranes 115. As the deionized water, etc., is supplied from the inlet 111, electrolysis of water is performed in the electrolysis chamber 113, wherein the following reaction is occurring at the surface of the electrode plates 117 and in the vicinity thereof: ${\text{2H}}_{\text{2}} {\text{O + 2e}}^{\text{-}} {\text{→ 2OH}}^{\text{-}} {\text{+ H}}_{\text{2}} \text{↑}$Moreover, at the surface of the electrode plates 116 outside the electrolysis chamber 113 sandwiching the respective membranes 115, in other words between each electrode plate 116 and each membrane 115, the following reaction is occurring: ${\text{H}}_{\text{2}} {\text{O - 2e}}^{\text{-}} {\text{→ 2H}}^{\text{+}} {\text{+ 1/2 · O}}_{\text{2}} \text{↑}$

As this H⁺ ion permeates the membrane 115 and passes through, a part thereof accepts an electron e⁻ from the cathode plate 117 to become hydrogen gas dissolved in the generated electrolyzed water on the cathode side. This causes the electrolyzed water generated on the cathode side (i.e. inside the electrolysis chamber 113) to become reducing potential water having a lower oxidation/ reduction potential (ORP) than electrolyzed water generated using conventional membrane electrolysis technology.

In addition, since the remainder of the H⁺ ion passed through the membrane 115 reacts with the OH⁻ ion in the electrolysis chamber 113 and reverts to water, the pH of the reducing potential water generated with the electrolysis chamber 113 changes slightly towards neutrality. In other words, reducing potential water having a pH that is not very high yet having a low ORP is obtained. The reducing potential water including the hydroxide ion generated in this manner is supplied from the outlet 112.

It should be noted that when wanting to make the reducing potential water obtained through such electrolysis processing a certain desired pH level, the pH level of the raw water may be adjusted beforehand using a pH buffer acting salt solution such as phthalate, phosphate, or borate. This is because the pH of the raw water is not changed much with this reducing potential water generation apparatus 11. More specifically, for instance if a pH that tends towards alkalinity is wanted for intended applications such as rinsing silicon wafers or drinking, the pH level of the raw water may be managed and adjusted to approach alkalinity. If a pH that is substantially neutral for intended applications such as drinking, injection solution, intravenous drip solution, or dialysis fluid, the pH level of the raw water may be adjusted to be substantially neutral. Moreover, if a pH that is slightly acidic for intended applications such as cosmetics, the pH level of the raw water may be adjusted to approach slightly acidic levels.

While that shown in FIG. 5 has been described as an apparatus that generates reducing potential water in the embodiment described above, this apparatus 11 is also applicable to cases where oxidizing potential water is produced. In this case, the positive electrodes (+) of the direct- current power source 12 may be connected to the two electrode plates 117 and 117 arranged inside the electrolysis chamber 113, and the negative electrodes (-) of the direct- current power source 12 connected to the electrode plates 116 and 116 arranged outside the electrolysis chamber 113, to apply voltage to the two pairs of mutually facing electrode plates 116 and 117 sandwiching the respective membranes 115.

As the deionized water or the like is supplied from the inlet 111, electrolysis of the water is performed in the electrolysis chamber 113, wherein the following reaction is occurring at the surface of the electrode plates 117 and in the vicinity thereof: ${\text{H}}_{\text{2}} {\text{O -2e}}^{\text{-}} {\text{→ 2H}}^{\text{+}} \text{+ 1 / 2 · 02 ↑}$Meanwhile, at the surface of the electrode plates 116 outside the electrolysis chamber 113 sandwiching the respective membranes 115, in other words at the water screen between each electrode plate 116 and each membrane 115, the following reaction is occurring: ${\text{2H}}_{\text{2}} {\text{O + 2e}}^{\text{-}} {\text{→ 20H}}^{\text{-}} {\text{+ H}}_{\text{2}} \text{↑}$

As this OH· ion permeates the membrane 115 and passes through, a part thereof donates an electron e⁻ to the cathode plate 117 to become oxygen gas dissolved in the generated electrolyzed water on the anode side. This causes the electrolyzed water generated on the anode side (i.e. inside the electrolysis chamber 113) to become oxidizing potential water having a higher oxidation/ reduction potential (ORP) than electrolyzed water generated using conventional membrane electrolysis technology.

In addition, since the remainder of the OH· ion passed through the membrane 115 reacts with the H⁺ ion in the electrolysis chamber 113 and reverts to water, the pH of the oxidizing potential water generated with the electrolysis chamber 113 changes slightly towards neutrality. In other words, oxidizing potential water having a pH that is not very low yet having a high ORP is obtained. The oxidizing potential water including the hydrogen ion generated in this manner is supplied from the outlet 112.

Incidentally, continuous water flow electrolysis processing using the reducing potential water generation apparatus 11 shown in FIG. 5 was carried out under electrolysis conditions where the cathodes (-) of the direct-current power source 12 are connected to the two electrode plates 117 and 117 arranged inside the electrolysis chamber 113, the anodes (+) of the direct- current power source 12 are connected to the electrode plates 116 and 116 arranged outside the electrolysis chamber 113 (electrode plate effective surface area is 1 dm²), and a 5 A constant current is passed through Fujisawa City tap water having a pH of 7.9, ORP of 473 mV and flowing at a rate of 1 liter per minute (a preferable flowing rate in the present reducing potential water generation apparatus 11 is 1 through 3 liters per minute, more preferably, 1 through 1.8 liters per minute). Here, a cation-exchange film made by DuPont(tm), the Nafion(R) Membrane, was used as the membrane 115, the distance between the electrode plates 116 and 117 was 1.2 mm, and the distance between the electrode plates 117 and 117 inside the electrolysis chamber 113 was 1.4 mm.

As a result, a reducing potential water with a pH of 9.03 and ORP of -720 mV was obtained immediately following electrolysis processing. This reducing potential water was left to stand and the pH and ORP were measured after 5 minutes, 10 minutes, and 30 minutes. The following results were obtained: after 5 minutes, pH = 8.14 and ORP = -706 mV; after 10 minutes, pH = 8.11 and ORP = -710 mV; and after 30 minutes, pH = 8.02 and ORP = -707 mV. In other words, at the time point immediately following electrolysis processing, the pH of the processing water was higher than 9 but then the pH dropped shortly thereafter, and stabilized near pH 8. This is considered as being caused by the fact that the H⁺ ion generated near the water screen between the membrane 115 and the anode plate 116 passes through the membrane 115, moves to the electrolysis chamber 113, and then undergoes a neutralization reaction with the OH⁻ ion in this electrolysis chamber 113 to revert to the previous water. This neutralization reaction progresses with time to reach chemical equilibrium in concentration, even when the reducing potential water is left standing following electrolysis processing.

### Reduction activity/ radical scavenging evaluation testing for precious metal colloid catalyst added hydrogen- dissolved water

In the following, various evaluation tests of reduction activity and radical scavenging activity as expressed through the chemical activation of inert molecular hydrogen in hydrogen- dissolved water when a precious metal colloid catalyst (platinum (Pt) colloid/ palladium (Pd) colloid) is added to the hydrogen- dissolved water of the present invention are shown through both working examples and reference examples, respectively.

In the two forms of evaluation testing mentioned above, the reduction activity evaluation testing uses methylene blue (tetramethylthionine chloride: C₁₆H₁₈ClN3S • 3(H₂O)) as the antioxidation subject; on the other hand, in the radical scavenging activity evaluation testing, a radical that is relatively stable in aqueous solution, the DPPH radical (1,1-diphenyl-2-picrylhydrazyl) is used as the antioxidation subject.

Here, to describe the principle behind reduction activity evaluation for the case where methylene blue, which is categorized as an oxidation/ reduction pigment, is used as the antioxidation subject, the oxidized methylene blue solution (local maximum absorption wavelength of approximately 665 nm; hereafter methylene blue is also referred to as 'MB') takes on a blue color, however, when this is subjected to reduction and becomes reduced methylene blue (leucomethylene blue), the color changes from the blue color to being colorless. The degree to which this blue color disappears estimates the reduction activity or in other words, the reducing power. It should be noted that while the reduced methylene blue produces a white deposit due to low solubility, as it becomes oxidized again, it becomes oxidized methylene blue and the blue color returns. That is, the color change reaction of the methylene blue solution is reversible.

Meanwhile, to describe the principle behind radical scavenging activity evaluation for the case where a DPPH radical is used as the antioxidation subject, the DPPH radical solution (local maximum absorption wavelength of approximately 520 nm; hereafter may be referred to as 'DPPH') takes on a deep red color, and as this DPPH is reduced and no longer a radical, this deep red color fades. The degree to which the color fades estimates the radical scavenging activity or in other words, the antioxidation power. It should be noted that the color change reaction of the DPPH radical solution is nonreversible.

The description of these evaluation tests will be made in the following order:
(1) Reduction activity evaluation of Pt colloid catalyst-added electrolyzed water using methylene blue color change
(2) Reduction activity evaluation of Pt colloid/ Pd colloid catalyst-added hydrogen- dissolved water (degasification treatment + hydrogen gas inclusion treatment) using methylene blue color change
(3) Reduction activity evaluation of Pt colloid catalyst-added electrolyzed water (pre-electrolysis processing addition/ post-electrolysis processing addition) using methylene blue color change
(4) Antioxidation activity evaluation of Pt colloid catalyst-added electrolyzed water using color change of the DPPH radical
(5) Antioxidation activity evaluation of catalyst- added hydrogen- dissolved water (degasification treatment + hydrogen gas inclusion treatment) using color change of the DPPH radical (1) Reduction activity evaluation of Pt colloid catalyst-added electrolyzed water using methylene blue color change

### (1-A): Reducing power evaluation test procedures

Standard buffer solutions 6.86 (phosphate solution) and 9.18 (borate solution) manufactured by Wako Pure Chemical Industries, Ltd. are respectively diluted to one- tenth strength in purified water to prepare pH buffer solutions. In the following, these two types of dilution water are respectively referred to as 'base water 6.86' and 'base water 9.18'. In addition, a solution having 0.6 g of a Tanaka Kikinzoku- manufactured platinum colloid (particle size distribution is 2 through 4 nm, including polyvinylpyrrolidone as a dispersion agent) 4% solution dissolved in 500 mL of distilled water manufactured by Wako Pure Chemical Industries, Ltd. is referred to as 'Pt standard solution'. It should be noted that the platinum component concentration C(Pt) in the Pt standard solution becomes a 48 mg/L concentration using the formula C(Pt) = 0.6 g x 0.04 / 500 mL. Then using either base water 6.86 or base water 9.18 of the two species described above with the Pt standard solution, a total of eight species of sample solution, four species each, are prepared. These are described below:
i. base water (6.86)
ii. Pt colloid- containing solution, where 6 mL of Pt standard solution is added to 1494 mL of base water (6.86)
iii. a solution where base water (6.86) has been subjected to electrolysis processing
iv. a solution where 6 mL of Pt standard solution is added to 1494 mL of base water (6.86) to make a Pt colloid- containing solution, and this solution is subjected to electrolysis processing
v. base water (9.18)
vi. Pt colloid- containing solution, where 6 mL of Pt standard solution is added to 1494 mL of base water (9.18)
vii. a solution where base water (9.18) has been subjected to electrolysis processing
viii. a solution where 6 mL of Pt standard solution is added to 1494 mL of base water (9.18) to make a Pt colloid- containing solution, and this solution is subjected to electrolysis processing

It should be noted that the pH, ORP (mV), temperature T (°C), and Pt colloid concentration for each sample solution of the total 8 described above in i through viii are collectively shown in the following table 2.

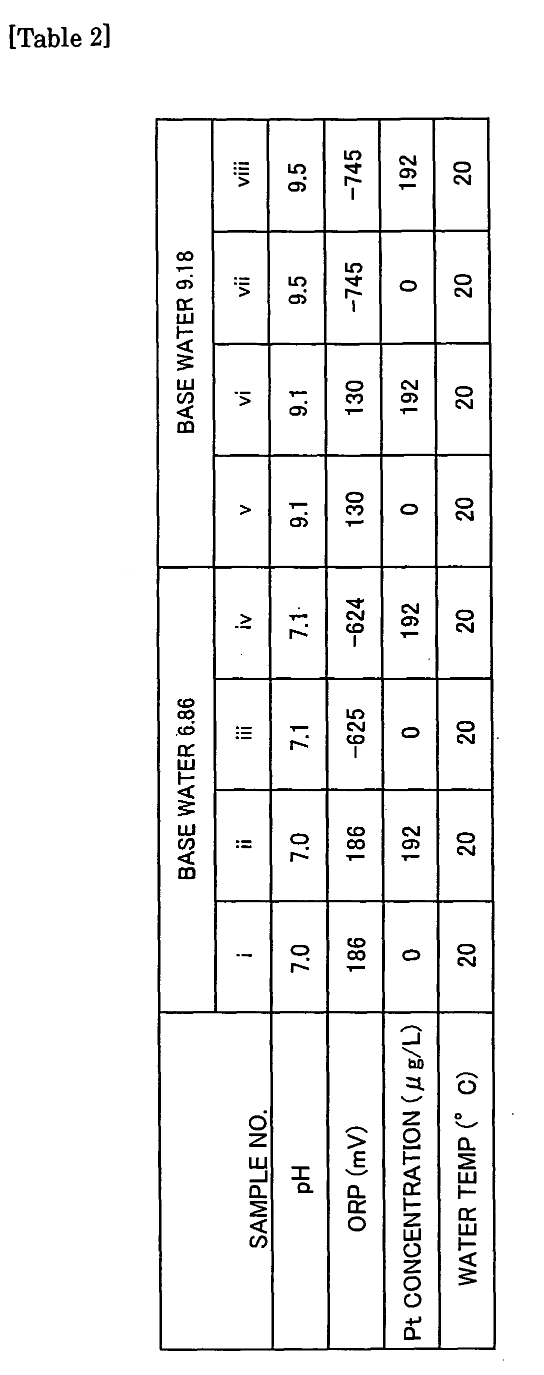

In order to examine the respective reduction activity of each sample solution of the total 8 described above in i through viii, 10 mL of methylene blue (1 g/ L concentration) is added to 350 mL of each solution to prepare a methylene blue mole concentration of 74.4 µM, and the methylene blue light absorbance (A589: the light absorbance at wavelength 589 nm) of each sample solution is measured using a spectrophotometer.

### (1-B): Disclosure of reference examples and working examples

### (Reference example 1)

The methylene blue light absorbance (A589) of a solution where methylene blue is added to the catalyst- free solution (base water 6.86) of sample i is given as reference example 1, and the result thereof is shown in FIG. 6.

### (Reference example 2)

The methylene blue light absorbance (A589) of a solution where methylene blue is added to the catalyst- added solution (base water 6.86 + Pt standard solution) of sample ii is given as reference example 2, and the result thereof is shown in FIG. 6.

### (Reference example 3)

The methylene blue light absorbance (A589) of a solution where methylene blue is added to the catalyst- free electrolyzed water (base water 6.86 + electrolysis processing) of sample iii is given as reference example 3, and the result thereof is shown in FIG. 6.

### (Working example 1)

The methylene blue light absorbance (A589) of a solution where methylene blue is added to the catalyst- added electrolyzed water (base water 6.86 + electrolysis processing + Pt standard solution) of sample iv is given as working example 1, and the result thereof is shown in FIG. 6 for comparison with reference examples 1 through 3.

### (Reference example 4)

The methylene blue light absorbance (A589) of a solution where methylene blue is added to the catalyst- free solution (base water 9.18) of sample v is given as reference example 4, and the result thereof is shown in FIG. 7.

### (Reference example 5)

The methylene blue light absorbance (A589) of a solution where methylene blue is added to the catalyst- added solution (base water 9.18 + Pt standard solution) of sample vi is given as reference example 5, and the result thereof is shown in FIG. 7.

### (Reference example 6)

The methylene blue light absorbance (A589) of a solution where methylene blue is added to the catalyst- free electrolyzed water (base water 9.18 + electrolysis processing) of sample vii is given as reference example 6, and the result thereof is shown in FIG. 7.

### (Working example 2)

The methylene blue light absorbance (A589) of a solution where methylene blue is added to the catalyst- added electrolyzed water (base water 9.18 + electrolysis processing + Pt standard solution) of sample viii is given as working example 2, and the result thereof is shown in FIG. 7 for comparison with reference examples 4 through 6.

### (1-C): Examination of working examples

Examining the results of working examples 1 and 2 in comparison with those of reference examples 1 through 6, it may be said that the catalyst- added electrolyzed waters of working examples 1 and 2 has the specific methylene blue reduced irrespective of the difference in pH thereof, yet only the catalyst- added electrolyzed water exhibits significant reduction activity. It should be noted that when it was checked with the human eye whether or not there had been a change in the blue color of the methylene blue solution, only the catalyst- added electrolyzed waters of working examples 1 and 2 were colorless and clear, allowing visual confirmation that the blue color of the methylene blue had disappeared. However, visual confirmation that the blue color of the methylene blue had disappeared could not be accomplished with reference examples 1 through 6. In addition, a large amount of white- colored deposit (reduced methylene blue) was visually confirmed for the catalyst- added hydrogen- dissolved waters of working examples 1 and 2.

### (2) Reduction activity evaluation of Pt colloid/ Pd colloid catalyst-added hydrogen- dissolved water (degasification treatment + hydrogen gas inclusion treatment) using methylene blue color change

### (2-A): Reducing power evaluation test procedures

Solutions of Tris-HCl with a concentration of 50mM are prepared by respectively diluting a special order 1M Tris-HCl (pH 7.4) and a special order 1M Tris-HCl (pH 9.0) manufactured by Nippon Gene Co., Ltd. and sold by Wako Pure Chemical Industries, Ltd. to one- twentieth strength with distilled water manufactured by Wako Pure Chemical Industries, Ltd. In the following, these two types of dilution water are respectively referred to as 'base water 7.4' and 'base water 9.0'. In addition, a solution having 0.6 g of a Tanaka Kikinzoku- manufactured palladium colloid (particle size distribution is 2 to 4 nm, including polyvinylpyrrolidone as a dispersion agent) 4% solution dissolved in 500 mL of distilled water manufactured by Wako Pure Chemical Industries, Ltd. is referred to as 'Pd standard solution'. It should be noted that the palladium component concentration C(Pd) in the Pd standard solution becomes a 48 mg/L concentration using, from the same formula as the Pt colloid, C(Pd) = 0.6 g x 0.04 / 500 mL.

Next, collecting 84 mL of base water 7.4 and base water 9.0, respectively, 4 mL of MB solution in 1 g/L concentration is added to each to prepare base water 7.4 and base water 9.0 that respectively contain a 121.7 µM concentration of methylene blue (MB). 50 mL of each of these MB-containing base waters 7.4 and 9.0 are further collected into individual degasification bottles and subjected three times to a process that includes 10 minute degasification with a vacuum pump followed by 10 minute hydrogen gas inclusion. This process aims to remove gaseous components other than hydrogen from the hydrogen- dissolved water.

3 mL of the respective hydrogen gas- included, MB- containing base water 7.4 and base water 9.0 obtained in this manner is collected and poured into respective sealed, hydrogen gas- replaced, quartz cells. Measurements are then taken of the change in methylene blue light absorbance (Δ A572: change in light absorbance at wavelength 572 nm) that occurs when the Pt reference solution, Pd standard solution, or mixed solution of Pt standard solution and Pd standard solution with a mole ratio of approximately 1 is respectively added to the quartz cells.

### (2-B): Disclosure of working examples

### (Working example 3)

The change in MB light absorbance (Δ A572) in a solution where an amount of Pt standard solution sufficient to give a Pt colloid concentration of 190 µg/L has been added to MB- containing hydrogen- dissolved water (MB-containing base water 7.4 + degasification treatment + hydrogen gas inclusion treatment) is given as working example 3, and the result thereof is shown in both FIG. 8 and FIG. 9.

### (Working example 4)

The change in MB light absorbance (Δ A572) in a solution where an amount of Pt standard solution sufficient to give a Pt colloid concentration of 190 µg/L has been added to MB- containing hydrogen- dissolved water (MB-containing base water 9.0 + degasification treatment + hydrogen gas inclusion treatment) is given as working example 4, and the result thereof is shown in FIG. 8 for comparison with working example 3. It should be noted that the difference between the sample waters of working example 3 and working example 4 is the pH.

### (Working example 5)

The change in MB light absorbance (Δ A572) in a solution where an amount of Pt standard solution sufficient to give a Pt colloid concentration of 95 µg/L has been added to MB- containing hydrogen- dissolved water (MB-containing base water 7.4 + degasification treatment + hydrogen gas inclusion treatment) is given as working example 5, and the result thereof is shown in FIG. 9 for comparison with working example 3. It should be noted that the difference between the sample waters of working example 3 and working example 5 is the Pt colloid concentration.

### (Working example 6)

The change in MB light absorbance (Δ A572) in a solution where an amount of Pd standard solution sufficient to give a palladium colloid concentration of 444 µg/L has been added to MB- containing hydrogen-dissolved water (MB- containing base water 7.4 + degasification treatment + hydrogen gas inclusion treatment) is given as working example 6, and the result thereof is shown in both FIG. 10 and FIG. 11.

### (Working example 7)

The change in MB light absorbance (Δ A572) in a solution where an amount of Pd standard solution sufficient to give a palladium colloid concentration of 444 µg/L has been added to MB- containing hydrogen-dissolved water (MB- containing base water 9.0 + degasification treatment + hydrogen gas inclusion treatment) is given as working example 7, and the result thereof is shown in FIG. 10 for comparison with working example 6. It should be noted that the difference between the sample waters of working example 6 and working example 7 is the pH.

### (Working example 8)

The change in MB light absorbance (Δ A572) in a solution where an amount of Pd standard solution sufficient to give a palladium colloid concentration of 111 µg/L has been added to MB- containing hydrogen-dissolved water (MB- containing base water 7.4 + degasification treatment + hydrogen gas inclusion treatment) is given as working example 8, and the result thereof is shown in FIG. 11 for comparison with working example 6. It should be noted that the difference between the sample waters of working example 6 and working example 8 is the palladium colloid concentration.

### (Working example 9)

The change in MB light absorbance (Δ A572) in a solution where an amount of a mixed solution of Pt standard solution and Pd standard solution with a mole ratio of approximately 1 sufficient to give a precious metal mixed (Pt + Pd) colloid concentration of 160 µg/L has been added to MB- containing hydrogen- dissolved water (MB- containing base water 7.4 + degasification treatment + hydrogen gas inclusion treatment) is given as working example 9, and the result thereof is shown in both FIG. 12 and FIG. 13.

### (Working example 10)

The change in MB light absorbance (Δ A572) in a solution where an amount of mixed solution, similar to working example 9, sufficient to give a precious metal mixed (Pt + Pd) colloid concentration of 160 µg/L has been added to MB- containing hydrogen- dissolved water (MB- containing base water 9.0 + degasification treatment + hydrogen gas inclusion treatment) is given as working example 10, and the result thereof is shown in FIG. 12 for comparison with working example 9. It should be noted that the difference between the sample waters of working example 9 and working example 10 is the pH.

### (Working example 11)

The change in MB light absorbance (Δ A572) in a solution where an amount of mixed solution, similar to working example 9, sufficient to give a precious metal mixed (Pt + Pd) colloid concentration of 80 µg/L has been added to MB- containing hydrogen- dissolved water (MB- containing base water 7.4 + degasification treatment + hydrogen gas inclusion treatment) is given as working example 11, and the result thereof is shown in FIG. 13 for comparison with working example 9. It should be noted that the difference between the sample waters of working example 9 and working example 11 is the precious metal (Pt + Pd) colloid concentration.

### (2-C): Examination of working examples

FIG. 8, which compares working examples 3 and 4, shows the MB reduction activity of Pt colloid- added hydrogen- dissolved water occurring at pH 7.4 and pH 9.0. According to this diagram, both examples show high levels of MB reduction activity without seeing a substantial difference in MB reduction activity due to difference in pH.

FIG. 9, which compares working examples 3 and 5, shows the MB reduction activity of Pt colloid- added hydrogen- dissolved water occurring at Pt colloid concentrations of 95 µg/L and 190 µg/L. According to this diagram, the higher Pt colloid concentration also has higher MB reduction activity. From this, an increase in Pt colloid concentration may be considered effective towards increasing MB reduction activity.

FIG. 10, which compares working examples 6 and 7, shows the MB reduction activity of Pd colloid- added hydrogen- dissolved water occurring at pH 7.4 and pH 9.0. According to this diagram, both examples show high levels of MB reduction activity without seeing a substantial difference in MB reduction activity due to difference in pH.

FIG. 11, which compares working examples 6 and 8, shows the MB reduction activity of Pd colloid- added hydrogen- dissolved water occurring at Pd colloid concentrations of 111 µg/L and 444 µg/L. According to this diagram, the higher Pd colloid concentration also has higher MB reduction activity. From this, an increase in Pd colloid concentration may be considered effective towards increasing MB reduction activity.

FIG. 12, which compares working examples 9 and 10, shows the MB reduction activity of precious metal mixed (Pt + Pd) colloid- added hydrogen-dissolved water occurring at pH 7.4 and pH 9.0. According to this diagram, both examples show high levels of MB reduction activity without seeing a substantial difference in MB reduction activity due to difference in pH.

FIG. 13, which compares working examples 9 and 11, shows the MB reduction activity of precious metal mixed (Pt + Pd) colloid- added hydrogen-dissolved water occurring at precious metal mixed (Pt + Pd) colloid concentrations of 80 µg/L and 160 µg/L. According to this diagram, the higher precious metal mixed (Pt + Pd) colloid concentration also has higher MB reduction activity. From this, an increase in precious metal mixed (Pt + Pd) colloid concentration may be considered effective towards increasing MB reduction activity.

In addition, comparing FIG. 8 (working examples 3 and 4: MB reduction activity of Pt colloid- added hydrogen- dissolved water) and FIG. 10 (working examples 6 and 7: MB reduction activity of Pd colloid- added hydrogen- dissolved water), it may be understood that although working examples 3 and 4 have lower concentrations, these show substantially the same MB reduction activity as working examples 6 and 7. Moreover, comparing the mole concentrations (µM) of both, since the Pt colloid is 0.98 µM and the Pd colloid 4.17 µM, the Pt colloid uses a lower mole concentration. This means that regarding MB reduction activity expected for the precious metal catalyst according to the present invention, it may be said that the Pt colloid is superior to the Pd colloid in terms of catalytic activity because substantially the same MB reduction activity can be obtained with a smaller dosage.

Meanwhile, comparing FIG. 8 (working examples 3 and 4: MB reduction activity of Pt colloid- added hydrogen- dissolved water) and FIG. 12 (working examples 9 and 10: MB reduction activity of precious metal mixed (Pt + Pd) colloid- added hydrogen- dissolved water), it may be understood that both show superior MB reduction activity. Even comparing the mole concentrations (µM) of both, since the Pt colloid is 0.98 µM and the precious metal mixed (Pt + Pd) colloid 1.07 µM, both are substantially the same. Therefore, regarding MB reduction activity expected for the precious metal catalyst according to the present invention, the Pt colloid and the precious metal mixed (Pt + Pd) colloid are substantially the same in terms of catalytic activity.

### (3) Reduction activity evaluation of Pt colloid catalyst-added electrolyzed water (pre- electrolysis processing addition/ post- electrolysis processing addition) using methylene blue color change

### (3-A): Reducing power evaluation test procedures

2000 mL of base water 6.86 similar to that prepared in (1-A) described above is prepared, and 4 mL of Pt standard solution from this is added to 1000 mL to prepare approximately 1 liter of Pt colloid- containing base water 6.86. For the time being, the Pt colloid is not added to the remaining 1000 mL. In this manner, approximately 1 liter of Pt colloid- free base water 6.86 and approximately 1 liter of Pt colloid- containing base water 6.86 are prepared.

Next, both of the samples are subjected to electrolysis processing separately. 2.86 mL of the respective obtained electrolyzed waters (hydrogen- dissolved water) is collected and poured into respective sealed, hydrogen gas- replaced quartz cells.

Moreover, only 0.14 mL of the 1 g/L concentration MB solution that has been degasified and hydrogen gas included beforehand is added to the Pt colloid- free cell. At this point, both cells are set in the spectrophotometer and placed on stand-by.

Next, 12 µL in a 48 mg/L concentration of Pt colloid solution is added to the Pt colloid- free cell, and into the Pt colloid- containing cell, 0.14 mL of 1 g/L concentration MB solution that has already been through degasification treatment and hydrogen gas inclusion treatment is added, and measurement of both cell solutions is begun. It should be noted that the Pt colloid concentrations added to each cell are prepared so that each respectively becomes approximately 182 µg/L.

### (3-B): Disclosure of working examples

### (Working example 12)

The minimum value of MB light absorbance (A572: the light absorbance at wavelength 572 nm) of the pre-catalyst addition electrolyzed water (MB- containing base water 6.86 + Pt colloid pre-electrolysis addition) that occurs within 30 minutes from the start of measurement is given as working example 12, and the result thereof is shown in FIG. 14.

### (Working example 13)

The minimum value of MB light absorbance (A572) of the post-catalyst addition electrolyzed water (MB- containing base water 6.86 + Pt colloid post-electrolysis addition) that occurs within 30 minutes from the start of measurement is given as working example 13, and the result thereof is shown in FIG. 14 for comparison with working example 12.

### (3-C): Examination of working examples

FIG. 14, which compares working examples 12 and 13, shows the MB reduction activity of electrolyzed water when the period of adding the Pt colloid is different (before vs. after electrolysis processing). According to this diagram, it may be understood that adding the Pt colloid before electrolysis processing allows higher MB reduction activity to be obtained. The reason for this is still being studied, however it is speculated that this stems from the activated hydrogen at the root of the MB reduction activity making the oxidizing power of the oxidant such as oxygen in the electrolyzed water ineffective. This is the reason derived from the fact that when the dissolved oxygen concentration of the electrolyzed water on which electrolysis processing had been implemented using Pt colloid -containing activated carbon processing water as the raw water was measured immediately after electrolysis processing thereof, the concentration of dissolved oxygen in this electrolyzed water was found to be substantially zero. Should this be the case, not only in this exemplary electrolysis processing, but also in hydrogen inclusion treatment or hydrogen gas bubbling processing, pre-processing addition of the catalyst (Pt colloid) is considered preferable from the standpoint that higher levels of MB reduction activity are obtained (because of the oxidizing power of the oxidant such as oxygen being made ineffective). Moreover, even in the case of obtaining dissolved hydrogen water by employing processing where, for instance, a reducing agent is added to the raw water, addition of the Pt colloid to the raw water beforehand is considered preferable from the standpoint that higher levels of MB reduction activity similar to that described above may be obtained. It should be noted that the catalyst is not limited to the Pt colloid. Pre-processing addition of a catalyst such as Pd colloid, or mixed colloid of Pt colloid and Pd colloid is similarly preferable from the standpoint of obtaining higher levels of MB reduction activity (catalytic activity). This emanates from the fact that hydrogen may be efficiently occluded into a precious metal colloid catalyst during electrolysis processing when the precious metal colloid catalyst is added prior to that electrolysis processing, and the hydrogen stored in the precious metal colloid catalyst allows even higher levels of MB reduction activity (catalytic activity).

### (4) Antioxidation activity evaluation of Pt colloid catalyst-added electrolyzed water using color change of the DPPH radical

### (4-A): Antioxidation activity evaluation test procedures

In order to examine the respective antioxidation activity of each sample solution of the total 8 samples i through viii shown in table 2, similar to that prepared in (1-A) above, 4 mL of DPPH (0.16 g/L concentration) is added to 16 mL of each solution to prepare a DPPH mole concentration of 81.15 (µM), and the change in DPPH light absorbance (A540: the light absorbance at wavelength 540 nm) of each solution 3 minutes after adding the DPPH is measured using a spectrophotometer.

### (4-B): Disclosure of reference examples and working examples

### (Reference example 7)

The change in DPPH light absorbance (Δ A540) of a solution where DPPH is added to the catalyst- free solution (base water 6.86) of sample i is given as reference example 7, and the result thereof is shown in FIG. 15. It should be noted that the change in DPPH light absorbance (Δ A540) in the same drawing shows the difference (Δ A540) between the light absorbance of this sample i (blank) and the light absorbance of samples i through iv. Accordingly, the change in DPPH light absorbance (Δ A540) for reference example 7 is zero.

### (Reference example 8)

The change in DPPH light absorbance (Δ A540) of a solution where DPPH is added to the catalyst- added solution (base water 6.86 + Pt standard solution) of sample ii is given as reference example 8, and the result thereof is shown in FIG. 15.

### (Reference example 9)

The change in DPPH light absorbance (Δ A540) of a solution where DPPH is added to the catalyst- free solution (base water 6.86 + electrolysis processing) of sample iii is given as reference example 9, and the result thereof is shown in FIG. 15.

### (Working example 14)

The change in DPPH light absorbance (Δ A540) of a solution where DPPH is added to the catalyst- added electrolyzed water (base water 6.86 + electrolysis processing + Pt standard solution) of sample iv is given as working example 14, and the result thereof is shown in FIG. 15 for comparison with reference examples 7 through 9.

### (Reference example 10)

The change in DPPH light absorbance (Δ A540) of a solution where DPPH is added to the catalyst- free solution (base water 9.18) of sample v is given as reference example 10, and the result thereof is shown in FIG. 16. It should be noted that the change in DPPH light absorbance (Δ A540) in the same drawing shows the difference (Δ A540) between the light absorbance of this sample v (blank) and the light absorbance of samples v through viii. Accordingly, the change in DPPH light absorbance (Δ A540) for reference example 10 is zero.

### (Reference example 11)

The change in DPPH light absorbance (Δ A540) of a solution where DPPH is added to the catalyst- added solution (base water 9.18 + Pt standard solution) of sample vi is given as reference example 11, and the result thereof is shown in FIG. 16.

### (Reference example 12)

The change in DPPH light absorbance (Δ A540) of a solution where DPPH is added to the catalyst- free electrolyzed water (base water 9.18 + electrolysis processing) of sample vii is given as reference example 12, and the result thereof is shown in FIG. 16.

### (Working example 15)

The change in DPPH light absorbance (Δ A540) of a solution where DPPH is added to the catalyst- added electrolyzed water (base water 9.18 + electrolysis processing + Pt standard solution) of sample viii is given as working example 15, and the result thereof is shown in FIG. 16 for comparison with reference examples 10 through 12.

### (4-C): Examination of working examples

Examining the results of working examples 14 and 15 in comparison with those of reference examples 7 through 12, it may be said that the catalyst- added electrolyzed waters of working examples 14 and 15 has the specific DPPH radical scavenged with both base waters 6.86 and 9.18, and shows significant antioxidation activity and radical scavenging activity. However, the Pt colloid catalyst was added before electrolysis processing. It should be noted that, as shown in FIG. 15, DPPH radical scavenging activity is found in reference example 9 even though catalyst- free electrolyzed water is used. This indicates that with high-concentration hydrogen-dissolved water such as electrolyzed water disclosed in this specification, when an oxidation substance to be reduced is a radical having a strong oxidizing power, an expression of radical scavenging activity is expected to occur through forcible hydrogen degasification reaction from molecular hydrogen with that radical, even without the assistance of a catalyst.

### (5) Antioxidation activity evaluation of catalyst-added hydrogen-dissolved water (degasification treatment + hydrogen gas inclusion treatment) using color change of the DPPH radical

### (5-A): Antioxidation activity evaluation test procedures

'Base water 7.4' and 'base water 9.0' are prepared as with that prepared in (2-A) above. Next, 406 µM of DPPH solution and 50 mL each of base water 7.4 and base water 9.0 are collected and subjected three times to a process that includes 10 minute degasification with a vacuum pump followed by 10 minutes of hydrogen gas inclusion. This process aims to remove gaseous components other than hydrogen from the hydrogen-dissolved water.

0.3 mL of the hydrogen gas- included DPPH solution obtained in this manner, and 2.7 mL each of base water 7.4 and base water 9.0 are collected and poured into respective sealed, hydrogen gas- replaced, quartz cells. Measurements of the change in DPPH light absorbance (Δ A540: change in light absorbance at wavelength 540 nm) for both that to which the Pt standard solution has been added and that to which it has not are then taken over 30 minutes respectively using a spectrophotometer.

### (5-B): Disclosure of reference examples and working examples

### (Reference example 13)

The change in DPPH light absorbance (ΔA540) of a solution where Pt standard solution has not been added to the hydrogen- dissolved water (base water 7.4 + degasification treatment + hydrogen gas inclusion treatment) is given as reference example 13, and the result thereof is shown in FIG. 17.

### (Working example 16)

The change in DPPH light absorbance (Δ A540) in a solution where an amount of Pt standard solution sufficient to give a Pt colloid concentration of 190 µg/L has been added to hydrogen- dissolved water (base water 7.4 + degasification treatment + hydrogen gas inclusion treatment) is given as working example 16, and the result thereof is shown in FIG. 17 for comparison with reference example 13. It should be noted that the difference between reference example 13 and working example 16 is whether or not the Pt colloid has been added.

### (Reference example 14)

The change in DPPH light absorbance (Δ A540) of a solution where Pt standard solution has not been added to the hydrogen- dissolved water (base water 9.0 + degasification treatment + hydrogen gas inclusion treatment) is given as reference example 14, and the result thereof is shown in FIG. 18.

### (Working example 17)

The change in DPPH light absorbance (Δ A540) in a solution where an amount of Pt standard solution sufficient to give a Pt colloid concentration of 190 µg/L has been added to hydrogen- dissolved water (base water 9.0 + degasification treatment + hydrogen gas inclusion treatment) is given as working example 17, and the result thereof is shown in FIG. 18 for comparison with reference example 14. It should be noted that the difference between reference example 14 and working example 17 is whether or not the Pt colloid has been added.

### (5-C): Examination of working examples

FIG. 17, which compares reference example 13 and working example 16, shows the DPPH radical scavenging activity in pH 7.4 hydrogen-dissolved water where the difference is whether or not the Pt colloid is added.

FIG. 18, which compares reference example 14 and working example 17, shows the DPPH radical scavenging activity in pH 9.0 hydrogen-dissolved water where the difference is whether or not the Pt colloid is added. According to these diagrams, with the Pt colloid- free reference examples 13 and 14, the change in light absorbance seen may be considered as only that corresponding to natural fading during the duration of measurement (30 minutes). Meanwhile, with the Pt colloid- containing working examples 16 and 17, the expression of DPPH radical scavenging that clearly surpasses natural fading is observed. It should be noted that there was no substantial difference observed in levels of DPPH radical scavenging due to difference in pH.

### Reduction activity evaluation testing of enzyme hydrogenase catalyst-added hydrogen- dissolved water

Next, evaluation of reduction activity as expressed through the chemical activation of inert molecular hydrogen in hydrogen- dissolved water when an enzyme hydrogenase catalyst is added to the hydrogen- dissolved water of the present invention is shown through both working examples and reference examples, respectively. In this reduction activity evaluation test, the oxidation/reduction pigment methylene blue is used as the antioxidation subject as with the reduction activity testing for precious metal colloid catalyst- added hydrogen- dissolved water. Since the reduction activity evaluation principle in this case is similar to that described for the precious metal colloid catalyst above, repetitive description thereof is omitted. (6) Reduction activity evaluation of enzyme hydrogenase catalyst-added hydrogen- dissolved water (degasification treatment + hydrogen gas inclusion treatment) using methylene blue color change

### (6-A): Reduction activity evaluation test procedures

In the same manner as that prepared in (2-A) above, 'base water 7.4' and 'base water 9.0' are prepared. Next, collecting 84 mL of each of base water 7.4 and base water 9.0, respectively, 4 mL of MB solution in 1 g/L concentration is added to each to prepare base water 7.4 and base water 9.0 that respectively contain a 121.7 µM concentration of methylene blue (MB). 50 mL of each of these MB- containing base waters 7.4 and 9.0 are further collected and subjected three times to a process that includes 10 minute degasification with a vacuum pump followed by 10 minute hydrogen gas inclusion. This process aims to remove gaseous components other than hydrogen from the hydrogen- dissolved water. Meanwhile, a 125 µM concentration of hydrogenase solution is diluted with distilled water to one-fourth strength. This is then poured into 1 mL microcapsules and the oxygen is removed by infusing these capsules with nitrogen gas (inert gas).

3 mL of the respective hydrogen gas- included, MB- containing base water 7.4 and base water 9.0 obtained in this manner is collected and poured into respective sealed, hydrogen gas- replaced, quartz cells. Measurements are then taken of the change in methylene blue light absorbance (Δ A572) that occurs when the hydrogenase solution prepared as described above is added to the quartz cells.

### (6-B): Disclosure of reference examples and working examples

### (Working example 18)

The change in MB light absorbance (Δ A572) in a solution where 10 µL of the hydrogenase solution prepared as described above has been added to MB- containing hydrogen- dissolved water (MB- containing base water 7.4 + degasification treatment + hydrogen gas inclusion treatment) is given as working example 18, and the result thereof is shown in FIG. 19.

### (Reference example 15)

The change in MB light absorbance (Δ A572) in a solution where the hydrogenase solution has not been added to MB- containing hydrogen-dissolved water (MB- containing base water 7.4 + degasification treatment + hydrogen gas inclusion treatment) is given as reference example 15, and the result thereof is shown in FIG. 19 for comparison with working example 18. It should be noted that the difference between the sample waters of working example 18 and reference example 15 is whether or not the enzyme hydrogenase has been added.

### (Working example 19)

The change in MB light absorbance (Δ A572) in a solution where 10 µL of the hydrogenase solution prepared as described above has been added to MB- containing hydrogen- dissolved water (MB- containing base water 9.0 + degasification treatment + hydrogen gas inclusion treatment) is given as working example 19, and the result thereof is shown in FIG. 20.

### (Reference example 16)

The change in MB light absorbance (Δ A572) in a solution where the hydrogenase solution has not been added to MB- containing hydrogen-dissolved water (MB- containing base water 9.0 + degasification treatment + hydrogen gas inclusion treatment) is given as reference example 16, and the result thereof is shown in FIG. 20 for comparison with working example 19. It should be noted that the difference between the sample waters of working example 19 and reference example 16 is whether or not the enzyme hydrogenase has been added.

### (6-C): Examination of working examples

Examining the results of working examples 18 and 19 in comparison with those of reference examples 15 and 16, it may be said that the catalyst-added hydrogen- dissolved waters of working examples 18 and 19 have the methylene blue specifically reduced irrespective of the difference in pH thereof, yet only the catalyst- added hydrogen- dissolved water exhibits significant reduction activity. It should be noted that when it was checked with the human eye whether or not there had been a change in the blue color of the methylene blue solution, only the catalyst- added hydrogen- dissolved waters of working examples 18 and 19 were colorless and clear, allowing visual confirmation that the blue color of the methylene blue had disappeared. However, visual confirmation that the blue color of the methylene blue had disappeared could not be accomplished with reference examples 15 and 16. In addition, a large amount of white- colored deposit (reduced methylene blue) was visually confirmed for the catalyst- added hydrogen- dissolved waters of working examples 18 and 19.

### Quantitative analysis of dissolved hydrogen concentration through oxidation/ reduction titration of oxidation/ reduction pigment

### (A) Development of idea

It has been proven that hydrogen generated through the negative electrode reaction during electrolysis processing is dissolved in the electrolyzed water (electrolyzed reducing water) that has been subjected to electrolysis processing in the reducing potential water generation apparatus 11 developed by the applicants. Approximately what concentration of hydrogen is dissolved in this electrolyzed water may be measured in a way with a dissolved hydrogen meter. Here, the expression 'in a way' is used because generally used dissolved hydrogen meters employ a measuring principle whereby electrochemical physical quantities occurring in the electrode reaction are replaced with the concentration of dissolved hydrogen using a table look-up protocol so that the readings tend to vary significantly depending on the external causes such as liquid properties of the test water.

However, as description was made based on the working examples already described above, with the catalyst- free electrolyzed water where no catalyst is added to the electrolyzed water, even when an oxidation/ reduction pigment (an antioxidation subject) such as oxidized methylene blue is added, this pigment does not show the color change specific to the reduction reaction; but on the other hand, with catalyst- added electrolyzed water where a catalyst has been added to the electrolyzed water, when this pigment is added, the pigment shows the color change specific to the reduction reaction. In other words, the oxidation/ reduction reaction of the oxidation/ reduction pigment may be visually recognized by observing the change in color of the solution (catalyst- added electrolyzed water + oxidation/ reduction pigment).

Through a process of trial and error as this testing was repeated, the inventors realized that the color change reaction of the oxidation/ reduction pigment methylene blue from blue to clear tended to occur more swiftly as the reducing power of the catalyst- added electrolyzed water increased. More specifically, when comparing the reducing power of the catalyst- added electrolyzed water and the reducing power consumed to reduce the oxidation/ reduction pigment methylene blue that is added, some sort of correlation was noticed between the size of the residual reducing power or the difference between the two reducing powers when the former is larger than the latter, and the speed of the color change reaction of the oxidation/ reduction pigment methylene blue.

In keeping with this discovery, as zealous research on the possible industrial utilization of this correlation progressed, the inventors ended up wondering if it was possible to perform quantitative analysis of the explicit antioxidation power (dissolved hydrogen concentration) of the catalyst-added electrolyzed water through the oxidation/ reduction reaction of the oxidation/ reduction pigment methylene blue.

It has been noticed that a dissolved hydrogen concentration quantitative analysis method can be provided as a means of realizing the above-mentioned idea comprising the following: dripping a solution of the oxidation/ reduction pigment into a predetermined amount of test water, which includes a precious metal colloid catalyst during titration under the condition of being isolated from the outside environment; calculating the amount of dissolved hydrogen in test water from the dripped amount of oxidation/ reduction pigment up to the end point of the pigment color change through reduction reaction of that oxidation/ reduction pigment employing the precious metal colloid catalyst; and performing quantitative analysis of the dissolved hydrogen concentration in test water based on the predetermined amount of test water and the dripped amount of pigment. In addition, it also has been noticed that as an apparatus for performing that analysis, a dissolved hydrogen concentration quantitative analysis apparatus or a gas-impermeable tester isolated from the outside environment is suitably used comprising: a cylinder-shaped tube having a closed and an open end and a pusher capable of being inserted into the cylinder-shaped tube from the open end in a piston-like manner where a stirrer is movable; wherein a solution injection part is deployed on any one of the closed end, the inside wall, or the pusher of this cylinder-shaped tube so as to inject a solution into the test water holding compartment demarcated by the closed end, the inside wall and the pusher of this cylinder-shaped tube under the condition of being isolated from the outside environment. It should be noted that as a modified example of the quantitative analysis method, a dissolved hydrogen concentration quantitative analysis method may be used for performing quantitative analysis of the dissolved hydrogen concentration in test water based on the color change speed of the oxidation/ reduction pigment through reduction reaction of that pigment with the assistance of the precious metal colloid catalyst when dripping only a predetermined amount of solution with a predetermined concentration of the oxidation/ reduction pigment into a predetermined amount of test water, which includes the precious metal colloid catalyst, instead of the quantitative analysis method based on the total dripped amount until the end point of the oxidation/ reduction pigment color change through reduction reaction of that pigment.

### (B) Testing objectives

When a solution with a predetermined concentration of oxidation/ reduction pigment methylene blue is dripped into the hydrogen- dissolved water that includes catalyst- added electrolyzed water, the fact that the total dripped amount of methylene blue added until this post-drip solution no longer causes the reducing color reaction to be displayed (hereafter, also referred to as 'end point') becomes a measure of the quantitative analysis of the dissolved hydrogen concentration (explicit antioxidation power) is verified through the following tests.

### (C) Outline of effective dissolved hydrogen concentration quantitative analysis method

In order to quantitatively analyze the effective amount of reducing power (antioxidation power) expressed through the chemical activation of inert molecular hydrogen in the hydrogen- dissolved water, or in other words, the effective dissolved hydrogen concentration DH (mg/L) when a catalyst is added to the hydrogen- dissolved water according to the present invention, methylene blue oxidation/ reduction titration was carried out on the catalyst-(Pt colloid) added hydrogen- dissolved water using Pt colloid as the catalyst and methylene blue as the oxidation/ reduction pigment.

### (D) Testing procedures

The basic testing procedures include preparing a number of sample waters (already having respective features such as dissolved hydrogen concentration measured), adding the catalyst (Pt colloid) to these samples, and delivering drops of the methylene blue. Comparative evaluation is then made of whether or not there exists correlation between the effective amount of dissolved hydrogen concentration found from each total amount of methylene blue added and the actual reading of the dissolved hydrogen meter.

If there is a correlation between the two, it can be considered that the legitimacy of the dissolved hydrogen concentration quantitative analysis through methylene blue redox titration, and the fact that the key material expressing the explicit antioxidant function is dissolved hydrogen can be objectively validated.

In keeping with such basic thinking, to begin with, a one-fortieth strength Pt standard solution is prepared by diluting the Pt standard solution described earlier to a concentration of one-fortieth strength. It should be noted that the platinum component concentration C(Pt) in this one-fortieth strength Pt standard solution becomes a 192 mg/L concentration using the formula C(Pt) = 24 g x 0.04 / 500 mL.

Next, a 1 g/L concentration (mole concentration by volume: 2677.4 µM) of methylene blue solution and a 10 g/L concentration (mole concentration by volume: 26773.8 µM) of methylene blue solution are prepared. Here, two types of different concentrations of methylene blue solution are prepared because changing the concentration of the methylene blue solution to be added in response to the hydrogen concentration which would be dissolved in the water to be tested is expected to result in allowing the added amount of the solution to be reduced and improve test accuracy. Nevertheless, the Pt concentration in the Pt standard solution and the MB concentration in the methylene blue solution are not limited to these, but may be adjusted as appropriate in response to conditions such as the amount of hydrogen which would be dissolved in the water to be tested.

Next, 50 mL of one-fortieth strength Pt standard solution prepared as described above and 50 mL of each of the two types of different concentrations of methylene blue solution are respectively collected in individual degasification bottles, these are subjected three times to a process that includes 10 minutes of degasification using a vacuum pump followed by 10 minutes of nitrogen gas inclusion, so as to prepare the methylene blue solution and one-fortieth strength Pt standard solution that has undergone the nitrogen gas replacement. This process aims to remove other gaseous components besides nitrogen (inert gas) in each of the solutions.

Next, 200 mL of test water is poured into an acrylic, gas-impermeable tester together with a magnet stirrer. This tester has been created for this testing and has a structure whereby the bottom is formed by attaching a round acrylic plate to one end along the length of a hollow, cylinder-shaped, acrylic tube, and the open end has a structure that has a pusher configured with a round plate having a diameter that is slightly smaller than the inner diameter of this tube so as to seal in a piston-like manner allowing movement along the length of the tube. A sealing ring made of a material such as silicon rubber is attached to the pusher so as to seal its entire surroundings. On the inside wall of this tester, a solution injection part configured with a hollow, cylinder-shaped, acrylic tube directed so as to radiate out towards the outside wall is provided in this tester to allow injection of MB solution or one-fortieth strength Pt standard solution separated from the outside environment into the test water holding compartment demarcated by the bottom surface, side wall, and pusher of this tester. In addition, a removable rubber stopper is provided for this solution injection part to allow syringe needle insertion. When pouring the test water into the test water holding compartment of the tester configured in this manner, the test water is softly pumped while the pusher is removed from the tester and then the pusher is attached to prevent vapor from forming inside the test water holding compartment. This allows the test water inside the test water holding compartment of the tester to be sealed in a condition separate from the outside environment. In addition, when the one-fortieth strength Pt standard solution or MB solution is poured into the test water holding compartment of the tester, such solution is collected through suction to prevent vapor from developing inside the syringe. The solution is softly injected by inserting the needle of the syringe into the rubber stopper equipped with a solution injection part and pushing the piston of the syringe. It should be noted that the tester disclosed here is merely an example. Other appropriate vessels may be used as long as they meet conditions including: 1) the tester is made of a gas-impermeable material, and does not occlude hydrogen (for example, stainless steel is not a suitable material for the tester since it occludes hydrogen, although it is gas-impermeable); 2) test water holding compartment can be isolated from outside environment; 3) volume of test water holding compartment is adjustable; 4) test water holding compartment is air-tight and water-tight; 5) one-fortieth strength Pt standard solution and MB solution may be poured in while the test water holding compartment is isolated from the outside environment; and 6) the stirrer is moveable.

Next, the tester containing the test water described above is placed bottom- down on a magnetic stirring table and stirring with the stirrer is begun.

Next, 1 mL of the one-fortieth strength Pt standard solution that has been subjected to the nitrogen gas replacement described above is injected to the test water holding compartment using a syringe and this is sufficiently stirred and mixed.

Next, a predetermined density of methylene blue solution that has undergone the above- mentioned nitrogen gas replacement is injected a little bit at a time using a syringe while visually observing the color change of the test water. Here, if the dissolved hydrogen concentration of the test water is greater than the amount of methylene blue poured in, then the methylene blue is reduced and becomes colorless. However, as the amount of methylene blue solution poured in gradually increases, the added methylene blue and the dissolved hydrogen of the test water counteract each other, and in time the change in the methylene blue from blue to colorless can no longer be observed. Making this point the end point, the concentration of dissolved hydrogen DH in the test water can be found from the methylene blue concentration of the methylene blue solution and the total amount of methylene blue solution added.

### (E) Finding the effective concentration of dissolved hydrogen

In the following, the meaning of the 'effective dissolved hydrogen concentration DH' is explained while showing the formula for finding the effective dissolved hydrogen concentration DH in the test water from the concentration and total added amount of the methylene blue solution added to the test water and the process of deriving the formula.

To begin with, in the following description, the volume of water to be tested is given as 200 mL and the methylene blue volume mole concentration of the methylene blue solution to be added to the test water is given as N(µmol/L). Moreover, given that the total amount of methylene blue solution added to reach the end point is A (mL), the total added amount of methylene molecules B(mol) becomes 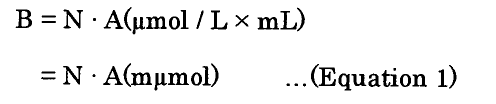 Here, given that the chemical formula of the methylene blue molecule is given as MBCl, and the chemical formula of the hydrogen molecule as H₂, the reaction in the solution between the hydrogen molecule activated by the Pt colloid and the methylene blue molecule may be expressed with the following reaction formula 1. ${\text{H}}_{\text{2}} \text{+ MBCl → HCl + MBH}$

Here, HCl is hydrochloric acid, and MBH is reduced methylene blue. According to reaction formula 1, 1 mole of hydrogen molecules and 1 mole of methylene blue molecules react and generate 1 mole of reduced methylene blue molecules. In order to explain the reception of electrons, the reaction formula may be written divided into two half equations as follows: ${\text{H}}_{\text{2}} {\text{→ H}}^{\text{+}} {\text{+ (H}}^{\text{+}} {\text{+ 2e}}^{\text{-}} \text{)}$${\text{MB}}^{\text{+}} {\text{+ (H}}^{\text{+}} {\text{+ 2e}}^{\text{-}} \text{) → MBH}$

Half reaction 1 means that the 1 mole of hydrogen molecules releases 2 moles of electrons, and half equation 2 means that the 1 mole of methylene blue cations, or 1 mole of methylene blue molecules accepts 2 moles of electrons. Here, 1 mole of hydrogen molecules is equivalent to 2 grams since 2 moles of electrons are released. Meanwhile, 1 mole of methylene blue cations, or 1 mole of methylene blue molecules is equivalent to 2 grams since 2 moles of electrons are accepted. As a result, since the gram equivalence of both the hydrogen molecule and the methylene blue cation or the methylene blue molecule is 2, the hydrogen molecule and the methylene blue molecule react at a rate of 1 to 1 in terms of the mole ratio.

In keeping with this, the total amount of methylene blue B added to the test water described above is also the amount of hydrogen molecules consumed.

Accordingly, given a total amount of hydrogen molecules to be measured as C(mµmol), the following may be obtained from Equation 1:

C = B = N · A(mµmol) (Equation 2)

Moreover, if the volume of test water is 200 mL and the value of the effective hydrogen molecule mole concentration by volume H₂ (mol/L) of the test water is the mole count C(mol) divided by volume (mL), then ${\text{H}}_{\text{2}} \text{(mol/L) = C / 200(mµmol/mL)} \text{= C / 200(µmol/L)}$

Moreover, in the case of exchanging this unit with mass concentration (g/L), given the corresponding mass concentration of hydrogen molecules as D, from the proportional expression relating to the hydrogen molecule H₂:

1 mol / 2 g = H₂ (µmol/L) / D (Equation 4)

if this Equation 4 is replaced with Equation 3, then $\text{D = 2 · C / 200 (µg/L)} \text{= C / 100 (µg/L)}$This is the mass concentration of effective hydrogen molecules included in 200 mL of test water. It should be noted that the above- mentioned effective hydrogen molecule mass concentration D is of the microgram order, however, both the numerator and the denominator may be multiplied by 1000 to give: $\text{D = C · 1000 / 100 · 1000 (µg/L)} {\text{= C · 10}}^{\text{-5}} \text{(mg/L)}$

Then from the relationship in Equation. 2, since the hydrogen molecule mole count C of Equation 6 may be replaced with the total amount of methylene blue B, it may be established that:

D = N · A (mµmol)·10⁻⁵ (mg/L) (Equation 7)

From this Equation 7, it may be understood that the effective hydrogen molecule mass concentration D (mg/L) included in the test water may be found by multiplying the methylene blue mole concentration by volume (µmol/L) by the total amount (mL) of methylene blue solution added to reach the end point.

However, the test water not only includes the hydrogen molecules (hydrogen gas) tested in the quantitative analysis here, but also includes various types of ions, oxygen molecules (oxygen gas), carbon dioxide (carbon dioxide gas), and the like. Of these, to give exemplary substance names involved in the oxidation/ reduction reaction occurring in the test water, oxygen molecules, hypochlorite, hypochlorous acid, etc. may be given besides the hydrogen molecules. Including the oxidation/ reduction reaction, such oxygen molecules, etc., normally act as the main oxidizing agent, and except for certain special cases, do not act as the reducing agent. In particular, in the test where methylene blue such as that described here is reduced, the oxygen molecules, etc. act as an oxidizing agent, and instead of reducing the methylene blue, act to oxidize the reduced methylene blue changing it to oxidized methylene blue. In other words, even if the methylene blue reduced by the activation of the molecular hydrogen either remains reduced methylene blue and clear, or remains a white deposit, in the case where it exists together with the oxygen molecule, etc, the reduced methylene blue ends up being oxidized again and returning to the original oxidized methylene blue. In addition, even if not through the methylene blue, since the activated hydrogen molecule and the oxygen molecule directly react and take an equivalent amount of the reducing power of the hydrogen molecule, this equivalent amount of methylene cannot be reduced. In other words, as shown in FIGS. 21 and 22, in the case where the oxygen molecules, etc. also exist in the hydrogen- dissolved water, an amount of hydrogen molecules equivalent to these amounts is consumed, and the total amount of methylene blue added until the equivalence point also becomes reduced in accordance with the amount of oxide.

In light of this, it may be said that the dissolved hydrogen concentration measured through quantitative analysis using methylene blue is the effective dissolved hydrogen concentration minus that consumed by oxidizing agents such as dissolved oxygen.

### (F) Disclosure of reference examples and working examples

### (Reference example 17)

Using alkali electrolyzed water that has been subjected to continuous electrolysis processing using electrolysis conditions of electrolysis range '4' at normal water level with a 'Mini Water' electrolyzed water generation apparatus (equipped with an active charcoal filter) manufactured by MiZ Co., Ltd. as the test water, 1 mL of one-fortieth strength Pt standard solution that has been subjected to the nitrogen gas replacement described above is injected into the test water holding compartment using a syringe. This is then sufficiently stirred and mixed, and thereafter while visually observing the color change of the test water, a 1 g/L concentration (mole concentration by volume: 2677.4 µM) of methylene blue solution is added a little at a time to this test water using a syringe. The total amount of methylene blue injected until reaching the end point was 1 mL, and the measured dissolved hydrogen concentration DH found by replacing the values in Equation 7 was 0.03 (mg/L). The total amount of methylene blue injected until reaching the equivalence point was 1 mL, and the measured dissolved hydrogen concentration DH found by replacing the values in Equation 7 was 0.03 (mg/L). For the test water according to this working example 17, the pH, oxidation/ reduction potential ORP (mV), electric conductance EC (mS/m), water temperature T (°C), dissolved oxygen concentration DO (mg/L), measured dissolved hydrogen concentration DH (mg/L), and the measured dissolved hydrogen concentration DH (mg/L) found by replacing the values in Equation 7 are shown in Table 3, and the measured value and the effective value of DH are shown in FIG. 23. It should be noted that the types of instruments used to measure each physical property are the same as those described above.

### (Reference example 18)

Using test water that consists of purified water processed by passing Fujisawa city water through an ion exchange column manufactured by Organo Corporation, boiled, and then subjected to hydrogen gas bubbling processing while allowing the temperature to cool to 20°C, 1 mL of one-fortieth strength Pt standard solution that has undergone the nitrogen gas replacement described above is injected into 200 mL of this test water in a test water holding compartment using a syringe. This is then sufficiently stirred and mixed, and thereafter while visually observing the color change of the test water, a 10 g/L concentration (mole concentration by volume: 26773.8 µM) of methylene blue solution is injected a little bit at a time into the test water using a syringe. The total amount of methylene blue solution injected until reaching the end point was 6.2 mL, and the measured dissolved hydrogen concentration DH found by replacing the values in Equation 7 was 1.66 (mg/L). Each physical property value of the test water according to this reference example 18 is shown in Table 3, and the actual measured value and effective value of the dissolved hydrogen concentration DH are shown in FIG. 23.

### (Working example 20)

Using electrolyzed water as test water, which is base water 6.86 of the above- mentioned sample i that has been subjected to electrolysis processing using a continuous flow method under conditions of a 1 L/min flow and 5A constant current, 1 mL of one-fortieth strength Pt standard solution that has undergone the nitrogen gas replacement described above is injected to 200 mL of this test water in a test water holding compartment using a syringe. This is then sufficiently stirred and mixed, and thereafter while visually observing the color change of the test water, a 10 g/L concentration (mole concentration by volume: 26773.8 µM) of methylene blue solution is injected a little bit at a time into the test water using a syringe. The total amount of methylene blue solution injected until reaching the end point was 5.9 mL, and the measured dissolved hydrogen concentration DH found by replacing the values in Equation 7 was 1.58 (mg/L). Each physical property value of the test water according to this working example 20 is shown in Table 3, and the actual measured value and effective value of the dissolved hydrogen concentration DH are shown in FIG. 23.

### (Working example 21)

Using electrolyzed water as test water, which is base water 9.18 of the above- mentioned sample v that has been subjected to electrolysis processing using a continuous flow method under conditions of a 1 L/min flow and 5A constant current, 1 mL of one-fortieth strength Pt standard solution that has undergone the nitrogen gas replacement described above is injected to 200 mL of this test water in a test water holding compartment using a syringe. This is then sufficiently stirred and mixed, and thereafter while visually observing the color change of the test water, a 10 g/L concentration (mole concentration by volume: 26773.8 µM) of methylene blue solution is injected a little bit at a time into the test water using a syringe. The total amount of methylene blue solution injected until reaching the end point was 5.0 mL, and the measured dissolved hydrogen concentration DH found by replacing the values in Equation 7 was 1.34 (mg/L). Each physical property value of the test water according to this working example 21 is shown in Table 3, and the actual measured value and effective value of the dissolved hydrogen concentration DH are shown in FIG. 23.

### (Working example 22)

Using electrolyzed water as test water, which is a pH buffer solution of standard buffer solution 4.01 (phthalate solution) manufactured by Wako Pure Chemical diluted to one-tenth strength with purified water that has been subjected to electrolysis processing using a continuous flow method under conditions of a 1 L/min flow and 5A constant current, 1 mL of one-fortieth strength Pt standard solution that has undergone the nitrogen gas replacement described above is injected into 200 mL of this test water in a test water holding compartment using a syringe. This is then sufficiently stirred and mixed, and thereafter while visually observing the color change of the test water, a 10 g/L concentration (mole concentration by volume: 26773.8 µM) of methylene blue solution is injected a little bit at a time into the test water using a syringe. The total amount of methylene blue solution injected until reaching the end point was 6.3 mL, and the measured dissolved hydrogen concentration DH found by replacing the values in Equation 7 was 1.69 (mg/L). Each physical property value of the test water according to this working example 22 is shown in Table 3, and the actual measured value and effective value of the dissolved hydrogen concentration DH are shown in FIG. 23.

### (Working example 23)

Using circulating electrolyzed water as test water, which is base water 6.86 of the above- mentioned sample i that has been subjected to electrolysis processing using a continuous flow circulating method (volume of circulatory water: 0.8 liters) for 3 minutes under conditions of a 1 L/min flow and 5A constant current, 1 mL of one-fortieth strength Pt standard solution that has undergone the nitrogen gas replacement described above is injected to 200 mL of this test water in a test water holding compartment using a syringe. This is then sufficiently stirred and mixed, and thereafter while visually observing the color change of the test water, a 10 g/L concentration (mole concentration by volume: 26773.8 µM) of methylene blue solution is injected a little bit at a time into the test water using a syringe. The total amount of methylene blue solution injected until reaching the end point was 9.6 mL, and the measured dissolved hydrogen concentration DH found by replacing the values in Equation 7 was 2.57 (mg/L). Each physical property value of the test water according to this working example 23 is shown in Table 3, and the actual measured value and effective value of the dissolved hydrogen concentration DH are shown in FIG. 23.

### (Working example 24)

Using circulating electrolyzed water as test water, which is base water 9.18 of the above- mentioned sample v that has been subjected to electrolysis processing using a continuous flow circulating method (volume of circulatory water: 0.8 liters) for 3 minutes under conditions of a 1 L/min flow and 5 Å constant current, 1 mL of one-fortieth strength Pt standard solution that has undergone the nitrogen gas replacement described above is injected to 200 mL of this test water in a test water holding compartment using a syringe. This is then sufficiently stirred and mixed, and thereafter while visually observing the color change of the test water, a 10 g/L concentration (mole concentration by volume: 26773.8 µM) of methylene blue solution is injected a little bit at a time into the test water using a syringe. The total amount of methylene blue solution injected until reaching the end point was 12.3 mL, and the measured dissolved hydrogen concentration DH found by replacing the values in Equation 7 was 3.29 (mg/L). Each physical property value of the test water according to this working example 24 is shown in Table 3, and the actual measured value and effective value of the dissolved hydrogen concentration DH are shown in FIG. 23.

### (Working example 25)

Using circulating electrolyzed water as test water, which is the same pH buffer solution as working example 22 that has been subjected to electrolysis processing using a continuous flow circulating method (volume of circulatory water: 0.8 liters) for 3 minutes under conditions of a 1 L/min flow and 5A constant current, 1 mL of one-fortieth strength Pt standard solution that has undergone the nitrogen gas replacement described above is injected to 200 mL of this test water in a test water holding compartment using a syringe. This is then sufficiently stirred and mixed, and thereafter while visually observing the color change of the test water, a 10 g/L concentration (mole concentration by volume: 26773.8 µM) of methylene blue solution is injected a little bit at a time into the test water using a syringe. The total amount of methylene blue solution injected until reaching the end point was 12.4 mL, and the measured dissolved hydrogen concentration DH found by replacing the values in Equation 7 was 3.32 (mg/L). Each physical property value of the test water according to this working example 25 is shown in Table 3, and the actual measured value and effective value of the dissolved hydrogen concentration DH are shown in FIG. 23.

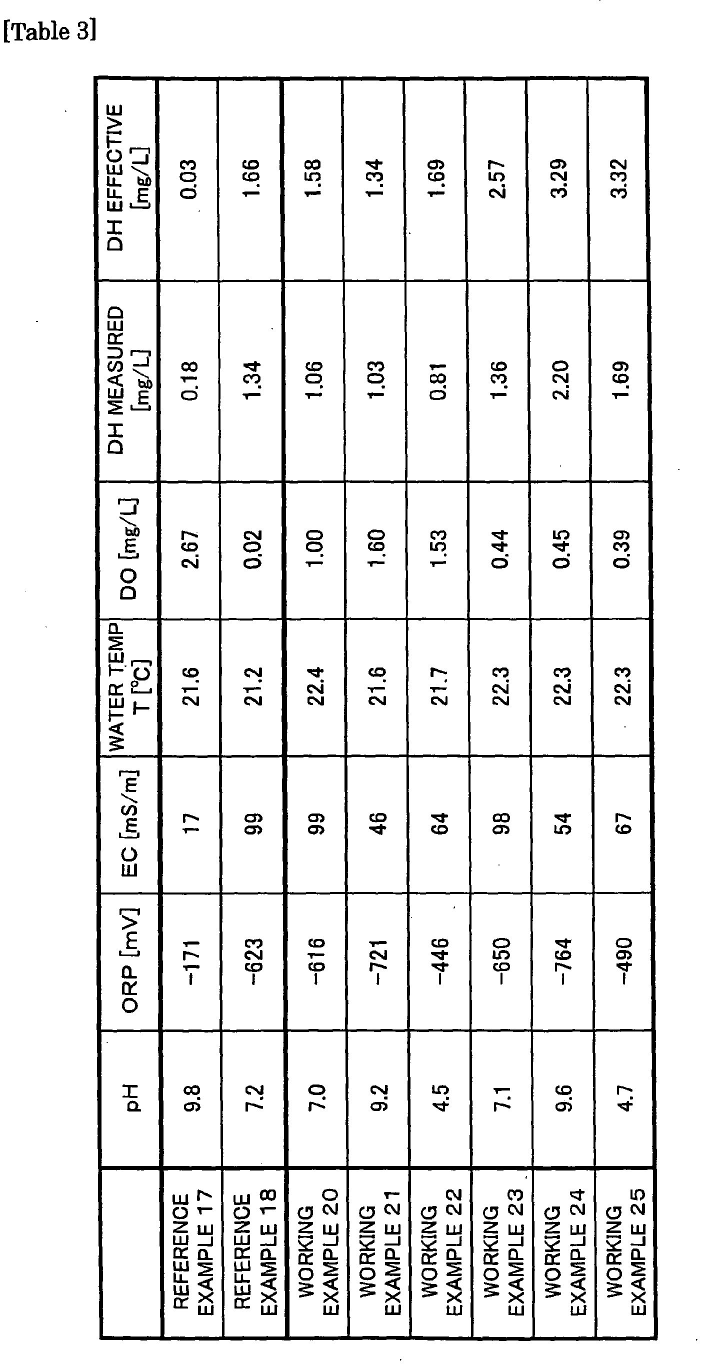

### (G) Examination of working examples

According to Table 3 and FIG. 23, it may be understood that there is commensurate correlation between the actual measured value and the effective value of the dissolved hydrogen concentration DH since when the actual measured value is high, the effective value grows higher in response thereto. In addition, compared to the dissolved hydrogen concentration DH effective value of reference example 17, the respective effective values of DH in reference example 18 and working examples 20 through 25 all showed high concentrations exceeding 1.3 (mg/L). In particular, while the molecular hydrogen saturated solvent concentration under normal temperature (20°C) and atmospheric pressure is approximately 1.6 (mg/L), which approaches that of water, the DH effective values of working examples 20 through 25 showed between 2.5 and 3.3 (mg/L), which are exceedingly high concentrations.

Therefore oxygen molecules may be thought of as being the main oxidizing agent remaining in the test water since in the quantitative analysis testing of dissolved- hydrogen concentrations performed herein, water that was pre-treated with activated charcoal was used (without adding a reducing agent) in all cases to scavenge the chlorine- based oxidizers such as hypochlorous acid. It should be noted that even if the oxygen molecules are temporarily scavenged with the activated charcoal, as long as there is no sort of reducing agent used, it is difficult to scavenge with only activated charcoal because oxygen quickly blends back into the water as soon as the test water hits the outside air.

Nevertheless, with the premise that the proposed antioxidation method according the present invention is used, the fact that the concentration of oxidizing material such as dissolved oxygen may be kept as low as possible while also making the dissolved hydrogen concentration as high as possible with a reducing potential water generation apparatus such as that developed by the applicants herein is important when anticipating expression of reduction activity and antioxidation activity that may be derived from the antioxidant- functioning water according to the combination of catalysts and hydrogen- dissolved water according to the present invention.
Therefore, in attempt to define the dissolved hydrogen water according to the present invention from the standpoint of the effective value of dissolved hydrogen concentration DH found using dissolved hydrogen concentration quantitative analysis that uses oxidation/ reduction pigment according to the present invention, it is preferable that the DH effective value be greater than the saturation concentration under atmospheric pressure (for example, 1.6 or greater under an atmospheric pressure of 1, 3.2 or greater under an atmospheric pressure of 2, 4.8 or greater under an atmospheric pressure of 3, and the like). Furthermore, when the atmospheric pressure is 1, as the dissolved hydrogen concentration DH effective value becomes higher preference increases, such as in the following order: 1.7 or greater, 1.8 or greater, 1.9 or greater, 2.0 or greater, 2.1 or greater, 2.2 or greater, 2.3 or greater, 2.4 or greater, 2.5 or greater, 2.6 or greater, 2.7 or greater, 2.8 or greater, 2.9 or greater, 3.0 or greater, 3.1 or greater, 3.2 or greater, and 3.3 or greater (all units are mg/L). This is because reduction activity and antioxidation activity derived from the antioxidant- functioning water according to the combination of catalysts and hydrogen- dissolved water according to the present invention may be anticipated with higher levels.

This information proposes a new quantitative analysis method of hydrogen concentration for hydrogen- dissolved water including electrolyzed water as well as a new measure of the explicit antioxidation power held by this water. In addition, with dissolved hydrogen concentration measurement using an existing dissolved hydrogen meter, handling and measurement procedures are complicated, in terms of measurement precision such measurement is also incapable of providing sufficient satisfaction, and furthermore, related costs are extremely high. However, with the dissolved hydrogen concentration quantitative analysis method according to the present invention that uses oxidation/ reduction pigment, handling and measurement procedures is relatively simple, and if the oxidation material included in the test water is scavenged, high precision is realized in terms of accuracy because it is based on the principle of performing direct, quantitative analysis through the chemical reaction of the number of molecules of molecular hydrogen with the oxidation/ reduction pigment, and moreover, the related costs are extremely low.

### Radical scavenging activity evaluation test using epinephrine oxidation method in XOD experiment system

Next, a radical scavenging activity evaluation test using an epinephrine oxidation method in an XOD experiment system is described in the following (A). In addition, radical scavenging activity expressed in antioxidant-functioning water (hereafter, may be referred to as 'AOW') according to the present invention when using this testing method is shown in the following (B) through both working examples and reference examples, respectively, where precious metal colloid catalysts (Pt colloid/ Pd colloid/ Pt and Pd mixed colloid) added into hydrogen-dissolved water as AOW are given as examples. It should be noted that the XOD experiment system is an experiment system that generates a superoxide anion radical (hereafter, may be simply referred to as (·O₂⁻)) by reducing one electron of oxygen using the electrons released when xanthine is oxidized through a process where xanthine oxidase (XOD), which is an organism enzyme, acts on xanthine in the oxygen-dissolved solution system.

### (A) Radical scavenging activity evaluation test using epinephrine oxidation method in XOD experiment system

### (A-1) Development of idea

The cytochrome c reduction method is known as a conventional free radical measurement method, as described in 'Free radical and medicine' (Issued by Hirokawa Shoten ISBN 4-567-49380-X, p133-141).

With this cytochrome c reduction method (see FIG. 24), in the XOD experiment system, the reduction reaction of the oxidized cytochrome c (ferricytochrome c (Fe³⁺) into oxidized cytochrome c (ferrocytochrome c (Fe²⁺) by the assistance of the superoxide anion radical (·O₂⁻) being retarded by a test sample such as SOD or antioxidizing agent is observed through a change over time in the local maximum absorption (wavelength: 550 nm) of the oxidized cytochrome c using a spectrophotometer. This method utilizes reactivity of (·O₂⁻) as a single-electron reducing agent.

To further explain this measurement principle in detail, in the cytochrome c reduction method, when a test sample such as an antioxidizing agent reduces and scavenges (·O₂⁻) prior to (or simultaneous to) the reduction reaction of the oxidized cytochrome c due to (·O₂⁻), the reduced amount of oxidized cytochrome c, namely, the generated amount of reduced cytochrome c is controlled (either upward tendency is controlled, or downward tendency begins to show). Then, the local maximum absorption (A550) of the reduced cytochrome c also decreases. To consider this with time, the local maximum absorption (A550) of the reduced cytochrome c per unit time decreases along with the scavenged amount of (·O₂⁻). By observing transition of this local maximum absorption (A550) over time, SOD-like activity of the test sample can be measured.

However, when a test sample having remarkable reducing power such as ascorbic acid or the antioxidant-functioning water according to the present invention is used, there are problems with the SOD-like activity measurement system using the above-mentioned conventional cytochrome c reduction method in that it cannot obtain highly precise measurement results, as well as it leads to opposite measurement results to the expected true results. This is because (·O₂⁻) is used as a reducing agent. In other words, AOW and ascorbic acid of the present invention reduces and scavenges (·O₂⁻), and even reduces the oxide cytochrome c. As a result, it may be considered that, for example, (·O₂⁻) is completely reduced and scavenged, and the oxidized cytochrome c is also reduced with its residual reducing power. Therefore, there has been a risk of not obtaining highly precise measurement results, as well as of leading to opposite measurement results, such as low SOD-like activity appearance, to the expected true results.

As a result of zealous research on the free radical reaction reagent suitable for radical scavenging activity evaluation testing of antioxidant-functioning water according to the present invention, the inventers ended up wondering if oxidation of reduced epinephrine can be used.

When the reduced epinephrine is oxidized by the superoxide anion radical (·O₂⁻), it changes to red adrenochrome (oxidized epinephrine), and its local maximum absorption (A480) increases. In this case, (·O₂⁻) acts as an oxidizing agent. It should be noted that the reduced epinephrine is difficult to be oxidized by a normal oxygen molecular, and even if it is oxidized, it does not indicate a color change reaction to red. That is, it has been confirmed through experiment that the local maximum absorption (A480) of the reduced epinephrine does not increase even in oxygen-dissolved solution system. This means that the reduced epinephrine is suitable as a free radical reaction reagent to distinguish a normal oxygen molecular (O₂) from a superoxide anion radical (·O₂⁻).

In the epinephrine oxidation method (see FIG. 25), to begin with, it is assumed that a sufficient amount of (·O₂⁻) has been generated in the XOD experiment system. (·O₂⁻) generated in this manner changes the reduced epinephrine to oxidized epinephrine in conformity with the following chain reaction. ${\text{RH}}_{\text{3}} {\text{}}^{\text{-}} {\text{+ ·O}}_{\text{2}} {\text{}}^{\text{-}} {\text{+ 2H}}^{\text{+}} {\text{→ ·RH}}_{\text{3}} {\text{+ H}}_{\text{2}} {\text{O}}_{\text{2}}$${\text{·RH}}_{\text{3}} {\text{+ O}}_{\text{2}} {\text{→ RH}}_{\text{2}} {\text{+ ·O}}_{\text{2}} {\text{}}^{\text{-}} {\text{+ H}}^{\text{+}}$${\text{RH}}_{\text{2}} {\text{+ ·O}}_{\text{2}} {\text{}}^{\text{-}} {\text{+ H}}^{\text{+}} {\text{→ ·RH + H}}_{\text{2}} {\text{O}}_{\text{2}}$${\text{·RH + O}}_{\text{2}} {\text{→ R + ·O}}_{\text{2}} {\text{}}^{\text{-}} {\text{+ H}}^{\text{+}}$

Where (RH₃⁻) is reduced epinephrine, and (R) is oxidized epinephrine (adrenochrome.) While the reactivity between the reduced epinephrine and (·O₂⁻) is low in the vicinity of the physiological region in pH, the speed of a secondary reaction between the reduced epinephrine, and (·O₂⁻) increases if a large dosage of reduced epinephrine is administered. Therefore, with this epinephrine oxidation method, it is preferable that the mole concentration of reduced epinephrine be set to a high concentration of approximately 1 mM, for example. Furthermore, the reduced epinephrine tends to be easily oxidized by trace amounts of a transition metal such as an iron ion. In order to avoid influence from the above-mentioned disturbances, it is necessary to include a chelating agent such as EDTA in the test water.

At this time, prior to (or simultaneous to) the oxidation reaction of reduced epinephrine due to (·O₂⁻), the oxidized amount of reduced epinephrine, that is, the generated amount of oxidized epinephrine is being gradually reduced as a test sample such as an antioxidizing agent reduces and scavenges (·O₂⁻). Then, the local maximum absorption (A480) of the oxidized epinephrine tends to rise gradually. To consider this with time, the local maximum absorption (A480) of the reduced epinephrine per unit time tends to rise gradually along with the scavenged amount of (·O₂⁻). Therefore, by observing transition of this local maximum absorption (A480) over time, SOD-like activity (radical scavenging activity) of the test sample can be measured.

More specifically, to consider a graph where the horizontal axis indicates elapsed time and the vertical axis indicates light absorbance (A480), the SOD-like activity can be represented by the amount of change (Δ A480) in the light absorbance (A480) per unit time (ΔT). In other words, the tangent slope (ΔA/ ΔT) in the graph represents the SOD-like activity. Therefore, regarding the SOD-like activity graph of a test sample, it can be determined that when a positive slope (continuously growing characteristic) is large, the SOD-like activity is low; whereas, when the positive slope is small, the SOD-like activity is high. On the other hand, it can be determined that when a negative slope (continuously dropping characteristic) is large, the SOD-like activity is high; whereas, the negative slope is small, the SOD-like activity is low. However, comparing the case of a small positive slope to the case of a small negative slope from the standpoint of high or low radical scavenging activity shows that the latter has higher radical scavenging activity than the former.

This is a measurement principle of the radical scavenging activity (SOD-like activity) using the epinephrine oxidation method.

### (A-2) Reagents to be used

Reagents to be used for radical scavenging activity (SOD-like activity) testing using the epinephrine oxidation method are given below.
(1) Dulbecco's phosphoric acid buffer physiological salt (PBS) manufactured by Wako Pure Chemical Industries, Ltd.
(2) Xanthine (2,6-dioxopurine) manufactured by Wako Pure Chemical Industries, Ltd.
(3) Xanthineoxidase suspension (derived from buttermilk) manufactured by Wako Pure Chemical Industries, Ltd.
(4) EDTA-2Na 2 hydrate manufactured by Dojindo Laboratories, and sold by Wako Pure Chemical Industries, Ltd.
(5) 1 mol/ L sodium hydroxide solution manufactured by Wako Pure Chemical Industries, Ltd.
(6) L(+)-ascorbic acid manufactured by Wako Pure Chemical Industries, Ltd.
(7) (±)epinephrine (dl-epinephrine) (dl-adrenaline) manufactured by Wako Pure Chemical Industries, Ltd.

### (A-3) Reagent preparation method

Preparation methods for the respective reagents given in the above section (A-2) are described below.

### (1) Preparation of PBS buffer stock solution containing EDTA

Two packages of 500 mL of Dulbecco's phosphoric acid buffer physiological salt (hereafter, referred to as 'PBS') are dissolved into 100 mL of distilled water, and is then divided into two equal parts. 0.19 g of EDTA-2Na is dissolved into one of the 50 mL of solution. This is considered as an EDTA stock solution. 0.5 mL of this EDTA stock solution and 49.5 mL of a solution containing no EDTA are collected, respectively, and then are mixed together.

The mixed solution obtained here is a PBS buffer stock solution containing EDTA (1.9 mg). This is used diluted to one-tenth strength PBS concentration. If many metal ion species are contained in test water, the EDTA stock solution may be diluted to one-tenth strength. In this case, since the EDTA concentration is high, almost all of metal ions, which are factors that degrade measurement accuracy, can be removed. It should be noted that the PBS buffer stock solution containing EDTA is prepared in order to fix the pH of the solution to the vicinity of approximately 7.4, which falls within the physiological region of solution characteristics, and prevent measurement accuracy from being degraded due to metal ions.

### (2) Preparation of 1.5 mM of xanthine solution

0.228 ~ 0.23 g of xanthine and 80 drops of 1 (mol/ L) sodium hydroxide are added to 350 mL of distilled water, and the xanthine is dissolved. 35 mL of that solution is collected, and the PBS buffer stock solution containing EDTA is added to make 100 mL of solution.

### (3) Preparation of xanthineoxidase solution

Xanthineoxidase suspension is diluted with the PBS buffer stock solution containing EDTA to one-one hundredth strength. This must be prepared for each experiment.

### (4) Preparation of epinephrine solution

100 mL of distilled water is poured into a vial together with a stirrer, a rubber stopper is inserted, and an injection needle for exhaustion and an injection needle connected to a nitrogen gas cylinder are inserted into the rubber stopper. Under this condition, nitrogen gas is included into the distilled water while vigorously stirring the vial containing the distilled water, which is placed on the stirring table, and replacement in the distilled water is made with nitrogen gas. This nitrogen gas replacement treatment is carried out for 30 minutes. 0.15 g of epinephrine is then poured into the vial, the stopper is inserted, and nitrogen gas is included while softly stirring with the stirrer. This nitrogen gas replacement treatment continues until the experiment ends. This is the preparation of the epinephrine solution. Tips on the above-mentioned preparation are to stir vigorously before epinephrine is added, and to stir softly after epinephrine is added.

### (A-4) Testing procedures

In the conventional XOD experiment system, all reagents and a test solution are added into a cell sequentially, and xanthineoxidase (XOD) is added last. While the reaction has begun with the addition of this XOD, measurement using a spectrophotometer has also begun.

However, with the conventional method, when the test solution is added, (·O₂⁻) has not been sufficiently generated yet. Therefore, at this time, oxygen molecules, which are materials for generating (·O₂⁻), exist without change. In other words, since the generated amount of (·O₂⁻) increases as the oxygen molecules change to (·O₂⁻) with time after XOD is added, an appropriate amount of (·O₂⁻) cannot be obtained immediately after the test water is added. Furthermore, since the amount of dissolved oxygen, which is a material for generating (·O₂⁻), is not understood clearly with the conventional method, it is not clear whether or not the same amount of (·O₂⁻) is generated every time.

Through consideration of the above, the inventors have come to believe that it is desirable to begin by adding the PBS buffer stock solution containing EDTA, the xanthineoxidase solution, and the distilled water for supplying oxygen to a cell and mixing together. After a specified period has passed, that is, after an appropriate amount of (·O₂⁻) has been generated, the test water and the epinephrine solution are added and measurement is begun.

Therefore, in this epinephrine oxidation method, each reagent solution or the test water is poured into a cell sequentially according to the following procedures. In addition, a waiting period is included according to need. It should be noted that the cell has 3 mL of capacity of which each of the test water and the distilled water for supplying oxygen occupy approximately 1/3, respectively, and the remaining approximately 1/3 is occupied by a xanthineoxidase solution, an epinephrine solution, and a xanthine solution such as a pH buffer solution containing EDTA.
(1) Add 300 µL of PBS buffer stock solution containing EDTA.
(2) Add 300 µL of xanthine solution.
(3) Add 900 µL (approximately 1/3 of the cell capacity) of distilled water for supplying oxygen.
(4) Add 100 µL of xanthineoxidase solution.
(5) Wait 5 minutes to generate an appropriate amount of (·O₂⁻).
(6) Add 1 mL (approximately 1/3 of the cell capacity) of test water (fluid).
(7) Add 400 µL of epinephrine solution.
(8) Immediately start measurement of change in light absorbance (A480) over time using a spectrophotometer.
(9) Wait 140 seconds to level out the differences in the local concentration gradient of reagents within the cell.

In other words, measurement data taken within 140 seconds from the start of measurement is excluded as a rule from a radical scavenging activity characteristic graph to be described later. The period for observing the change over time is set to 15 minutes except in the 140 second waiting period described in step (9). This is because a clear difference in the radical scavenging activity between platinum and palladium may not be recognized with an observation period of around 5 to 10 minutes.

It should be noted that it has been found through the experiment that an indication of radical scavenging activity can be found through the conventional method where all reagents are collectively added and xanthineoxidase is added last, then beginning the reaction and measurement. However, it is preferable that test water be added after an appropriate amount of (·O₂⁻) has been generated in the XOD experiment system, and a clearer indication of the actual radical scavenging activity may be found. Furthermore, with the conventional method, since it tends to take a relatively long time to generate an appropriate amount of (·O₂⁻) depending on the production lot of the xanthineoxidase, it is difficult to grasp the radical scavenging activity of the test water or the like in a short period of time. In particular, the conventional method has poor accuracy for the usage of finding the difference in catalytic activity between platinum and palladium. The timing of adding each reagent and of waiting should be specified as described above including such above-mentioned practical factors.

### (B) Disclosure of working examples and reference examples according to radical scavenging activity evaluation testing by epinephrine oxidation method in XOD experiment system

### (Reference example 19)

Radical scavenging activity measurement data obtained in conformity with testing procedures described in (A-4) when distilled water (manufactured by Wako Pure Chemical Industries, Ltd.) is employed as test water is given as reference example 19. It should be noted that under the testing condition where xanthineoxidase with different production lots are used, the radical scavenging activity measurement data of this reference example 19 may have slightly different radical scavenging activity characteristics depending on the production lots.

### (Reference example 20)

Radical scavenging activity measurement data obtained in conformity with the same testing procedures as those of reference example 19 when hydrogen gas-replaced distilled water (manufactured by Wako Pure Chemical Industries, Ltd.) is employed as test water is given as reference example 20. It should be noted that, as with reference example 19, under the testing condition where xanthineoxidase with different production lots are used, the radical scavenging activity measurement data of this reference example 20 may have slightly different radical scavenging activity characteristics depending on the production lots.

### (Working example 26)

Radical scavenging activity measurement data obtained in conformity with the same testing procedures as those of reference example 19 when antioxidant-functioning water (AOW) is used as test water, which is obtained by adding an amount of Pt standard solution described in working examples 3 through 5 sufficient to give a Pt colloid concentration of 48 µg/ L to distilled water (manufactured by Wako Pure Chemical Industries, Ltd., hereafter, the same), and then subjecting it to hydrogen gas replacement, is given as working example 26.

### (Working example 27)

Radical scavenging activity measurement data obtained in conformity with the same testing procedures as those of working example 26 when AOW is used as test water, which is obtained by adding an amount of the same Pt standard solution as that of working example 26 sufficient to give a Pt colloid concentration of 96 µg/ L to distilled water, and then subjecting it to hydrogen-gas replacement, is given as working example 27.

### (Working example 28)

Radical scavenging activity measurement data obtained in conformity with the same testing procedures as those of working example 26 when AOW is used as test water, which is obtained by adding an amount of the same Pt standard solution as that of working example 26 sufficient to give a Pt colloid concentration of 192 µg/ L to distilled water, and then subjecting it to hydrogen-gas replacement, is given as working example 28.

### (Working example 29)

Radical scavenging activity measurement data obtained in conformity with the same testing procedures as those of working example 26 when AOW is used as test water, which is obtained by adding an amount of the same Pt standard solution as that of working example 26 sufficient to give a Pt colloid concentration of 384 µg/ L to distilled water, and then subjecting it to hydrogen-gas replacement, is given as working example 29.

### (Working example 30)

Radical scavenging activity measurement data obtained in conformity with the same testing procedures as those of working example 26 when AOW is used as test water, which is obtained by adding an amount of the same Pt standard solution as that of working example 26 sufficient to give a Pt colloid concentration of 768 µg/ L to distilled water, and then subjecting it to hydrogen-gas replacement, is given as working example 30.

### (Working example 31)

Radical scavenging activity measurement data obtained in conformity with the same testing procedures as those of reference example 19 when antioxidant-functioning water (AOW) is used as test water, which is obtained by adding an amount of Pd standard solution described in working examples 6 through 8 sufficient to give a Pd colloid concentration of 48 µg/ L to distilled water, and then subjecting it to hydrogen-gas replacement, is given as working example 31.

### (Working example 32)

Radical scavenging activity measurement data obtained in conformity with the same testing procedures as those of working example 31 when AOW is used as test water, which is obtained by adding an amount of the same Pd standard solution as that of working example 31 sufficient to give a Pd colloid concentration of 96 µg/ L to distilled water, and then subjecting it to hydrogen-gas replacement, is given as working example 32.

### (Working example 33)

Radical scavenging activity measurement data obtained in conformity with the same testing procedures as that of working example 31 when AOW is used as test water, which is obtained by adding an amount of the same Pd standard solution as that of working example 31 sufficient to give a Pd colloid concentration of 192 µg/ L to distilled water, and then subjecting it to hydrogen-gas replacement, is given as working example 33.

### (Working example 34)

Radical scavenging activity measurement data obtained in conformity with the same testing procedures as that of working example 31 when AOW is used as test water, which is obtained by adding an amount of the same Pd standard solution as that of working example 31 sufficient to give a Pd colloid concentration of 384 µg/ L to distilled water, and then subjecting it to hydrogen-gas replacement, is given as working example 34.

### (Working example 35)

Radical scavenging activity measurement data obtained in conformity with the same testing procedures as that of working example 31 when AOW is used as test water, which is obtained by adding an amount of the same Pd standard solution as that of working example 31 sufficient to give a Pd colloid concentration of 768 µg/ L to distilled water, and then subjecting it to hydrogen-gas replacement, is given as working example 35.

### (Working example 36)

Radical scavenging activity measurement data obtained in conformity with the same testing procedures as those of reference example 19 when AOW is used as test water, which is obtained by adding an amount of platinum colloid solution that has approximately a particle size distribution of 1 to 2 nm and has been manufactured by the inventors based on the above-described thesis 'Fabrication and Use of Pt Colloids (Pt koroido no tsukurikata to tsukaikata)' written by Mr. NANBA, et al. sufficient to give a Pt colloid concentration of 66 µg/ L to distilled water, and then subjecting it to hydrogen gas replacement, is given as working example 36.

### (Working example 37)

Radical scavenging activity measurement data obtained in conformity with the same testing procedures as those of working example 36 when AOW is used as test water, which is obtained by adding an amount of the same platinum colloid solution as that of working example 36 sufficient to give a Pt colloid concentration of 96 µg/ L to distilled water, and then subjecting it to hydrogen-gas replacement, is given as working example 37.

### (Working example 38)

Radical scavenging activity measurement data obtained in conformity with the same testing procedures as those of working example 36 when AOW is used as test water, which is obtained by adding an amount of the same platinum colloid solution as that of working example 36 sufficient to give a Pt colloid concentration of 144 µg/ L to distilled water, and then subjecting it to hydrogen-gas replacement, is given as working example 38.

### (Working example 39)

Radical scavenging activity measurement data obtained in conformity with the same testing procedures as those of working example 36 when AOW is used as test water, which is obtained by adding an amount of the same platinum colloid solution as that of working example 36 sufficient to give a Pt colloid concentration of 192 µg/ L to distilled water, and then subjecting it to hydrogen-gas replacement, is given as working example 39.

### (Working example 40)

Radical scavenging activity measurement data obtained in conformity with the same testing procedures as those of working example 36 when AOW is used as test water, which is obtained by adding an amount of the same platinum colloid solution as that of working example 36 sufficient to give a Pt colloid concentration of 384 µg/ L to distilled water, and then subjecting it to hydrogen-gas replacement, is given as working example 40.

### (Working example 41)

Radical scavenging activity measurement data obtained in conformity with the same testing procedures as those of working example 36 when AOW is used as test water, which is obtained by adding an amount of the same platinum colloid solution as that of working example 36 sufficient to give a Pt colloid concentration of 768 µg/ L to distilled water, and then subjecting it to hydrogen-gas replacement, is given as working example 41.

### (Working example 42)

Radical scavenging activity measurement data obtained in conformity with the same testing procedures as those of reference example 19 when AOW is used as test water, which is obtained by adding a mixed (Pt + Pd) colloid solution of 96 µg/ L with a mole ratio of 1 to 2 for the same Pt standard solution as that of working example 26 and the same Pd standard solution as that of working example 31, mixed and prepared to distilled water, and then subjecting it to hydrogen gas replacement, is given as working example 42.

### (Working example 43)

Radical scavenging activity measurement data obtained in conformity with the same testing procedures as those of working example 42 when AOW is used as test water, which is obtained by adding an amount of the same mixed (Pt + Pd) colloid solution as that of working example 42 sufficient to give a concentration of 192 µg/ L to distilled water, and then subjecting it to hydrogen-gas replacement, is given as working example 43.

### (Working example 44)

Radical scavenging activity measurement data obtained in conformity with the same testing procedures as those of working example 42 when AOW is used as test water, which is obtained by adding an amount of the same mixed (Pt + Pd) colloid solution as that of working example 42 sufficient to give a concentration of 384 µg/ L to distilled water, and then subjecting it to hydrogen-gas replacement, is given as working example 44.

### (Working example 45)

Radical scavenging activity measurement data obtained in conformity with the same testing procedures as those of working example 42 when AOW is used as test water, which is obtained by adding an amount of the same mixed (Pt + Pd) colloid solution as that of working example 42 sufficient to give a concentration of 768 µg/ L to distilled water, and then subjecting it to hydrogen-gas replacement, is given as working example 45.

### (Working example 46)

Radical scavenging activity measurement data obtained in conformity with the same testing procedures as those of reference example 19 when AOW is used as test water, which is obtained by adding a mixed (Pt + Pd) colloid solution of 144 µg/ L with a mole ratio of 1 to 5 for the same Pt standard solution as that of working example 26 and the same Pd standard solution as that of working example 31, mixed and prepared to distilled water, and then subjecting it to hydrogen-gas replacement, is given as working example 46.

### (Working example 47)

Radical scavenging activity measurement data obtained in conformity with the same testing procedures as those of working example 46 when AOW is used as test water, which is obtained by adding a mixed (Pt + Pd) colloid solution of 240 µg/ L with a mole ratio of 1 to 10 for the same Pt standard solution as that of working example 26 and the same Pd standard solution as that of working example 31, mixed and prepared to distilled water, and then subjecting it to hydrogen-gas replacement, is given as working example 47.

### (Working example 48)

Radical scavenging activity measurement data obtained in conformity with the same testing procedures as those of working example 46 when AOW is used as test water, which is obtained by adding a mixed (Pt + Pd) colloid solution of 336 µg/ L with a mole ratio of 1 to 15 for the same Pt standard solution as that of working example 26 and the same Pd standard solution as that of working example 31, mixed and prepared to distilled water, and then subjecting it to hydrogen-gas replacement, is given as working example 48.

### (Working example 49)

Radical scavenging activity measurement data obtained in conformity with the same testing procedures as those of working example 46 when AOW is used as test water, which is obtained by adding a mixed (Pt + Pd) colloid solution of 432 µg/ L with a mole ratio of 1 to 20 for the same Pt standard solution as that of working example 26 and the same Pd standard solution as that of working example 31, mixed and prepared to distilled water, and then subjecting it to hydrogen-gas replacement, is given as working example 49.

### (Working example 50)

Radical scavenging activity measurement data obtained in conformity with the same testing procedures as those of working example 46 when AOW is used as test water, which is obtained by adding a mixed (Pt + Pd) colloid solution of 528 µg/ L with a mole ratio of 1 to 25 for the same Pt standard solution as that of working example 26 and the same Pd standard solution as that of working example 31, mixed and prepared to distilled water, and then subjecting it to hydrogen-gas replacement, is given as working example 50.

### (Reference example 21)

Radical scavenging activity measurement data obtained in conformity with testing procedures, which are the testing procedures described in (A-4) with an altered part, when hydrogen gas-replaced distilled water is employed as test water is given as reference example 21. The alteration of the testing procedures is that instead of adding 300 µL of xanthine solution and 100 µL of xanthineoxidase solution to a tester cell, that is, instead of removing the (·O₂⁻) generation system, the amount of distilled water for supplying oxygen is increased from 900 µL to 1300 µL.

### (Working example 51)

Radical scavenging activity measurement data obtained in conformity with testing procedures, which are given by changing a part of the testing procedures described in (A-4), when AOW is used as test water, which is obtained by adding a mixed (Pt + Pd) colloid solution of 144 µg/ L with a mole ratio of 1 to 5 for the same Pt standard solution as that of working example 26 and the same Pd standard solution as that of working example 31, mixed and prepared to distilled water, and then subjecting it to hydrogen-gas replacement, is given as working example 51. The alteration of the testing procedures is the same as that of reference example 21.

### (Working example 52)

Radical scavenging activity measurement data obtained in conformity with the same testing procedures as those of working example 51 when AOW is used as test water, which is obtained by adding a mixed (Pt + Pd) colloid solution of 240 µg/ L with a mole ratio of 1 to 10 for the same Pt standard solution as that of working example 26 and the same Pd standard solution as that of working example 31, mixed and prepared to distilled water, and then subjecting it to hydrogen-gas replacement, is given as working example 52.

### (Working example 53)

Radical scavenging activity measurement data obtained in conformity with the same testing procedures as those of working example 51 when AOW is used as test water, which is obtained by adding a mixed (Pt + Pd) colloid solution of 336 µg/ L with a mole ratio of 1 to 15 for the same Pt standard solution as that of working example 26 and the same Pd standard solution as that of working example 31, mixed and prepared to distilled water, and then subjecting it to hydrogen-gas replacement, is given as working example 53.

### (Working example 54)

Radical scavenging activity measurement data obtained in conformity with the same testing procedures as those of working example 51 when AOW is used as test water, which is obtained by adding a mixed (Pt + Pd) colloid solution of 432 µg/ L with a mole ratio of 1 to 20 for the same Pt standard solution as that of working example 26 and the same Pd standard solution as that of working example 31, mixed and prepared to distilled water, and then subjecting it to hydrogen-gas replacement, is given as working example 54.

### (Working example 55)

Radical scavenging activity measurement data obtained in conformity with the same testing procedures as those of working example 51 when AOW is used as test water, which is obtained by adding a mixed (Pt + Pd) colloid solution of 528 µg/ L with a mole ratio of 1 to 25 for the same Pt standard solution as that of working example 26 and the same Pd standard solution as that of working example 31, mixed and prepared to distilled water, and then subjecting it to hydrogen-gas replacement, is given as working example 55. (Reference example 22)

Radical scavenging activity measurement data for test water obtained in conformity with testing procedures described in (A-4) when pH buffer solution (base water 6.86) is used as test water, which is obtained by diluting standard buffer solution 6.86 (phosphate solution) manufactured by Wako Pure Chemical Industries, Ltd. to one- tenth strength with purified water, is given as reference example 22.

### (Reference example 23)

Radical scavenging activity measurement data for test water obtained in conformity with the same testing procedures as those of reference example 22 when catalyst-free electrolyzed water is used as test water, which is the same base water 6.86 as that of reference example 22 that has been subjected to electrolysis processing using a continuous flow method under conditions of a 1.5 L/min flow and 5A constant current, is given as reference example 23.

### (Working example 56)

1 liter of the same base water 6.86 as that of reference example 22 has been collected and the same Pt standard solution as that of working example 26 sufficient to give a concentration of 48 µg/ L has been added to that water to prepare Pt colloid- containing base water 6.86. Radical scavenging activity measurement data for test water (AOW) obtained in conformity with the same testing procedures as those of reference example 22 when catalyst added one-pass electrolyzed water is used as test water (AOW), which is the Pt colloid-containing base water 6.86 prepared in this manner that has been subjected to electrolysis processing using a continuous flow method under the same conditions as those of reference example 23, is given as working example 56.

### (Working example 57)

1 liter of the same base water 6.86 as that of reference example 22 has been collected and the same Pt standard solution as that of working example 26 sufficient to give a concentration of 96 µg/ L has been added to that water to prepare Pt colloid- containing base water 6.86. Radical scavenging activity measurement data for test water (AOW) obtained in conformity with the same testing procedures as those of working example 56 when catalyst added one-pass electrolyzed water is used as test water (AOW), which is the Pt-containing base water 6.86 prepared in the above-mentioned manner that has been subjected to electrolysis processing using a continuous flow method under the same conditions as those of reference example 23, is given as working example 57.

### (Working example 58)

1 liter of the same base water 6.86 as that of reference example 22 has been collected, and the same Pt standard solution as that of working example 26 sufficient to give a concentration of 192 µg/ L has been added to that water so as to prepare Pt colloid- containing base water 6.86. Radical scavenging activity measurement data for test water (AOW) obtained in conformity with the same testing procedures as those of working example 56 when catalyst added one-pass electrolyzed water is used as test water (AOW), which is the Pt-containing base water 6.86 prepared in the above-mentioned manner that has been subjected to electrolysis processing using a continuous flow method under the same conditions as those of reference example 23, is given as working example 58.

### (Working example 59)

1 liter of the same base water 6.86 as that of reference example 22 has been collected, and the same Pt standard solution as that of working example 26 sufficient to give a concentration of 384 µg/ L has been added to that water so as to prepare Pt colloid- containing base water 6.86. Radical scavenging activity measurement data for test water (AOW) obtained in conformity with the same testing procedures as those of working example 56 when catalyst added one-pass electrolyzed water is used as test water (AOW), which is the Pt-containing base water 6.86 prepared in the above-mentioned manner that has been subjected to electrolysis processing using a continuous flow method under the same conditions as those of reference example 23, is given as working example 59.

### (Working example 60)

1 liter of the same base water 6.86 as that of reference example 22 has been collected, and the same Pt standard solution as that of working example 26 sufficient to give a concentration of 768 µg/ L has been added to that water so as to prepare Pt colloid- containing base water 6.86. Radical scavenging activity measurement data for test water (AOW) obtained in conformity with the same testing procedures as those of working example 56 when catalyst added one-pass electrolyzed water is used as test water (AOW), which is the Pt-containing base water 6.86 prepared in the above-mentioned manner that has been subjected to electrolysis processing using a continuous flow method under the same conditions as those of reference example 23, is given as working example 60.

### (Working example 61)

1 liter of the same base water 6.86 as that of reference example 22 has been collected, and the same Pd standard solution as that of working example 31 sufficient to give a concentration of 48 µg/ L has been added to that water so as to prepare Pd colloid- containing base water 6.86. Radical scavenging activity measurement data for test water (AOW) obtained in conformity with the same testing procedures as those of reference example 22 when catalyst added one-pass electrolyzed water is used as test water (AOW), which is the Pd-containing base water 6.86 prepared in the above-mentioned manner that has been subjected to electrolysis processing using a continuous flow method under the same conditions as those of reference example 23, is given as working example 61.

### (Working example 62)

1 liter of the same base water 6.86 as that of reference example 22 has been collected, and the same Pd standard solution as that of working example 31 sufficient to give a concentration of 96 µg/ L has been added to that water so as to prepare Pd colloid- containing base water 6.86. Radical scavenging activity measurement data for test water (AOW) obtained in conformity with the same testing procedures as those of working example 61 when catalyst added one-pass electrolyzed water is used as test water (AOW), which is the Pd-containing base water 6.86 prepared in the above-mentioned manner that has been subjected to electrolysis processing using a continuous flow method under the same conditions as those of reference example 23, is given as working example 62.

### (Working example 63)

1 liter of the same base water 6.86 as that of reference example 22 has been collected, and the same Pd standard solution as that of working example 31 sufficient to give a concentration of 192 µg/ L has been added to that water so as to prepare Pd colloid- containing base water 6.86. Radical scavenging activity measurement data for test water (AOW) obtained in conformity with the same testing procedures as those of working example 61 when catalyst added one-pass electrolyzed water is used as test water (AOW), which is the Pd-containing base water 6.86 prepared in the above-mentioned manner that has been subjected to electrolysis processing using a continuous flow method under the same conditions as those of reference example 23, is given as working example 63.

### (Working example 64)

1 liter of the same base water 6.86 as that of reference example 22 has been collected, and the same Pd standard solution as that of working example 31 sufficient to give a concentration of 384 µg/ L has been added to that water so as to prepare Pd colloid- containing base water 6.86. Radical scavenging activity measurement data for test water (AOW) obtained in conformity with the same testing procedures as those of working example 61 when catalyst added one-pass electrolyzed water is used as test water (AOW), which is the Pd-containing base water 6.86 prepared in the above-mentioned manner that has been subjected to electrolysis processing using a continuous flow method under the same conditions as those of reference example 23, is given as working example 64.

### (Working example 65)

1 liter of the same base water 6.86 as that of reference example 22 has been collected, and the same Pd standard solution as that of working example 31 sufficient to give a concentration of 768 µg/ L has been added to that water so as to prepare Pd colloid- containing base water 6.86. Radical scavenging activity measurement data for test water (AOW) obtained in conformity with the same testing procedures as those of working example 61 when catalyst added one-pass electrolyzed water is used as test water (AOW), which is the Pd-containing base water 6.86 prepared in the above-mentioned manner that has been subjected to electrolysis processing using a continuous flow method under the same conditions as those of reference example 23, is given as working example 65.

### (Working example 66)

1 liter of the same base water 6.86 as that of reference example 22 has been collected, and the same Pt standard solution as that of working example 26 sufficient to give a concentration of 48 µg/ L has been added to that water so as to prepare Pt colloid- containing base water 6.86. Radical scavenging activity measurement data for test water (AOW) obtained in conformity with the same testing procedures as those of reference example 22 when pre-catalyst addition circulating electrolyzed water is used as test water (AOW), which is the Pt colloid-containing base water 6.86 prepared in this manner that has been subjected to electrolysis processing using a continuous flow circulating method (volume of circulatory water: 0.8 liters) for three minutes under conditions of a 1.5 L/min flow and 5A constant current, is given as working example 66.

### (Working example 67)

1 liter of the same base water 6.86 as that of reference example 22 has been collected, and the same Pt standard solution as that of working example 26 sufficient to give a concentration of 96 µg/ L has been added to that water so as to prepare Pt colloid- containing base water 6.86. Radical scavenging activity measurement data for test water (AOW) obtained in conformity with the same testing procedures as those of working example 66 when catalyst added circulating electrolyzed water is used as test water (AOW), which is the Pt colloid-containing base water 6.86 prepared in the above-mentioned manner that has been subjected to electrolysis processing using a continuous flow circulating method (volume of circulatory water: 0.8 liters) for three minutes under the same conditions as that of working example 66, is given as working example 67.

### (Working example 68)

1 liter of the same base water 6.86 as that of reference example 22 has been collected, and the same Pt standard solution as that of working example 26 sufficient to give a concentration of 192 µg/ L has been added to that water to prepare Pt colloid- containing base water 6.86. Radical scavenging activity measurement data for test water (AOW) obtained in conformity with the same testing procedures as those of working example 66 when catalyst added circulating electrolyzed water is used as test water (AOW), which is the Pt colloid-containing base water 6.86 prepared in the above-mentioned manner that has been subjected to electrolysis processing using a continuous flow circulating method (volume of circulatory water: 0.8 liters) for three minutes under the same conditions as that of working example 66, is given as working example 68.

### (Working example 69)

1 liter of the same base water 6.86 as that of reference example 22 has been collected, and the same Pt standard solution as that of working example 26 sufficient to give a concentration of 384 µg/ L has been added to that water so as to prepare Pt colloid- containing base water 6.86. Radical scavenging activity measurement data for test water (AOW) obtained in conformity with the same testing procedures as those of working example 66 when catalyst added circulating electrolyzed water is used as test water (AOW), which is the Pt colloid-containing base water 6.86 prepared in the above-mentioned manner that has been subjected to electrolysis processing using a continuous flow circulating method (volume of circulatory water: 0.8 liters) for three minutes under the same conditions as that of working example 66, is given as working example 69.

### (Working example 70)

1 liter of the same base water 6.86 as that of reference example 22 has been collected, and the same Pt standard solution as that of working example 26 sufficient to give a concentration of 768 µg/ L has been added to that water so as to prepare Pt colloid- containing base water 6.86. Radical scavenging activity measurement data for test water (AOW) obtained in conformity with the same testing procedures as those of working example 66 when catalyst added circulating electrolyzed water is used as test water (AOW), which is the Pt colloid-containing base water 6.86 prepared in the above-mentioned manner that has been subjected to electrolysis processing using a continuous flow circulating method (volume of circulatory water: 0.8 liters) for three minutes under the same conditions as that of working example 66, is given as working example 70.

### (Working example 71)

1 liter of the same base water 6.86 as that of reference example 22 has been collected, and the same Pd standard solution as that of working example 31 sufficient to give a concentration of 48 µg/ L has been added to that water so as to prepare Pd colloid- containing base water 6.86. Radical scavenging activity measurement data for test water (AOW) obtained in conformity with the same testing procedures as those of reference example 22 when catalyst added circulating electrolyzed water is used as test water (AOW), which is the Pd colloid-containing base water 6.86 prepared in the above-mentioned manner that has been subjected to electrolysis processing using a continuous flow circulating method (volume of circulatory water: 0.8 liters) for three minutes under conditions of a 1.5 L/ min flow and 5A constant current, is given as working example 71.

### (Working example 72)

1 liter of the same base water 6.86 as that of reference example 22 has been collected, and the same Pd standard solution as that of working example 31 sufficient to give a concentration of 96 µg/ L has been added to that water so as to prepare Pd colloid- containing base water 6.86. Radical scavenging activity measurement data for test water (AOW) obtained in conformity with the same testing procedures as those of working example 71 when catalyst added circulating electrolyzed water is used as test water (AOW), which is the Pd colloid-containing base water 6.86 prepared in the above-mentioned manner that has been subjected to electrolysis processing using a continuous flow circulating method (volume of circulatory water: 0.8 liters) for three minutes under the same conditions as those of working example 66, is given as working example 72.

### (Working example 73)

1 liter of the same base water 6.86 as that of reference example 22 has been collected, and the same Pd standard solution as that of working example 31 sufficient to give a concentration of 192 µg/ L has been added to that water so as to prepare Pd colloid- containing base water 6.86. Radical scavenging activity measurement data for test water (AOW) obtained in conformity with the same testing procedures as those of working example 71 when catalyst added circulating electrolyzed water is used as test water (AOW), which is the Pd colloid-containing base water 6.86 prepared in the above-mentioned manner that has been subjected to electrolysis processing using a continuous flow circulating method (volume of circulatory water: 0.8 liters) for three minutes under the same conditions as those of working example 66, is given as working example 73.

### (Working example 74)

1 liter of the same base water 6.86 as that of reference example 22 has been collected, and the same Pd standard solution as that of working example 31 sufficient to give a concentration of 384 µg/ L has been added to that water so as to prepare Pd colloid- containing base water 6.86. Radical scavenging activity measurement data for test water (AOW) obtained in conformity with the same testing procedures as those of working example 71 when catalyst added circulating electrolyzed water is used as test water (AOW), which is the Pd colloid-containing base water 6.86 prepared in the above-mentioned manner that has been subjected to electrolysis processing using a continuous flow circulating method (volume of circulatory water: 0.8 liters) for three minutes under the same conditions as those of working example 66, is given as working example 74.

### (Working example 75)

1 liter of the same base water 6.86 as that of reference example 22 has been collected, and the same Pd standard solution as that of working example 31 sufficient to give a concentration of 768 µg/ L has been added to that water so as to prepare Pd colloid- containing base water 6.86. Radical scavenging activity measurement data for test water (AOW) obtained in conformity with the same testing procedures as those of working example 71 when catalyst added circulating electrolyzed water is used as test water (AOW), which is the Pd colloid-containing base water 6.86 prepared in the above-mentioned manner that has been subjected to electrolysis processing using a continuous flow circulating method (volume of circulatory water: 0.8 liters) for three minutes under the same conditions as those of working example 66, is given as working example 75.

### (Working example 76)

Radical scavenging activity measurement data obtained in conformity with testing procedures altered the same as with reference example 21 when AOW is used as test water, which is obtained by adding the same Pt standard solution as that of working example 26 sufficient to give a Pt colloid concentration of 384 µg/ L to distilled water, and then subjecting it to hydrogen gas replacement, is given as working example 76.

### (Working example 77)

Radical scavenging activity measurement data obtained in conformity with the same testing procedures as that of working example 76 when AOW is used as test water, which is obtained by adding the same Pd standard solution as that of working example 31 sufficient to give a Pd colloid concentration of 384 µg/ L to distilled water, and then that subjecting it to hydrogen-gas replacement, is given as working example 77.

### (Reference example 24)

Radical scavenging activity measurement data obtained in conformity with the same testing procedures as that of reference example 19 when an AsA solution is used as test water, which is obtained by adding ascorbic acid (AsA) sufficient to give a concentration of 35.5 µM to distilled water, is given as reference example 24.

### (Reference example 25)

Radical scavenging activity measurement data obtained in conformity with the same testing procedures as that of reference example 19 when AsA solution is used as test water, which is obtained by adding ascorbic acid (AsA) sufficient to give a concentration of 71 µM to distilled water, is given as reference example 25.

### (Reference example 26)

Radical scavenging activity measurement data obtained in conformity with the same testing procedures as that of reference example 19 when AsA solution is used as test water, which is obtained by adding ascorbic acid (AsA) sufficient to give a concentration of 142 µM to distilled water, is given as reference example 26.

### (Reference example 27)

Radical scavenging activity measurement data obtained in conformity with the same testing procedures as that of reference example 19 when AsA solution is used as test water, which is obtained by adding ascorbic acid (AsA) sufficient to give a concentration of 284 µM to distilled water, is given as reference example 27.

### (C) Examination of working examples

FIG. 26, which compares reference examples 19, 20, and working examples 26 through 30, shows characteristics of the radical scavenging activity changing over time when Pt colloid catalyst-containing hydrogen-dissolved water (AOW) is expressed where the Pt colloid (particle size distribution: 2 to 4 nm) concentration is used as a main parameter. Here, reference examples 19 and 20 are both used as references in order to grasp the profiles of the radical scavenging activity characteristics when oxygen is removed from test water (fluid) occupying 1/3 of the tester cell capacity of 3 mL. It should be noted that it has been confirmed through an experiment not shown in the drawings that even when nitrogen gas-replaced distilled water is employed as test water, instead of the hydrogen gas-replaced distilled water of reference example 20, exactly the same characteristics of the radical scavenging activity changing over time can be obtained. This means that the type of gas used as a reference does not affect the characteristics of the radical scavenging activity changing over time as long as the same amount of oxygen is removed (hereafter, the same). Here, according to this drawing, when compared to the radical scavenging activity characteristics of reference examples 19 and 20 (hereafter, the characteristics of reference examples are abbreviated as 'reference characteristics'), the radical scavenging activity characteristics of AOWs of working examples 26 through 30 (hereafter, characteristics of working examples are abbreviated as 'subject characteristics') are significantly lower than the reference characteristics for each concentration after approximately 680 seconds from the start of measurement of change over time (hereafter, abbreviated as 'start of measurement') of the light absorbance (A480) using a spectrophotometer. In other words, it can be understood that AOWs of working examples 26 through 30 begin to express favorable radical scavenging activity in a wide concentration range after a certain time period has passed. In addition, analyzing the subject characteristics of working examples 26 through 30 in detail shows that the higher the Pt colloid concentration, the shorter the time required for radical scavenging. In other words, the radical scavenging activity expressed in AOW becomes higher depending on the Pt colloid concentration. It should be noted that when Pt colloid is employed as a precious metal catalyst, there are time ranges where the subject characteristics of working examples 26 through 30 are significantly higher than the reference characteristics for each concentration. The reason thereof will be described in the following section ' non-reactivity against oxidant, catalytic activity, and hydrogen-occluding capability of palladium (Pd) colloid', therefore it is no longer referred to at this time (hereafter, the same).

FIG. 27, which compares reference examples 19, 20, and working examples 31 through 35, shows characteristics of the radical scavenging activity changing over time when Pd colloid catalyst-containing hydrogen-dissolved water (AOW) is expressed where Pd colloid concentration is used as a main parameter. According to this drawing, when comparing the subject characteristics of working examples 31 through 35 to the reference characteristics of reference examples 19 and 20, in almost all time ranges from the start of measurement, the subject characteristics of AOW with a low concentration (working examples 31 through 33) are almost equivalent to the reference characteristics, but the subject characteristics of AOW with a high concentration (working examples 34 and 35) are all significantly lower than the reference characteristics. In other words, it can be understood that AOWs of working examples 31 through 35 begin to express favorable radical scavenging activity with high concentrations (working examples 34 and 35). In addition, analyzing the subject characteristics of working examples 34 and 35 in detail shows that the higher the Pd colloid concentration, the shorter the time required for radical scavenging. In other words, the radical scavenging activity expressed in AOW becomes higher depending on the Pd colloid concentration. In particular, with the subject characteristics of AOWs with a high concentration (working examples 34 and 35), the light absorption level begins to show a significant decrease after approximately 440 seconds and approximately 230 seconds, respectively, from the start of measurement. The reason can be guessed that since the (·O₂⁻) existence concentration (existing amount) acts as a switch for AOW with a high concentration (working examples 34 through 35), beginning to aggressively scavenge (·O₂⁻). It should be noted that when Pd colloid is employed as a precious metal catalyst, the subject characteristics of working examples 31 through 35 are mostly lower than the reference characteristics for each concentration. The reason thereof will be described in the following section 'non-reactivity against oxidant, catalytic activity, and hydrogen occlusion capability of palladium (Pd) colloid', and is therefore not described here (hereafter, the same).

FIG. 28, which compares reference examples 19, 20, and working examples 36 through 41, shows characteristics of the radical scavenging activity changing over time when Pt colloid catalyst-containing hydrogen-dissolved water (AOW) is expressed where Pt colloid (particle size distribution: 2 through 4 nm) concentration is used as a main parameter. According to this drawing, when compared to the reference characteristics of reference examples 19 and 20, the subject characteristics of AOW of working examples 36 through 41 are significantly lower than the reference characteristics for each concentration after approximately 920 seconds from the start of measurement. In other words, it can be understood that AOWs of working examples 36 through 41 begin to express favorable radical scavenging activity in a wide concentration range after a certain time period has passed. In addition, analyzing the subject characteristics of working examples 36 through 41 in detail shows that the higher the Pt colloid concentration, the shorter the time required for radical scavenging. In other words, the radical scavenging activity expressed in AOW becomes higher depending on the Pt colloid concentration.

Here, in order to clarify the relationship between the particle size parameters (particle size distribution: 2 to 4 nm/ particle size distribution: 1 to 2 nm) of Pt colloid and the radical scavenging activity, FIG. 26 where Pt colloid concentration with a particle size distribution of 2 to 4 nm is used as a main parameter is compared to FIG. 28 where Pt colloid concentration with a particle size distribution of 1 to 2 nm is used as a main parameter. In order to avoid influence from the concentration parameter, for example, working examples 27 and 37 using the same Pt concentration parameter (96 µg/ L) are compared. Working example 27 shows that the subject characteristic such as the light absorption level reaches a peak value after approximately 320 seconds from the start of measurement, and then gradually decreasing, and after approximately 440 seconds the light absorption level is once controlled to almost 0. Meanwhile, working example 37 shows that the subject characteristic such as the light absorption level reaches a peak value after approximately 760 seconds from the start of measurement, and then gradually decreasing, but the light absorption level is not controlled to almost 0 within the measurement time range. Next, working examples 29 and 40 using the same Pt concentration parameter (384 µg/ L) are compared. Working example 29 shows that the subject characteristic is controlled to almost 0 after approximately 170 seconds from the start of measurement, and that decreasing continues thereafter. Meanwhile, working example 40 shows that the subject characteristic such as the light absorption level reaches a peak value after approximately 260 seconds from the start of measurement, and then rapidly decreasing, and the light absorption level is controlled to almost 0 after approximately 340 seconds. Synthetically viewing the above-mentioned findings, a Pt colloid catalyst having a particle size distribution of 2 to 4 nm is more preferable than that having a particle size distribution of 1 to 2 nm as the Pt colloid catalyst to be used for the antioxidant-functioning water according to the present invention since having a particle size distribution of 2 to 4 nm can provide a better expression of the radical scavenging activity (which emanates from the fact that the time for control of the light absorption level to almost 0 is shorter).

FIG. 29, which compares reference examples 19, 20, and working examples 42 through 45, shows characteristics of the radical scavenging activity changing over time when mixed (Pt + Pd) colloid catalyst-containing hydrogen-dissolved water (AOW) is expressed where mixed (Pt + Pd) colloid (particle size distribution: 2 through 4 nm, Pt to Pd mixture mole ratio: 1 to 2) concentration is used as a main parameter. According to this drawing, when compared to the reference characteristics of reference examples 19 and 20, the subject characteristics of AOW of working examples 42 through 45 are significantly lower than the reference characteristics for each concentration after approximately 900 seconds from the start of measurement. In other words, it can be understood that AOWs of working examples 42 through 45 begin to express the favorable radical scavenging activity in a wide concentration range after a certain time period has passed. In addition, analyzing the subject characteristics of working examples 42 through 45 in detail shows that the higher the mixed (Pt + Pd) colloid concentration, the shorter the time required for radical scavenging. In other words, the radical scavenging activity expressed in AOW becomes higher depending on the mixed (Pt + Pd) colloid concentration.

FIG. 30, which compares reference examples 19, 20, and working examples 46 through 50, shows characteristics of the radical scavenging activity changing over time when mixed (Pt + Pd) colloid catalyst-containing hydrogen-dissolved water (AOW) is expressed where mixed (Pt + Pd) colloid (particle size distribution: 2 through 4 nm) concentration is used as a main parameter and Pt to Pd mixture mole ratio is used as a sub parameter. According to this drawing, when compared to the reference characteristics of reference examples 19 and 20, the subject characteristics of working examples 46 through 50 are significantly lower than the reference characteristics for each concentration after approximately 520 seconds from the start of measurement. In other words, it can be understood that AOWs of working examples 46 through 50 begin to express the favorable radical scavenging activity in a wide concentration range after a certain time period has passed. In addition, analyzing the subject characteristics of working examples 46 through 50 in detail shows that the higher the mixed (Pt + Pd) colloid concentration, the shorter the time required for radical scavenging. In other words, the radical scavenging activity expressed in AOW becomes higher depending on the mixed (Pt + Pd) colloid concentration.

FIG. 31, which compares reference example 21, and working examples 51 through 55, shows characteristics of the radical scavenging activity changing over time when mixed (Pt + Pd) colloid catalyst-containing hydrogen-dissolved water (AOW) is expressed where mixed (Pt + Pd) colloid (particle size distribution: 2 through 4 nm) concentration is used as a main parameter and Pt to Pd mixture mole ratio is used as a sub parameter. It should be noted that in reference example 21 and working examples 46 through 50, the (·O₂⁻) generation system is removed. According to this drawing, when compared to the reference characteristics of reference example 21, an (·O₂⁻) generating tendency is found in the subject characteristics of working examples 51 through 55 even though the (·O₂⁻) generation system has been removed. Since this point is described in the following description of FIG. 37, it is no longer referred to at this time. This (·O₂⁻) generating tendency depends on the mixed (Pt + Pd) colloid concentration, that is, the higher the concentration becomes, the more this (·O₂⁻) generating tendency is controlled. In other words, the radical scavenging activity expressed in AOW becomes higher depending on the mixed (Pt + Pd) colloid concentration. In addition, comprehensively taking into consideration the phenomena in relation to FIG. 37 to be described later, it can be concluded that the higher the mixed mole ratio of Pd colloid becomes, the more the (·O₂⁻) generating tendency is controlled.

FIG. 32, which compares reference examples 22, 23, and working examples 56 through 60, shows characteristics of the radical scavenging activity changing over time when Pt colloid catalyst added one-pass electrolyzed water (AOW) is expressed where Pt colloid (particle size distribution: 2 to 4 nm) concentration is used as a main parameter. According to this drawing, while the subject characteristics with a low concentration of working examples 56 through 60 (working examples 56 and 57) are almost equivalent to the reference characteristics, the subject characteristics of AOW with a high concentration (working examples 58 through 60) are significantly lower than the reference characteristics for each concentration after approximately 740 seconds from the start of measurement. In other words, it can be understood that AOWs of working examples 58 through 60 begin to express the favorable radical scavenging activity in a wide concentration range after a certain time period has passed. In addition, analyzing the subject characteristics of working examples 58 through 60 in detail shows that the higher the Pt colloid concentration, the shorter the time required for radical scavenging. In other words, the radical scavenging activity expressed in AOW becomes higher depending on the Pt colloid concentration.

FIG. 33, which compares reference examples 22, 23, and working examples 61 through 65, shows characteristics of the radical scavenging activity changing over time when Pd colloid catalyst added one-pass electrolyzed water is expressed where Pd colloid (particle size distribution: 2 through 4 nm) is used as a main parameter. According to this drawing, the subject characteristics of AOW with a low concentration of the working examples 61 through 65 (working examples 61 and 62) are almost equivalent to the reference characteristics, but the subject characteristics of AOW with a high concentration (working examples 63 through 65) are significantly lower than the reference characteristics for each concentration after approximately 320 seconds from the start of measurement. In other words, it can be understood that AOWs of working examples 63 through 65 begin to express the favorable radical scavenging activity in a wide concentration range after a certain time period has passed. In addition, analyzing the subject characteristics of working examples 63 through 65 in detail shows that the higher the Pd colloid concentration, the shorter the time required for radical scavenging. In other words, the radical scavenging activity expressed in AOW becomes higher depending on the Pd colloid concentration. In particular, in the case of the subject characteristics of AOW with the highest concentration (working example 65), the light absorption level begin to show a significant decrease after approximately 830 seconds from the start of measurement. The reason can be guessed that since the (·O₂⁻) existence concentration (existing amount) acts as a switch for AOW with a high concentration (working example 65), beginning to aggressively scavenge (·O₂⁻).

FIG. 34, which compares reference examples 22, 23, and working examples 66 through 70, shows characteristics of the radical scavenging activity changing over time when Pt colloid catalyst added circulating electrolyzed water (AOW) is expressed where Pt colloid (particle size distribution: 2 through 4 nm) concentration is used as a main parameter. According to this drawing, the subject characteristics of AOW with a low concentration of the working examples 66 through 70 (working examples 66 and 67) are almost equivalent to the reference characteristics, but the subject characteristics of AOW with a high concentration (working examples 68 through 70) are significantly lower than the reference characteristics for each concentration after approximately 700 seconds from the start of measurement. In other words, it can be understood that AOWs of working examples 68 through 70 begin to express favorable radical scavenging activity in a wide concentration range after a certain time period has passed. In addition, analyzing the subject characteristics of working examples 68 through 70 in detail shows that the higher Pt colloid concentration, the shorter the time required for radical scavenging. In other words, the radical scavenging activity expressed in AOW becomes higher depending on the Pt colloid concentration.

FIG. 35, which compares reference examples 22 and 23 and working examples 71 through 75, shows characteristics of the radical scavenging activity changing over time when Pd colloid catalyst added circulating electrolyzed water (AOW) is expressed where Pd colloid (particle size distribution is 2 through 4 nm) concentration is used as a main parameter. According to this drawing, while the subject characteristics of AOW with a low concentration of the working examples 71 through 75 (working examples 71 and 72) show a slight downward tendency after approximately 950 seconds from the start of measurement, the subject characteristics of AOW with a high concentration (working examples 73 through 75) are significantly lower than the reference characteristics for each concentration after approximately 650 seconds from the start of measurement. In other words, it can be understood that AOWs of working examples 71 through 75 begin to express the favorable radical scavenging activity in a wide concentration range after a certain time period has passed. In addition, analyzing the subject characteristics of working examples 71 through 75 in detail shows that the higher the Pd colloid concentration, the shorter the time required for radical scavenging. In other words, the radical scavenging activity expressed in AOW becomes higher depending on the Pd colloid concentration. In particular, with the subject characteristics of AOW with high concentration (working examples 73 through 75), the light absorption level begins to show a significant decrease after approximately 650 seconds, approximately 420 seconds, and approximately 230 seconds, respectively, from the start of measurement. The reason can be guessed that since the (·O₂⁻) existence concentration (existing amount) acts as a switch for AOW with a high concentration (working examples 73 through 75), beginning to aggressively scavenge (·O₂⁻).

Here, in order to clarify the relationship between electrolysis condition parameters for a Pt colloid catalyst (one-pass electrolysis/ circulating electrolysis) and the radical scavenging activity, FIG. 32 where Pt colloid catalyst added one-pass electrolyzed water is employed as test water is compared to FIG. 34 where Pt colloid catalyst added circulating electrolyzed water is employed as test water. In order to avoid influence from the concentration parameter, for example, working examples 58 and 68 using the same Pt concentration parameter (192 µg/ L) are compared. Working example 58 shows that the subject characteristic such as the light absorption level reaches its peak after approximately 680 seconds from the start of measurement, then gradually decreasing, and after approximately 880 seconds the light absorption level is controlled to almost 0. Meanwhile, working example 68shows that the subject characteristic such as the light absorption level reaches its peak after approximately 620 seconds from the start of measurement, then gradually decreasing, and after approximately 830 seconds the light absorption level is controlled to almost 0. Next, working examples 59 and 69 using the same Pt concentration parameter (384 µg/ L) are compared. Working example 59 shows that the subject characteristic such as the light absorption level reaches its peak after approximately 530 seconds from the start of measurement, then gradually decreasing, and after approximately 660 seconds the light absorption level is controlled to almost 0. Meanwhile, working example 69 shows that the subject characteristic such as the light absorption level reaches its peak after approximately 400 seconds from the start of measurement, then gradually decreasing, and after approximately 500 seconds the light absorption level is controlled to almost 0. Synthetically viewing the above-mentioned findings, the circulating electrolysis is more preferable than the one-pass electrolysis as the electrolysis condition (only for Pd colloid catalyst added electrolysis) for generating antioxidant-functioning water according to the present invention since that circulating electrolysis can provide a better expression of the radical scavenging activity (which emanates from the fact that the time for control of the light absorption level to almost 0 is shorter).

On the other hand, in order to clarify the relationship between electrolysis condition parameters for a Pd colloid catalyst (one-pass electrolysis/ circulating electrolysis) and the radical scavenging activity, FIG. 33 where Pd colloid catalyst added one-pass electrolyzed water is employed as test water is compared to FIG. 35 where Pd colloid catalyst added circulating electrolyzed water is employed as test water. In order to avoid influence from the concentration parameter, for example, working examples 63 and 73 using the same Pd concentration parameter (192 µg/ L) are compared. In working example 63, while the subject characteristics thereof are lower than the reference characteristics of reference examples 22 and 23 in all time ranges from the start of measurement, those subject characteristics show a gradual upward tendency. Meanwhile, the subject characteristics of working example 73 show a gradual upward tendency until reaching approximately 650 seconds (peak of light absorption level) from the start of measurement. In turn, those subject characteristics then show a gradual downward tendency, and the light absorption level is controlled to almost 0 after approximately 860 seconds. Next, working examples 65 and 75 using the same Pd concentration parameter (768 µg/ L) are compared. In working example 65, the subject characteristics show a gradual upward tendency until reaching approximately 680 to 800 seconds (peak of light absorption level) from the start of measurement. In turn, those subject characteristics then show a gradual downward tendency. Meanwhile, working example 75 shows that the subject characteristic such as the light absorption level reaches its peak upon start of measurement, then gradually decreasing, and after approximately 320 seconds the light absorption level is controlled to almost 0. Synthetically viewing the above-mentioned findings, the circulating electrolysis is more preferable than the one-pass electrolysis as the electrolysis condition (only for Pd colloid catalyst added electrolysis) for generating antioxidant-functioning water according to the present invention since that circulating electrolysis can provide a better expression of the radical scavenging activity (which emanates from the fact that the time for control of the light absorption level to almost 0 is shorter).

FIG. 36, which compares reference examples 19, 20, 24 through 27, shows characteristics of the radical scavenging activity changing over time when AsA solution is expressed where AsA solution concentration is used as a main parameter. According to this drawing, the radical scavenging activity characteristic of the AsA solution of reference examples 24 through 27 is lower than the characteristic of reference examples 19 and 20 for each concentration. In other words, a well-known fact can be confirmed that the radical scavenging activity is expressed in AsA solutions of reference examples 24 through 27 in a wide concentration range. In addition, another well-known fact can also be confirmed that the radical scavenging activity expressed in the AsA solutions of reference examples 24 through 27 becomes higher depending on the concentration. It should be noted that when comparing the radical scavenging activity expressed in the above-mentioned AsA solution to the radical scavenging activity expressed in antioxidant-functioning water according to the present invention, it can be known that, for example, the radical scavenging activity expressed in the antioxidant-functioning water of working example 75 exceeds by far that expressed in the AsA solution of reference example 24. Therefore, the radical scavenging activity, which is comparable to the AsA solution of reference examples 25 through 27, is expressed in that antioxidant-functioning water.

### Non-reactivity against oxidant, catalytic activity, and hydrogen-occluding capability of palladium (Pd) colloid

Existence of oxygen becomes a large barrier when the antioxidation method, antioxidant-functioning water, and usage thereof according to the present invention are attempted to be implemented in an oxygen-dissolved solution system such as a living organism. The living organisms are especially abundant in oxygen since oxygen is used to procure energy by oxidizing nutrients and perform various oxygen- added reactions essential for the living organisms. The true nature of the problem is decay in radical scavenging activity emanating from the fact that oxygen will consume the hydrogen dissolved in the antioxidant-functioning water by the assistance of a precious metal colloid catalyst, in other words, hydrogen and oxygen react to each other through a precious metal colloid catalyst, reverting to normal water, as well as a scavenging subject, (·O₂⁻) is adversely generated since the oxygen itself is reduced of one electron by the hydrogen, which is activated through a precious metal colloid catalyst. There is a tendency that the higher the catalytic activity becomes, the more the phenomenon according to this preposition increases. In short, since catalytic activity and radical scavenging activity have a trade-off relationship, the higher the catalytic activity becomes, the more the radical scavenging activity decays. Accordingly, it can be said that this is a deep-seated problem that cannot be easily solved.

As eager research progressed in order to solve such substantial problems, the inventors found that in the precious metal colloid catalysts, palladium (Pd) colloid especially shows non-reactivity against oxidant in comparison with platinum (Pt) colloid. As further research progressed based on this discovery, the inventors clarified that the important factors to be considered when searching for precious metal colloid catalysts available for the present invention are: non-reactivity against oxidant, catalytic activity, and hydrogen occlusion capability. Taking into consideration these three factors, the inventors have finally found that the favorable precious metal colloid catalyst is Pd colloid from the standpoint of overall capacity, thereby completing the invention.

To begin with, grounds for the conclusion that Pd colloid shows non-reactivity against oxidant in comparison with Pt colloid are described below.

FIG. 37, which compares reference example 21, working examples 76, and 77, shows characteristics of the radical scavenging activity changing over time when catalyst-containing hydrogen-dissolved water (AOW) is expressed where difference in a type of a precious metal catalyst (concentration is fixed) is used as a main parameter. It should be noted that in reference example 21 and working examples 76 through 77, the (·O₂⁻) generation system is removed. This drawing shows that although the (·O₂⁻) generation system is removed, the subject characteristic such as the light absorbance level for working example 76 reaches a peak value of approximately 0.046 (when approximately 160 seconds have passed) between approximately 140 to 200 seconds from the start of measurement. It also shows that the light absorbance level gradually increases after approximately 860 seconds from the start of measurement. On the other hand, the subject characteristic of working example 77 show almost the same tendency as the reference characteristic of reference example 21, but the light absorbance does not rise. These may be thought that in the subject characteristic of working example 76, (·O₂⁻) has been actively generated until approximately 160 seconds from the start of measurement, and the (·O₂⁻) generated in the above-mentioned manner is scavenged by the radical scavenging expressed in antioxidant-functioning water according to the present invention. In addition, it is considered that the reason why the light absorbance level turns to gradually increase after approximately 860 seconds from the start of measurement is because generation of (·O₂⁻) cannot be controlled and scavenged as a result of the radical scavenging activity of the antioxidant-functioning water according to the present invention having decayed or having been sapped.

Next, a theory regarding the working-action mechanism of the Pt colloid catalyst and the Pd colloid catalyst in a solution system where hydrogen and oxygen coexist (a solution system where oxygen is dissolved in hydrogen-dissolved water of the present invention) is explained.

FIG. 38 shows the working-action mechanism of the Pt colloid catalyst in the hydrogen-oxygen coexistent solution system.

As shown in this drawing, the Pt colloid catalyst absorbs hydrogen and oxygen, which are dissolved in the system, and passes to oxygen one electron released from the activated hydrogen (·H) (one-electron reduction of oxygen). At this time, the activated hydrogen (·H) loses one electron, and is released to the system as an H⁺ ion (hereafter, repetitive description hereof is omitted). That is, since the oxygen itself is reduced of one electron by the activated hydrogen through the Pt colloid catalyst, (·O₂⁻) is adversely generated although it is a primary scavenging subject. Thereafter, or at the same time, the Pt colloid catalyst absorbs (·O₂⁻) and hydrogen dissolved in the system, and passes to (·O₂⁻) one electron released from the activated hydrogen (one-electron reduction of (·O₂⁻) or two-electron reduction of oxygen). In short, when (·O₂⁻) itself is reduced of one electron by the activated hydrogen through Pt colloid catalyst, (·O₂²⁻) is generated. An ionic bond is formed between the (·O₂²⁻) generated in this manner and two H⁺ ions existing in the system, generating hydrogen peroxide (H₂O₂). Thereafter, or at the same time, Pt colloid catalyst absorbs hydrogen and hydrogen peroxide (H₂O₂), which are dissolved in the system, and passes to (H₂O₂) one electron released from the activated hydrogen (one-electron reduction of (H₂O₂) or three-electron reduction of oxygen). In short, when (H₂O₂) itself is reduced of one electron by the activated hydrogen through Pt colloid catalyst, (·OH) is generated. Thereafter, or at the same time, Pt colloid catalyst absorbs hydrogen and (·OH), which are dissolved in the system, and passes to (·OH) one electron released from the activated hydrogen (one-electron reduction of (·OH) or four-electron reduction of oxygen). In short, when (·OH) itself is reduced of one electron by the activated hydrogen through Pt colloid catalyst, an OH⁻ ion is then generated. An ionic bond is formed between the OH⁻ generated in this manner and an H⁺ ion, generating water (H₂O), and ending a series of reactions. The working-action mechanism of the Pt colloid catalyst in the solution system where hydrogen and oxygen coexist has been explained.
Meanwhile, the working-action mechanism of the Pd colloid catalyst is described forthwith.

FIG. 39 shows the working-action mechanism of Pd colloid catalyst in hydrogen-oxygen coexistent solution system. It should be noted that since the greatest difference in the description of the working-action mechanism of the Pd colloid catalyst from the above-mentioned working-action mechanism of the Pt colloid catalyst is something originating from non-reactivity against oxidant, this is mainly described, and other repetitive descriptions are omitted.

As shown in this drawing, while the Pd colloid catalyst absorbs hydrogen dissolved in the system, it does not absorb oxygen actively, or it tends to be difficult to pass one electron released from the activated hydrogen (·H) to oxygen even if passive absorption (due to collision of oxygen to Pd colloid catalyst) occurs (no oxygen molecules reduced). Accordingly, hardly any (·O₂⁻), which is a primary scavenging subject, is generated. The following working-action mechanism is the same as that of the Pt colloid catalyst, that is, (·O₂⁻), hydrogen peroxide (H₂O₂), or (·OH) existing in the system is reduced by the Pd colloid catalyst and hydrogen, which is dissolved in the system, collaboratively; wherein water (H₂O) is ultimately generated, ending a series of reactions.

Here, to give the characteristics of palladium (Pd), which is favorable as the precious metal colloid catalyst according to the present invention, palladium has atomic number 46, an atomic mass of 106.42, and is a transition metal atom, which was discovered by Wollaston in 1803. This has been named after the asteroid Pallas (Athens in Greek mythology) which was discovered the previous year. It is a precious atom with only 24,000 tons existing on the earth. Palladium has excellent hydrogen-capturing capabilities, where it can occlude 740 to 935 times its own volume. It is frequently used as a hydrogenising catalyst and for purifying hydrogen. Palladium is most frequently used as a catalyst. It has been used as a hydrogenising catalyst, as well as a palladium complex has been used as a catalyst which is used to generate acetaldehyde from ethylene. Palladium is also used as a metal for dental treatment and decorative trim.

To think about a reactive subject to the antioxidant-functioning water according to the present invention, for example, the reactive subject can be roughly classified into a free radical with unpaired electrons and strong oxidizing power, and an oxidizing material without unpaired electrons but oxidizing power. Of these, in the former case, it may be considered to express radical scavenging activity since molecular hydrogen with a strong reducing power emanating from the antioxidant-functioning water according to the present invention corresponds to the radical (i.e., a molecular hydrogen electron is given to the radical). Meanwhile, in the latter case, it may be considered to selectively express reduction activity since molecular hydrogen with a strong reducing power emanating from the antioxidant-functioning water according to the present invention selectively corresponds to the oxidizing material according to the target (i.e., an electron of the molecular hydrogen may be given according to the target.) Here, selective expression of the reduction activity may be considered to mean that the reduction activity is selectively expressed depending on the condition of a degree allowing easy flow of frontier electrons, which occupy the highest occupied orbital on the molecular hydrogen side, to the lowest-altitude orbit on the oxidizing material side according to the compatibility between the molecular hydrogen and oxidizing material, that is, the frontier electron theory. Giving a specific example just for reference, in test water where vitamin B2 or an oxidizing material is dissolved in Pd colloid catalyst-containing (concentration: 192 µg/ L) hydrogen-dissolved water (AOW) according to the present invention, the reduction activity of vitamin B2 cannot be found. In this case, it may be considered that reduction activity is not expressed because the molecular hydrogen with a strong reducing power emanating from the antioxidant-functioning water according to the present invention gave no electrons to the oxidizing material (vitamin B2). In short, it can be said that molecular hydrogen and oxidizing material (vitamin B2) are incompatible. On the other hand, in the test water where oxidized methylene blue or an oxidizing material is dissolved in the Pd colloid catalyst-containing (concentration: 192 µg/ L) hydrogen-dissolved water according to the present invention, the reduction activity of methylene blue can be found. In this case, it may be considered that the reduction activity can be found since molecular hydrogen with a strong reducing power emanating from the antioxidant-functioning water according to the present invention gave electrons to the oxidizing material (oxidized methylene blue.) In short, it can be said that molecular hydrogen and oxidizing material (oxidized methylene blue) are congenial.

In order to enhance the reactivity of the antioxidant-functioning water according to the present invention to the above-mentioned reaction subject, the fact that for example, the antioxidant-functioning water dissolves hydrogen with a certain concentration higher than the saturated concentration (in terms of the effective value of dissolved hydrogen concentration found using a dissolved hydrogen concentration quantitative analysis method that uses oxidation/ reduction pigment) under atmospheric pressure, and/ or that a considerable amount of hydrogen is occluded in the precious metal catalyst itself contained in the hydrogen-dissolved water may be considered favorable.

### Disclosure of additional working examples by DH quantitative analysis method using oxidation/ reduction pigment

Additional working examples by a DH quantitative analysis method using the above-mentioned oxidation/ reduction pigment are described below.

### (Working example 78)

Using the same catalyst added circulating electrolyzed water as that of working example 73 as test water (AOW), 1 mL of one-fortieth strength Pt standard solution that has undergone the nitrogen gas replacement described above is injected to 200 mL of this test water in a test water holding compartment using a syringe. This is then sufficiently stirred and mixed, and thereafter while visually observing the color change of the test water, a 10 g/ L concentration (mole concentration by volume: 26773.8 µM) of methylene blue solution is injected a little bit at a time into the test water using a syringe. The total amount of methylene blue solution injected until reaching the end point was 7.8 mL, and the measured dissolved hydrogen concentration DH found by replacing the values in Equation 7 was 2.09 (mg/ L). Each physical property value of the test water according to this working example 78 is shown in Table 4, and the effective value of the dissolved hydrogen concentration DH is shown in FIG. 40.

### (Working example 79)

Using catalyst-free circulating electrolyzed water as test water, which is the same base water 6.86 as that of reference example 22 that has been subjected to electrolysis processing using a continuous flow circulating method (volume of circulatory water: 0.8 liters) for 3 minutes under the same electrolysis conditions as those of working example 71, 1 mL of one-fortieth strength Pt standard solution that has undergone the nitrogen gas replacement described above is injected to 200 mL of this test water in a test water holding compartment using a syringe. This is then sufficiently stirred and mixed, and thereafter while visually observing the color change of the test water, a 10 g/ L concentration (mole concentration by volume: 26773.8 µM) of methylene blue solution is injected a little bit at a time into the test water using a syringe. The total amount of methylene blue solution injected until reaching the end point was 8.5 mL, and the measured dissolved hydrogen concentration DH found by replacing the values in Equation 7 was 2.28 (mg/ L). Each physical property value of the test water according to this working example 79 is shown in Table 4, and the effective value of the dissolved hydrogen concentration DH is shown in FIG. 40.

### (Working example 80)

Pd colloid-containing activated charcoal processing water, which is obtained by adding the same Pd standard solution as that of working example 31 sufficient to give a concentration of 384 µg/ L to activated charcoal processing water resulting from processing Fujisawa City tap water through an activated charcoal column, is prepared. Using catalyst added circulating electrolyzed water as test water (AOW), which is Pd colloid-containing activated charcoal processing water prepared in this manner that has been subjected to electrolysis processing using a continuous flow circulating method (volume of circulatory water: 0.8 liters) for 3 minutes under the same conditions as those of working example 71, 1 mL of one-fortieth strength Pt standard solution that has undergone the nitrogen gas replacement described above is injected to 200 mL of this test water in a test water holding compartment using a syringe. This is then sufficiently stirred and mixed, and thereafter while visually observing the color change of the test water, a 10 g/ L concentration (mole concentration by volume: 26773.8 µM) of methylene blue solution is injected a little bit at a time into the test water using a syringe. The total amount of methylene blue solution injected until reaching the end point was 9.7 mL, and the measured dissolved hydrogen concentration DH found by replacing the values in Equation 7 was 2.60 (mg/ L). Each physical property value of the test water according to this working example 80 is shown in Table 4, and the effective value of the dissolved hydrogen concentration DH is shown in FIG. 40.

### (Working example 81)

Using catalyst added circulating electrolyzed water as test water, which is activated charcoal processing water containing Pd colloid prepared in this manner that has been subjected to electrolysis processing using a continuous flow circulating method (volume of circulatory water: 0.8 liters) for 3 minutes under the same conditions as those of working example 79, 1 mL of one-fortieth strength Pt standard solution that has undergone the nitrogen gas replacement described above is injected to 200 mL of this test water in a test water holding compartment using a syringe. This is then sufficiently stirred and mixed, and thereafter while visually observing the color change of the test water, a 10 g/ L concentration (mole concentration by volume: 26773.8 µM) of methylene blue solution is injected a little bit at a time into the test water using a syringe. The total amount of methylene blue solution injected until reaching the end point was 10.6 mL, and the measured dissolved hydrogen concentration DH found by replacing the values in Equation 7 was 2.84 (mg/ L). Each physical property value of the test water according to this working example 81 is shown in Table 4, and the effective value of the dissolved hydrogen concentration DH is shown in FIG. 40.

### (Working example 82)

Pd colloid-containing activated charcoal processing water, which is obtained by adding the same Pd standard solution as that of working example 31 sufficient to give a concentration of 192 µg/ L to activated charcoal processing water resulting from processing Fujisawa City tap water through an activated charcoal column, is prepared.

Using catalyst added circulating electrolyzed water as test water (AOW), which is Pd colloid-containing activated charcoal processing water prepared in this manner that has been subjected to electrolysis processing using a continuous flow circulating method (volume of circulatory water: 0.8 liters) for 3 minutes under the same conditions as those of working example 80, 1 mL of one-fortieth strength Pt standard solution that has undergone the nitrogen gas replacement described above is injected to 200 mL of this test water in a test water holding compartment using a syringe. This is then sufficiently stirred and mixed, and thereafter while visually observing the color change of the test water, a 10 g/ L concentration (mole concentration by volume: 26773.8 µM) of methylene blue solution is injected a little bit at a time into the test water using a syringe. The total amount of methylene blue solution injected until reaching the end point was 12.0 mL, and the measured dissolved hydrogen concentration DH found by replacing the values in Equation 7 was 3.21 (mg/ L). Each physical property value of the test water according to this working example 82 is shown in Table 4, and the effective value of the dissolved hydrogen concentration DH is shown in FIG. 40.

**[Table 4]**

| | | pH | ORP[mV] | EC[mS/m] | WATER TEMP T [°C] | DH EFFECTIVE [mg/L] |
|---|---|---|---|---|---|---|
| | REFERENCE EXAMPLE 17 | 9.8 | -171 | 17 | 21.6 | 0.03 |
| | REFERENCE EXAMPLE 18 | 7.2 | -623 | 99 | 21.2 | 1.66 |
| | WORKING EXAMPLE 78 | 7.1 | -650 | 98 | 22.3 | 2.09 |
| | WORKING EXAMPLE 79 | 7.1 | -650 | 98 | 22.3 | 2.28 |
| | WORKING EXAMPLE 80 | 7.8 | -645 | 15 | 22.3 | 2.60 |
| | WORKING EXAMPLE 81 | 8.9 | -707 | 15 | 18.0 | 2.84 |
| | WORKING EXAMPLE 82 | 7.4 | -605 | 14 | 18.0 | 3.21 |

### Examination of influence of antioxidant-functioning water (AOW) upon life span of nematode Caenorhabditis elegans (C. elegans)

Caenorhabditis elegans (hereafter, referred to as 'C. elegans') is a type of nematoda, and is used worldwide as an aging model for multicellular organisms as well as for fruit-flies, mice, and rats. In addition, the complete genome sequence has been determined in C. elegans, and it has been noticed as a 'living test tube' for examining individual function/ action mechanisms of human hereditary disease-causing genes or oncogenes by combining methods such as gene disruption, expression analysis using GFP fusion genes, and the like.

It is worth noting that the longest life span of wild-type C. elegans is extremely short, approximately 25 days (see 'AGING AT A MOLECULAR LEVEL' written by Naoaki ISHII, Kodansha, 2001, p102-103). Usage of C. elegans as an experimental animal allows quick examination of the influence of antioxidant-functioning water upon the life span of animals.

Therefore, the inventors carried out testing in order to examine the influence upon the life span of C. elegans when antioxidant-functioning water (AOW) is used as feeding water for wild C. elegans through the supervision and cooperation of Mr. Naoaki Ishii, assistant professor of molecular life science, School of medicine, Tokai University, who wrote the document 'Aging at a Molecular Level' described above. What is called 'feeding water' here is water that is used during each manipulation of leaving larvae in water for 2 to 3 hours in step (4), and dripping water on the surface of an agar culture when determining either the life or death of the larvae, or when the agar culture becomes dry in step (8) from testing procedures in section (A-2) to be described later, which are carried out for testing groups of purified water and antioxidant-functioning water, respectively.

An outline of this testing protocol is described in the following section (A), working examples and reference examples of this test are described in the following section (B), and results and examination of the test are described in the following section (C). It should be noted that this test conforms to 'Section 2, Measurement of life span at various oxygen concentrations' described in P290-292 of 'Aging Model', which is described in P288-292 of 'Active oxygen experimental protocol - measurement method, gene analysis, and pathologic physiological model' edited by Naoyuki Taniguchi: Cell Engineering Separate Volume, Experimental protocol series, issued by Shujunsha, Co., Ltd. (hereafter, this reference is abbreviated as 'reference procedure'). The contents described in the above-mentioned 'Aging Model' are incorporated herein by reference. It should be noted that a part of the reference procedure is altered in consideration of special characteristics of this test such as examining the influence upon C. elegans when feeding water is changed. Therefore, in the description of this testing protocol, the altered portions of the reference procedure are mainly described.

### (A) Outline of Testing Protocol

### (A-1) Reagents to be used

Reagents to be used in this test are as follows.
(1) 5-fuluoro-2'-deoxyuridine (FudR) manufactured by Wako Pure Chemical Industries, Ltd.
(2) S buffer
   Sodium chloride: NaCl (0.1M)
   Potassium phosphate (pH6.0)

Since C. elegans are hermaphrodites, a measure is necessary to prevent from getting confused with the next generation. Therefore, reagent FudR is used in conformity with the reference procedure in order to inhibit the next generation of C. elegans from emerging. In addition, the S buffer is used to eliminate influences due to difference in pH.

### (A-2) Testing procedures

(1) Wild-type primary larvae that have been subjected to synchronized culture are collected in conformity with the reference procedure. 500 to 1000 larvae are placed in a 9 cm schale including the agar culture. The lifetime of larvae upon this manipulation is approximately four days.
(2) A suitable amount of the S buffer that has been collected by a Pasteur's pipette is poured into the above-mentioned schale, and are then suctioned in by the Pasteur's pipette together with the larvae in the schale. The suctioned S buffer and larvae are then transferred to a tube (diameter: 1.5 mm). When the tube is left standing upright, the larvae begin to settle at the bottom of the tube. After the larvae have settled, the S buffer (supernatant) in the tube is gently suctioned and removed using the Pasteur's pipette while being careful not to suction the larvae. As a result, the S buffer in the tube is removed and the larvae are collected in the tube.
(3) A suitable amount (approximately 100 to 200) of larvae collected in the tube in step (2) are taken out, and are divided and put into two tubes (diameter: 1.5 mm, capacity: approximately 1 to 2 cc), respectively. A control (purified water of reference example 28 to be described later) is then poured into one of the above-mentioned two tubes, and test water (antioxidant-functioning water of working example 83 to be described later) into the other.
(4) Caps are attached to the openings of both tubes, and the tubes are laid down and left to stand for 2 to 3 hours.
(5) Once they have been laid down and left to stand, the larvae are transferred from the respective tubes into two 9 cm schales including the agar culture, and are left to stand overnight at room temperature.
(6) Twenty 3 cm schales including the agar culture with slightly dried surfaces are prepared. One drop of colon bacillus, which is food for the larvae, is applied at nearly the center of each 3-cm schale. Thus, it becomes easy to observe the larvae since they gather to the center to seek for food.
(7) The larvae are transferred from the two respective 9-cm schales that have been left to stand overnight at room temperature in step (5) into ten of the 3-cm schales prepared in step (6) using a platinum wire for isolation to be described later, with ten larvae in each schale. These 100 larvae are used as one test group. When there are two test groups, this manipulation must be carried out for each test group. The reagent FudR is also added when dividing the larvae. Here, the platinum wire for isolation is a tool of the inventors own making where a platinum wire having a length of approximately 3 cm, and diameter of 50 to 100 µm is attached to a narrow-mouthed section of the 3 cm Pasteur's pipette, the tip of the platinum wire is sharpened into an acute angle using a file or the like, and the wire is bent at a right angle approximately 5 mm from the tip. The tip (the portion that touches larvae or agar culture) is sterilized by a burner flame each time it is used. It is used by scooping up a larva from the bottom. When the tip is made to lightly touch the agar after catching the larva, the larva shifts to the agar culture by itself.
(8) Life and death (lifetime) of the larvae in each group are examined every day (every other day in this test as a rule). In determination of either life or death of the larvae, a larva is determined to be dead when it does not react when stimulated by lightly touching the head of the larva using the platinum wire for isolation or applying a drop of water. When the agar culture becomes dry, several drops of water are applied onto the surface of the agar culture using the Pasteur's pipette.

### (B) Disclosure of working examples and reference examples

### (Reference example 28)

Life span data of a nematode C. elegans in conformity with testing procedure described in (A-2) when activated charcoal processing water (purified water) resulting from processing Fujisawa City tap water through an activated charcoal column is employed as feed water is given as reference example 28.

### (Working example 83)

Life span data of a nematode C. elegans obtained in conformity with the testing procedures described in (A-2) when catalyst added circulating electrolyzed water (AOW) is used as feed water, which is obtained by adding an amount of the Pd standard solution described in working examples 6 through 8 sufficient to give a Pd colloid concentration of 192 µg/ L to 1 liter of the same activated charcoal processing water (purified water) as that of reference example 28 that has been subjected to electrolysis processing (corresponding to two-pass electrolysis processing) using a continuous flow circulating method (volume of circulatory water: 0.8 liters) for 1 minute under the conditions of a 1 L/ min flow and 5A constant current, is given as working example 83.

### (C) Test results and examination thereof

FIGS. 41 and 42, which compare reference example 28 (a group that uses purified water as feed water) and working example 83 (a group that uses antioxidant-functioning water as feed water), show the influence of Pt colloid catalyst-containing electrolyzed water (AOW) upon the life span of C. elegans. In addition, Table 5 shows the results of a significant difference test for differences in average life span between two independent groups according to this test using a student's t-test.

**[Table 5]**

| STUDENT'S t-TEST: TEST RESULT USING TWO SAMPLES ASSUMING EQUIVALENT VARIANCE (RELATIVE RISK : 0.1%) | | |
|---|---|---|
| | ANTIOXIDANT FUNCTIONING WATER | PURIFIED WATER |
| AVERAGE LIFE SPAN | 20.05051 | 17.74737 |
| DISPERSION | 16.66069 | 14.97805 |
| NUMBER OF OBSERVED SAMPLES | 99 | 95 |
| POOLED DISPERSION | 15.83690 | |
| DIFFERENCE FROM HYPOTHESIS AVERAGE | 0 | |
| DEGREE OF FREEDOM | 192 | |
| t | 4.02961 | |
| P(T<=t) (ONE -SIDE) | 0.00004 | |
| BOUNDARY VALUE FOR t (ONE -SIDE) | 3.13325 | |
| P(T<=t) (TWO SIDES) | 8.0467E-05 | |
| BOUNDARY VALUES FOR t (TWO-SIDES) | 3.34199 | |
| TEST RESULT: | | |
| SINCE THE RESULT IS t = 4.03 > TWO-SIDED BOUNDARY VALUES FOR t (3.34) BASED ON THE RELATIVE RISK OF 0.1%, THE NULL HYPOTHESIS THAT 'AVERAGE LIFE SPANS OF A GROUP THAT USES ANTIOXIDANT FUNCTIONING WATER AS FEED WATER AND A GROUP THAT USES PURIFIED WATER AS FEED WATER ARE EQUAL TO EACH OTHER' IS REJECTED. ACCORDINGLY, THE AVERAGE LIFE SPAN (20.05 DAYS) OF THE GROUP THAT USES ANTIOXIDANT-FUNCTIONING WATER AS FEED WATER IS ONLY 2.3 DAYS LONGER THAN THE AVERAGE LIFE SPAN (17.75 DAYS) OF THE GROUP THAT USES PURIFIED WATER AS FEED WATER. THIS IS A SIGNIFICANT DIFFERENCE (t(192) = 4.03, SD = 0.57, AND P < 0.001). | | |

As shown in Table 5, since the result is t = 4.03 > two-sided boundary values for t (3.34) based on the relative risk of 0.1%, the null hypothesis that 'average life spans of a group that uses antioxidant-functioning water as feed water and a group that uses purified water as feed water are equal to each other' is rejected. Accordingly, the average life span (20.05 days) of a group that uses antioxidant-functioning water as feed water (number of samples: 99) is 2.3 days longer than the average life span (17.75 days) of a group that uses purified water as feed water (number of samples :95). This is a significant difference (t (192) = 4.03, SD = 0.57, and P < 0.001). Here, 't(192) = 4.03' represents t value, where '192' represents a degree of freedom, SD represents the standard deviation of differences in average life spans of the two groups, and p represents relative risk.

To examine the test results, the life span of the nematode C. elegans has been prolonged since antioxidant-functioning water controls the oxidizing disturbance emanating from active oxygen species. The active oxygen species have toxic effects upon living cells due to oxidizing disturbance to intracellular molecules such as protein, nucleic acid, and the like. In short, it has been clarified through a study using nematoda and fruit-flies (See references such as 'Life Span Determination Mechanism of Nematoda' Naoaki ISHII, Cell Engineering, vol. 21 No. 7, 2002, Agarwal, S. et al, Proc. Natl. Acad. Sci. U. S. A., 91, 12332-12335, 1994, Larsen, P. L. Proc. Natl. Acad. Sci. U. S. A., 90, 8905-8909, 1993, Sohal, R. S. et al, J. Biol. Chem., 270, 15671-15675, 1995) that the oxidizing disturbance emanating from the active oxygen species is involved in aging. It is certain that in this test as with the contents of these studies, findings suggesting that the oxidizing disturbance emanating from the active oxygen species is involved in aging have been obtained.

Description of the embodiments herein has been made to facilitate understanding of the present invention and is not intended to limit the invention in any way. Accordingly, each element disclosed in the above embodiments may include all possible design modifications and equivalents as falls within the technical scope of the invention.

More specifically, in the descriptions of the embodiments, reference examples, and working examples of the present invention, methylene blue was shown as an example of an oxidation/ reduction pigment, however, the oxidation/ reduction pigment is not limited to this. For example, acid yellow 3, acid yellow 23, acid yellow 25, acid yellow 36, acid orange 5, acid orange 6, acid orange 7, acid orange 10, acid orange 19, acid orange 52, acid green 16, acid green 25, acid violet 43, acid brown 2, acid black 1, acid black 31, acid blue 3, acid blue 9, acid blue 40, acid blue 45, acid blue 47, acid blue 59, acid blue 74, acid blue 113, acid blue 158, acid red 1, acid red 2, acid red 14, acid red 18, acid red 27, acid red 37, acid red 51, acid red 52, acid red 87, acid red 88, acid red 91, acid red 92, acid red 94, acid red 95, acid red 111, solvent yellow 2, solvent black 3, solvent blue 7, solvent blue 11, solvent blue 25, solvent red 3, solvent red 19, solvent red 23, solvent red 24, solvent red 73, direct yellow 1, direct yellow 9, direct yellow 12, direct yellow 59, direct green 1, direct green 6, direct green 59, direct brown 1, direct brown 6, direct black 4, direct black 22, direct black 38, direct blue 1, direct blue 6, direct blue 53, direct blue 86, direct blue 106, direct red 2, direct red 28, direct red 79, dispersed yellow 3, dispersed yellow 5, dispersed yellow 8, dispersed yellow 42, dispersed yellow 60, dispersed yellow 64, dispersed orange 3, dispersed orange 30, dispersed violet 26, dispersed violet 28, dispersed blue 1, dispersed blue 26, dispersed red 1, dispersed red 4, dispersed red 60, dispersed red 65, dispersed red 73, vat yellow 2, vat green 1, vat green 3, vat brown 1, vat brown 3, vat black 25, vat blue 1, vat blue 4, vat blue 20, vat red 10, vat red 41, pigment yellow 1, pigment yellow 3, pigment yellow 10, pigment yellow 12, pigment yellow 13, pigment yellow 14, pigment yellow 17, pigment yellow 24, pigment yellow 55, pigment yellow 81, pigment yellow 83, pigment yellow 93, pigment yellow 94, pigment yellow 95, pigment yellow 97, pigment yellow 98, pigment yellow 99, pigment yellow 108, pigment yellow 109, pigment yellow 110, pigment yellow 116, pigment yellow 117, pigment yellow 138, pigment yellow 151, pigment yellow 154, pigment orange 5, pigment orange 13, pigment orange 14, pigment orange 16, pigment orange 36, pigment orange 38, pigment orange 40, pigment orange 43, pigment green 4, pigment green 7, pigment green 8, pigment green 10, pigment green 36, pigment violet 1, pigment violet 3, pigment violet 19, pigment violet 23, pigment violet 33, pigment brown 25, pigment black 1, pigment blue 2, pigment blue 15, pigment blue 16, pigment blue 17, pigment blue 18, pigment blue 24, pigment red 1, pigment red 3, pigment red 5, pigment red 9, pigment red 22, pigment red 38, pigment red 48:1, pigment red 48:2, pigment red 48:3, pigment red 48:4, pigment red 49:1, pigment red 52:1, pigment red 53:1, pigment red 57:1, pigment red 60, pigment red 63:2, pigment red 64:1, pigment red 81, pigment red 83, pigment red 88, pigment red 112, pigment red 122, pigment red 123, pigment red 144, pigment red 146, pigment red 149, pigment red 151, pigment red 166, pigment red 168, pigment red 170, pigment red 174, pigment red 175, pigment red 176, pigment red 177, pigment red 178, pigment red 179, pigment red 185, pigment red 187, pigment red 208, food yellow 3, food green 3, food red 6, food red 17, basic yellow 1, basic yellow 2, basic yellow 11, basic orange 1, basic orange 11, basic green 4, basic violet 3, basic violet 4, basic violet 10, basic violet 14, basic brown 1, basic blue 1, basic blue 3, basic blue 9, basic blue 24, basic red 1, basic red 2, basic red 5, basic red 9, basic red 18, mordant yellow 1, mordant yellow 3, mordant orange 1, mordant violet 26, mordant black 11, mordant blue 13, mordant blue 29, mordant red 3, mordant red 11, mordant red 15, reactive yellow 2, reactive yellow 3, reactive yellow 17, reactive orange 1, reactive orange 2, reactive orange 16, reactive violet 2, reactive black 5, reactive blue 2, reactive blue 5, reactive blue 7, reactive blue 19, reactive red 1, reactive red 3, reactive red 6, reactive red 17, reactive red 22, and reactive red 41 may also be favorably used as a pigment with a prefix. In addition, acridine yellow G, alizarin, indamine, indoaniline, indocyanine green, urothion, urobilin, p-ethoxychrysoidne hydrochloride, m-cresol purple, o-cresol phthalein, cresol red, chloroethanoic acid, chlorophyll (a, b, c, d), chlorophenol red, new methylene blue, neutral red, variamine blue B hydrochloride, methylviologen, pyocyanin, indigo carmine, safranine T, phenosafranine, Capri blue, Nile blue, diphenylamine, xylenecyanol, nitrodiphenylamine, ferroin, N-phenylanthranilic acid, 2,6-sodium dichloroindophenol, 4-sodium diphenylaminesulfomic acid, N,N'-diphenylbenzidine, cinnavaline (antibiotics), toluylene blue, riboflavin (vitamin B2), acridine yellow G, p-ethoxychrysoidine hydrochloride, tetrazolium blue, and diformazan (reduced tetrazolium blue) may also be favorably used as a pigment with no prefix. Of these, in particular, methylviologen, variamine blue B hydrochloride, neutral red, pyocyanin, 2,6-sodium dichlorophenol, 4-sodium diphenylamine sulfonic acid, N,N'-diphenylbenzidine, cinnavalune (antibiotics), and toluylene blue may also be favorably used since their change in hue in this titration is as clear as methylene blue.

In addition, in the descriptions of the embodiments, reference examples, and working examples of the present invention, platinum (Pt) was shown as an example of a precious metal catalyst which is used for dissolved hydrogen concentration quantitative analysis, however, the precious metal catalyst is not limited to this. For example, platinum, palladium, rhodium, iridium, ruthenium, gold, silver, or rhenium, along with the respective salts thereof, alloy chemical compounds, or colloidal particles themselves such as complex chemical compounds, as well as mixtures thereof.

Finally, a method for hydrogen recompression treatment, which is a modified example where the antioxidation method according to the present invention is applied to medical care of patients, is described. To begin with, a catalyst solution according to the present invention such as Pt colloid solution or Pd colloid solution is delivered to the region of the patient's body to be subjected to treatment using a maneuver such as injection or intravenous drip. Next, the patient is placed in a recompression chamber such as that generally used for treatment of decompression sickness such as dysbarism, and the air pressure in the recompression chamber is gradually increased while observing the condition of the patient either from outside the chamber or inside the chamber. Here the gas supplied into the recompression chamber is adjusted so that hydrogen makes up between approximately 1 and 20 % of the partial pressure ratio of combined components. Then while observing the condition of the patient either from outside the chamber or inside, patient is kept in the gaseous environment that is between 2 and 3 absolute atmospheres and having an exemplary partial pressure ratio of 1:2:7 hydrogen: oxygen: nitrogen (trace amounts of other gaseous components are ignored) for approximately 1 hour, and following this, the pressure is gradually reduced to normal atmospheric pressure over a period of time equal to or longer than when pressure was being increased. Throughout this, in the region in the patient's body to be subjected to treatment (antioxidation subject), the hydrogen included in the biological fluid (hydrogen- dissolved water) via the pulmonary respiration and cutaneous respiration of the patient and the delivered catalyst meet at the subject region allowing electrons to be universally applied in the subject region. As a result, active oxygen species localized in the subject region are scavenged. Medicinal benefits in the subject region may be anticipated through this hydrogen recompression treatment method.

## Claims

1. An antioxidation method, comprising transforming an antioxidation subject, which is in an oxidation state due to a deficiency of electrons or needs to be protected from oxidation, into a reduced state of electrons being filled by promoting the breaking reaction of molecular hydrogen used as a substrate included in hydrogen- dissolved water into a product of active hydrogen via a process employing a catalyst, which is a precious metal colloid or a hydrogen oxidation/ reduction enzyme (except for those already existing in a living organism), on the hydrogen-dissolved water.

2. The antioxidation method set forth in claim 1, wherein
said hydrogen oxidation/ reduction enzyme is a hydrogenase.

3. The antioxidation method set forth in claim 1, wherein
said precious metal colloid includes platinum, palladium, rhodium, iridium, ruthenium, gold, silver, or rhenium, along with the respective salts thereof, alloy chemical compounds, or colloidal particles themselves such as complex chemical compounds, as well as mixtures thereof.

4. The antioxidation method set forth in any of claims 1 through 3, wherein
said hydrogen- dissolved water, which is all water that contains hydrogen, includes alkaline electrolyzed water generated on the cathode side when raw water is subjected to electrolysis processing between an anode and a cathode via a membrane, or water processed through bubbling or pressurized filling of hydrogen into raw water.

5. The antioxidation method set forth in any of claims 1 through 3, wherein
said hydrogen- dissolved water is a reducing potential water where the ORP is a negative value, and the ORP value corresponding to the pH shows a value lower than the value according to the Nernst equation or ORP = -59 pH - 80 (mV).

6. The antioxidation method set forth in any of claims 1 through 5, wherein
said hydrogen-dissolved water is water with dissolved hydrogen concentration is greater than the saturation concentration (in terms of effective value of dissolved hydrogen concentration value found using a dissolved hydrogen concentration quantitative analysis method that uses oxidation/ reduction pigment) under atmospheric pressure.

7. The antioxidation method set forth in any of claims 1 through 6, wherein said hydrogen-dissolved water is electrolyzed reducing potential water, which is generated by a reducing potential water generation apparatus, comprising:
an electrolytic chamber to which raw water is supplied;
at least one membrane, which separates the inside of said electrolytic chamber from outside thereof;
at least a pair of electrode plates, which are provided inside and outside the electrolytic chamber, respectively, and sandwiches a membrane; and
a power source circuit, which applies a voltage between both electrodes, wherein the electrode plate provided inside the electrolytic chamber is given as the cathode and the electrode plate provided outside the electrolytic chamber is given as the anode; wherein the electrode plates provided outside the electrolytic chamber are provided in contact with the membrane or leaving a slight space.

8. The antioxidation method set forth in any of claims 1 through 7, wherein
at least one reducing agent selected from the group consisting of sulfite, thiosulfate, ascorbic acid, and ascorbate is added to said hydrogen-dissolved water as required.

9. The antioxidation method set forth in any of claims 1 through 8, wherein
said oxidation subject is a general subject, which is either in an oxidation state due to a deficiency of electrons or needs to be protected from oxidation, and includes living cells.

10. An antioxidant-functioning water, which is hydrogen-dissolved water containing a precious metal colloid or a hydrogen oxidation/ reduction enzyme (except for those already existing in the living organism) that catalyzes the breaking reaction of molecular hydrogen used as a substrate included in the hydrogen-dissolved water into a product of active hydrogen, comprising an antioxidation function of transforming an antioxidation subject, which is in an oxidation state due to a deficiency of electrons or needs to be protected from oxidation , into a reduced state of electrons being filled by promoting said breaking reaction via said catalyst.

11. The antioxidant-functioning water set forth in claim 10, wherein
said hydrogen oxidation/ reduction enzyme is a hydrogenase.

12. The antioxidant-functioning water set forth in claim 10, wherein
said precious metal colloid includes platinum, palladium, rhodium, iridium, ruthenium, gold, silver, or rhenium, along with the respective salts thereof, alloy chemical compounds, or colloidal particles themselves such as complex chemical compounds, as well as mixtures thereof.

13. The antioxidant- functioning water set forth in any of claims 10 through 12, wherein
processing or manipulation for adjusting the activation time and/ or reaction time of the catalyst is employed on said catalyst.

14. The antioxidant- functioning water set forth in any of claims 10 through 13, wherein
said hydrogen- dissolved water, which is all water that contains hydrogen, includes electrolyzed water generated on the cathode side when raw water is subjected to electrolysis processing between an anode and a cathode via a membrane, or water processed through bubbling or pressurized filling of hydrogen into raw water.

15. The antioxidant- functioning water set forth in any of claims 10 through 14, wherein
said hydrogen- dissolved water is a reducing potential water where the ORP is a negative value, and the ORP value corresponding to the pH shows a value lower than the value according to the Nernst equation or ORP = -59 pH - 80 (mV).

16. The antioxidant- functioning water set forth in any of claims 10 through 15, wherein
said hydrogen-dissolved water is water with dissolved hydrogen concentration is greater than the saturation concentration (in terms of effective value of dissolved hydrogen concentration value found using a dissolved hydrogen concentration quantitative analysis method that uses oxidation/ reduction pigment) under atmospheric pressure.

17. The antioxidant- functioning water set forth in any of claims 10 through 16, wherein said hydrogen-dissolved water is electrolyzed reducing potential water, which is generated by a reducing potential water generation apparatus, comprising:
an electrolytic chamber to which raw water is supplied;
at least one membrane, which separates the inside of said electrolytic chamber from outside thereof;
at least a pair of electrode plates, which are provided inside and outside the electrolytic chamber, respectively, and sandwiches a membrane; and
a power source circuit, which applies a voltage between both electrodes, wherein the electrode plate provided inside the electrolytic chamber is given as the cathode and the electrode plate provided outside the electrolytic chamber is given as the anode; wherein the electrode plates provided outside the electrolytic chamber are provided in contact with the membrane or leaving a slight space.

18. The antioxidant- functioning water set forth in any of claims 10 through 17, wherein
at least one reducing agent selected from the group consisting of sulfite, thiosulfate, ascorbic acid, and ascorbate is added to said hydrogen-dissolved water as required.

19. A living organism- applicable fluid, **characterized by** being prepared using the antioxidant- functioning water set forth in any of claims 10 through 18 as a main component so as to allow usage on living organisms including drinking, injection, intravenous drip, dialysis, and rinsing.

20. An antioxidant, **characterized by** containing the antioxidant-functioning water in any of claims 10 through 18 as a main component.

21. An anti-aging agent, **characterized by** containing the antioxidant-functioning water in any of claims 10 through 18 as a main component.

22. An anti-deterioration agent, **characterized by** containing the antioxidant-functioning water in any of claims 10 through 18 as a main component.

23. An anti-decomposition agent, **characterized by** containing the antioxidant-functioning water in any of claims 10 through 18 as a main component.

24. An anti-contamination agent, **characterized by** containing the antioxidant-functioning water in any of claims 10 through 18 as a main component.

25. A deodorant, **characterized by** containing the antioxidant-functioning water in any of claims 10 through 18 as a main component.

26. A freshness-keeping agent, **characterized by** containing the antioxidant-functioning water in any of claims 10 through 18 as a main component.
